(19) Europäisches Patentamt / European Patent Office / Office européen des brevets

(11) **EP 3 802 621 B1**

(12) **EUROPEAN PATENT SPECIFICATION**

(45) Date of publication and mention
of the grant of the patent:
**30.07.2025 Bulletin 2025/31**

(21) Application number: **19811035.5**

(22) Date of filing: **31.05.2019**

(51) International Patent Classification (IPC):
**C12N 9/78** *(2006.01)*     **C12N 15/62** *(2006.01)*
**A61P 3/00** *(2006.01)*     **A61P 35/00** *(2006.01)*

(52) Cooperative Patent Classification (CPC):
**A61P 3/00; A61P 35/00; C12N 9/78; C12N 15/62;
C07K 2319/31**

(86) International application number:
**PCT/CN2019/089583**

(87) International publication number:
**WO 2019/228510 (05.12.2019 Gazette 2019/49)**

(54) **COMPOSITION AND APPLICATION OF ARGININE-DEPLETING AGENTS FOR CANCER, OBESITY, METABOLIC DISORDERS, AND RELATED COMPLICATIONS AND COMORBIDITIES**

ZUSAMMENSETZUNG UND ANWENDUNG VON ARGININABREICHERUNGSMITTELN FÜR KREBS, ADIPOSITAS, STOFFWECHSELERKRANKUNGEN UND VERWANDTE KOMPLIKATIONEN UND KOMORBIDITÄTEN

COMPOSITION ET APPLICATION D'AGENTS DE DÉPLÉTION D'ARGININE CONTRE LE CANCER, L'OBÉSITÉ, LES TROUBLES MÉTABOLIQUES ET LES COMPLICATIONS ET COMORBIDITÉS ASSOCIÉES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **31.05.2018 US 201862678300 P**

(43) Date of publication of application:
**14.04.2021 Bulletin 2021/15**

(73) Proprietors:
• **The Chinese University of Hong Kong**
**Shatin, New Territories**
**Hong Kong (CN)**
• **The Hong Kong Polytechnic University**
**Hung Hom, Kowloon**
**Hong Kong (CN)**

(72) Inventors:
• **LEUNG, Yun Chung**
**Kowloon, Hong Kong (CN)**
• **SHUM, Alisa Sau-Wun**
**N.T., Hong Kong (CN)**

(74) Representative: **Fabry, Bernd**
**IP2 Patentanwalts GmbH**
**Schlossstrasse 523**
**41238 Mönchengladbach (DE)**

(56) References cited:
**EP-A1- 0 956 864          EP-A1- 2 411 040
EP-A1- 2 411 040          EP-B1- 2 411 040
WO-A1-2004/001048      WO-A1-2019/228510
WO-A2-2005/027978      WO-A2-2005/028516
WO-A2-2011/008495      WO-A2-2014/138319
US-A- 5 196 195           US-A1- 2014 255 377
US-A1- 2016 115 468**

• **SALJOUGHIAN MANOUCHEHR: "Hypotension : A Clinical Care Review", 19 February 2014 (2014-02-19), XP55905332, Retrieved from the Internet <URL:https://www.uspharmacist.com/ article/hypotension-a-clinical-care-review> [retrieved on 20220325]**
• **SALJOUGHIAN MANOUCHEHR: "Hypotension : A Clinical Care Review", 19 February 2014 (2014-02-19), XP055905332, [retrieved on 20220325]**

- SALJOUGHIAN MANOUCHEHR: "Hypotension : A Clinical Care Review", 19 February 2014 (2014-02-19), XP055905332, Retrieved from the Internet <URL:https://www.uspharmacist.com/article/hypotension-a-clinical-care-review> [retrieved on 20220325]
- R. WILLIAM CALDWELL ET AL: "Arginase: A Multifaceted Enzyme Important in Health and Disease", PHYSIOLOGICAL REVIEWS, vol. 98, no. 2, 1 April 2018 (2018-04-01), US, pages 641 - 665, XP055659286, ISSN: 0031-9333, DOI: 10.1152/physrev.00037.2016
- LI MENGLI ET AL: "Review of arginase as a promising biocatalyst: characteristics, preparation, applications and future challenges", CRITICAL REVIEWS IN BIOTECHNOLOGY, 6 October 2021 (2021-10-06), US, pages 1 - 17, XP055892380, ISSN: 0738-8551, DOI: 10.1080/07388551.2021.1947962
- JALAN R ET AL: "l-Ornithine phenylacetate (OP): A novel treatment for hyperammonemia and hepatic encephalopathy", MEDICAL HYPOTHESES, EDEN PRESS, PENRITH, US, vol. 69, no. 5, 27 April 2007 (2007-04-27), pages 1064 - 1069, XP029096478, ISSN: 0306-9877, DOI: 10.1016/J.MEHY.2006.12.061
- HANY M EL-BASSOSSY ET AL: "Arginase inhibition alleviates hypertension in the metabolic syndrome", BRITISH JOURNAL OF PHARMACOLOGY, WILEY-BLACKWELL, UK, vol. 169, no. 3, 16 May 2013 (2013-05-16), pages 693 - 703, XP071170957, ISSN: 0007-1188, DOI: 10.1111/BPH.12144
- WISSAM ZAM: "Arginine enzymatic deprivation and diet restriction for cancer treatment", BRAZILIAN JOURNAL OF PHARMACEUTICAL SCIENCES, vol. 53, no. 3, 31 December 2019 (2019-12-31), XP055658678

Remarks:

The complete document including Reference Table(s) and the Sequence Listing(s) can be downloaded from the EPO website

Description

TECHNICAL FIELD

The present disclosure relates to arginase albumin binding domain (ABD) fusion proteins and methods of preparation and use thereof. Also provided are methods involving arginine depletion for the treatment of obesity, metabolic disorders, and/or related complications and/or comorbidities.

BACKGROUND

Arginase is a hydrolase that catalyzes the catabolism of arginine to ornithine and urea and is present in most living organisms. In humans, two isoforms of arginase exist, arginase type I and type II, which differ in tissue distribution and molecular characteristics.

Arginase I is found primarily in the cytoplasm of the liver and is a 35 kD protein that exists as a trimer. Arginase II, a ~38.5 kD protein, is typically located in the mitochondria of cells and is believed to be involved in the regulation of the arginine/ornithine concentrations in the cell.

Several types of tumors lack or produce low levels of argininosuccinate synthetase (ASS) and/or ornithine transcarbamoylase (OTC) that are required for arginine synthesis, and are therefore arginine-auxotrophic. Deprivation of arginine exploits a significant vulnerability of these tumor cells and leads to their rapid demise. Consequently, enzyme-mediated arginine depletion has been explored as a potential strategy for selective destruction of tumor cells.

A major impediment of developing enzyme-mediated arginine depletion as a treatment for cancer is the short circulating half-life (from several minutes to a few hours) of arginase due to its low molecular weight (< 50 kDa), and thus is quickly eliminated by renal clearance. In order to maintain an effective therapeutic concentration in systemic circulation, a frequent dosing schedule may be required, which may raise issues of patient compliance.

In view of this problem, different strategies have been evolved to improve the pharmacokinetic properties of therapeutic proteins. Conjugation of polyethylene glycol (PEG) to different therapeutic proteins, i.e. PEGylation, has been shown to increase their hydrodynamic radius and hence slow down their elimination by renal clearance. Fusion protein strategies based on the recycling mechanisms of the Fc neonatal receptor (FcRn) have also been explored, in which fusion of fragment crystallizable (Fc) region of immunoglobulin, albumin or albumin binding peptide to proteins has been shown to increase their circulation half-life. Nonetheless, there still exists a need to develop arginine depleting proteins with improved pharmacokinetics.

US 5196195A discloses a method for limiting nitric oxide production in a subject in need of such limiting, said method comprising parenterally administering an amount of an arginine depleting enzyme to effect reduction of plasma level of arginine to a nitric oxide production limiting level, wherein the arginine depleting agent is an enzyme selected from the group consisting of arginase, arginine decarboxylase and arginine deiminase.

EP 0956864A1 discloses tissue fibrosis inhibiting agent comprising an arginine-degrading enzyme, a variant or a modified derivative thereof as an active ingredient. This agent can be used to inhibit tissue fibrosis in liver, kidney, lung, pancreas, skin, etc.

WO 2014/138319 A2 provides a pharmaceutical composition containing albumin-binding arginine deiminase fusion protein (AAD) for treating cancer or certain other arginine-dependent diseases.

Obesity, metabolic disorders, and related complications and comorbidities are regarded as some of the most serious global public health problems in the 21st century, imposing huge socioeconomic burden. Moreover, the metabolic consequences of obesity are drivers of other disorders, such as type 2 diabetes mellitus, glucose intolerance, insulin resistance, hypercholesterolemia, dyslipidemia, hypertension, cardiovascular diseases and cancer. Thus, safe and effective methods for prevention and/or treatment of obesity, metabolic disorders, and related complications and comorbidities are in great need. Obesity is the result of an imbalance between energy intake and expenditure. Lifestyle interventions alone can be ineffective for some individuals to lose sufficient weight or maintain sustained weight loss to improve health. Anti-obesity medications, including orlistat (Xenical), lorcaserin (Belviq) and a combination of phentermine and topiramate (Qsymia), may be taken to achieve weight loss via reducing appetite or decreasing fat absorption. However, these drugs have various side effects, such as decreased absorption of fat-soluble vitamins, headache, fatigue, dizziness, diarrhea and fecal incontinence. Bariatric surgery, e.g. gastric banding, can be used to treat severe obesity for achieving long-term weight loss, and liposuction for removal of unwanted focal fat deposits has gained popularity for cosmetic reasons. However, these types of invasive procedures also carry substantial risks. Thus, there is a need to develop improved methods of treating obesity, metabolic disorders, and related complications and comorbidities.

SUMMARY

In order to address one or more of the above mentioned needs provided herein is an arginine depleting agent for use in treating obesity, metabolic disorders, and/or related complications and/or comorbidities, wherein the arginine depleting agent is an arginine catabolic enzyme selected from the group consisting of an arginase protein, an arginine deiminase protein, and an arginine decarboxylase protein, the metabolic disorder is selected from the group consisting of glucose intolerance, hyperglycemia, diabetes mellitus, hepatic steatosis, renal steatosis, pancreatic steatosis, cardiac steatosis, and insulin resistance; and the related complication is one or more conditions selected from the group consisting of diabetic nephropathy, diabetic vasculopathy, diabetic neuropathy, hypercholesterolemia, dyslipidemia, hypertriglyceridemia, hyperleptinemia, hyperinsulinemia, steatosis, steatohepatitis, hypertension, cardiovascular disease, and whitening of brown fat.

In a first embodiment of the present application, the metabolic disorder is insulin resistance.

In a second embodiment of the present application, the treatment of obesity comprises at least one of preventing fat mass gain and reducing fat mass.

In a third embodiment of the present application the metabolic disorder is selected from the group consisting of hepatic steatosis, renal steatosis, pancreatic steatosis and cardiac steatosis.

In a fourth embodiment of the present application, the arginine concentration in the subject's serum is maintained below 50 μM.

In a fifth embodiment of the present application, the arginase protein, arginine deiminase protein, or arginine decarboxylase protein further comprises one or more polyethylene glycol (PEG) groups.

In a sixth embodiment of the present application, the arginase protein comprises a polypeptide having SEQ ID NO: 101, SEQ ID NO: 102, SEQ ID NO: 103, or SEQ ID NO: 104.

In a seventh embodiment of the present application, the arginase protein comprises a polypeptide with at least 95% sequence homology with SEQ ID NO: 69, SEQ ID NO: 70, SEQ ID NO: 71, or SEQ ID NO: 72.

In an eighth embodiment of the present application, the arginase protein, arginine deiminase protein, or arginine decarboxylase protein further comprises an albumin binding domain or human serum albumin, or a human IgG Fc domain.

In a ninth embodiment of the present application wherein the ABD comprises a polypeptide with at least 93% sequence homology with SEQ ID NO: 66, SEQ ID NO: 67, or SEQ ID NO: 68.

In a tenth embodiment of the present application, the arginine catabolic enzyme is a fusion protein comprising an ABD polypeptide and an arginase polypeptide; an ABD polypeptide and an arginine deiminase polypeptide; or an ABD polypeptide and an arginine decarboxylase polypeptide.

In an eleventh embodiment of the present application, wherein the arginine catabolic enzyme is a fusion protein comprising an ABD polypeptide and an arginase polypeptide.

In a twelfth embodiment of the present application, the arginine catabolic enzyme comprises a polypeptide having at least 98% sequence homology with SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 75, or SEQ ID NO: 76.

In a thirteenth embodiment of the present application, the arginine catabolic enzyme comprises two $Mn^{2+}$ metal ions.

In a fourteenth embodiment of the present application, the arginine catabolic enzyme comprises one or more divalent metal ions selected from the group consisting of $Co^{2+}$ and $Ni^{2+}$.

## BRIEF DESCRIPTION OF THE DRAWINGS

The above and other objects and features of the present disclosure will become apparent from the following description of the invention, when taken in conjunction with the accompanying drawings, in which:

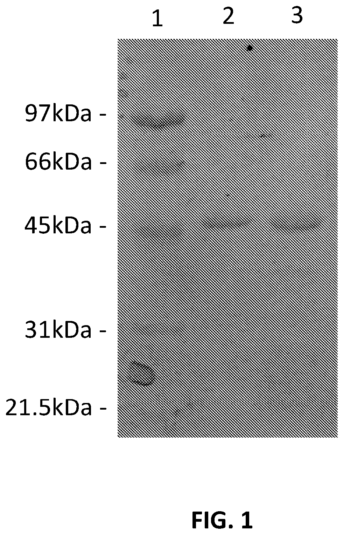

FIG. 1 illustrates purification of the albumin binding domain (ABD) recombinant human arginase (rhArg) fusion protein N-ABD094-rhArg (SEQ ID NO: 50) by sodium dodecyl sulfate polyacrylamide gel electrophoresis (SDS-PAGE). Lane 1: SDS-PAGE low range marker (Bio-rad); Lane 2: N-ABD094-rhArg (SEQ ID NO: 50) 20x dilution; Lane 3: N-ABD094-rhArg (SEQ ID NO: 50) 10x dilution.

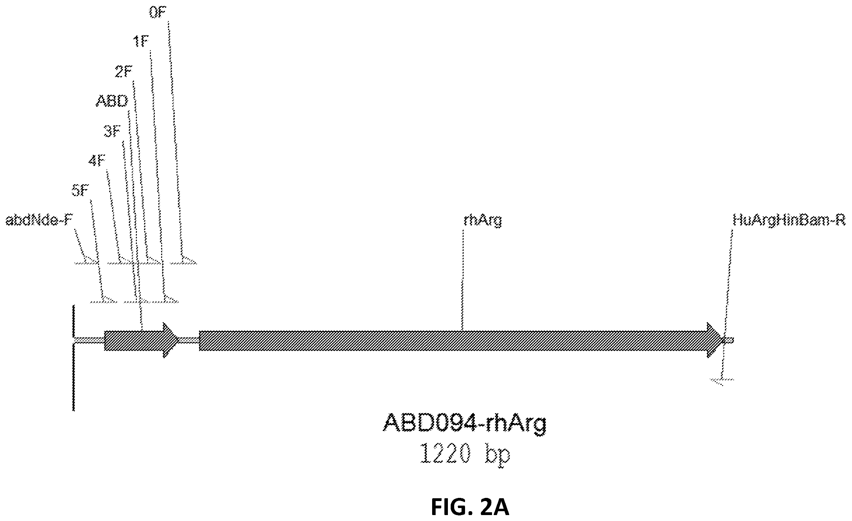

FIG. 2A illustrates an exemplary N-ABD094-rhArg gene (SEQ ID NO: 38) with the binding locations of the primers annotated.

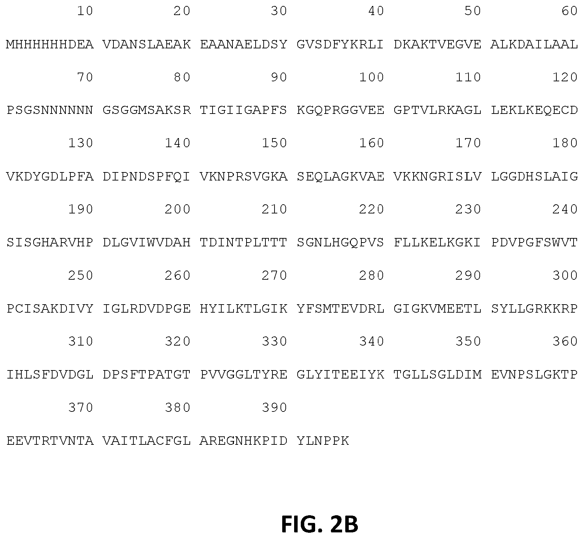

FIG. 2B illustrates the protein sequence of the N-ABD094-rhArg (SEQ ID NO: 50) fusion protein with a theoretical pI/Mw of 6.07 / 42555.38.

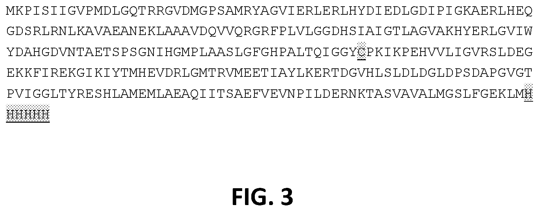

FIG. 3 illustrates the protein sequence of the engineered *Bacillus caldovelox* arginase BCA (S161C)-His (SEQ ID NO: 89) with a single site serine-to-cysteine mutation and a C-terminal histidine tag. The cysteine residue at position 161 and 6xHis-tag are highlighted.

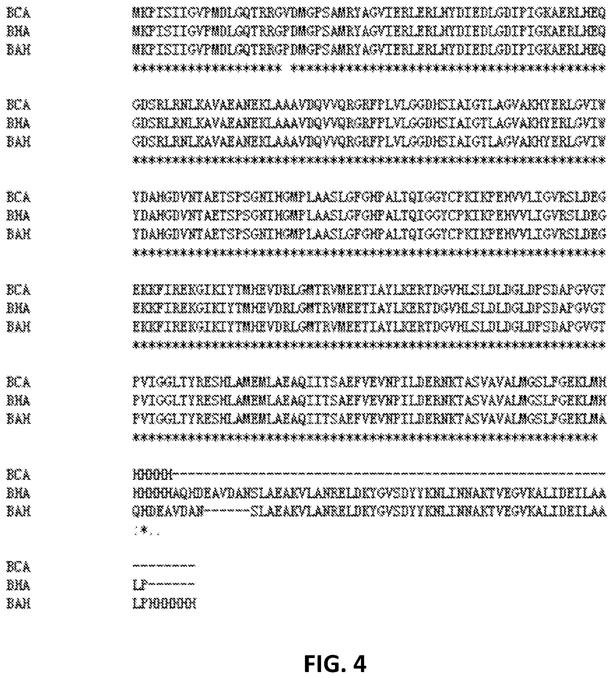

FIG. 4 illustrates the protein sequence alignment of BCA (S161C)-His (SEQ ID NO: 89), the BCA-6xHis-ABD fusion protein BHA (SEQ ID NO: 75) and the BCA-ABD-6xHis fusion protein BAH (SEQ ID NO: 76) by CLUSTALW. Scores of alignment: BCA versus BHA: 99.6721; BCA versus BAH: 98.0328; BHA versus BAH: 97.5138.

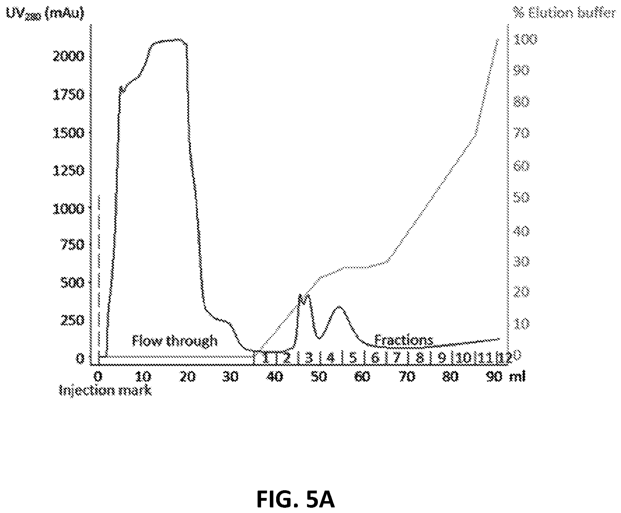

FIG. 5A illustrates the elution profile of the purification of BHA (SEQ ID NO: 75) from *E. coli* grown in 500 mL shake flask

4

culture by a single-step of nickel-charged 5 mL HiTrap chelating HP column chromatography. mAU, milli absorption unit; 100% B=0.5 M imidazole.

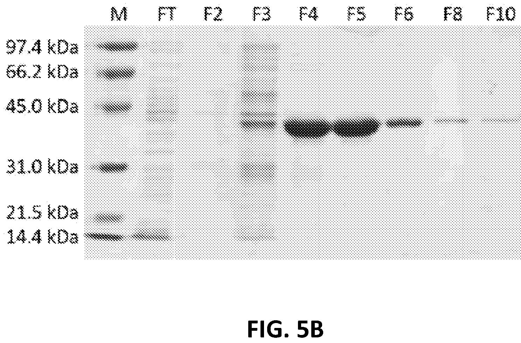

**FIG. 5B** illustrates the SDS-PAGE analysis of the column fractions from nickel affinity chromatography for BHA (SEQ ID NO: 75). M, SDS-PAGE molecular weight markers, low range (Bio-Rad); FT, flow through; F2-F10, eluted fractions from the column.

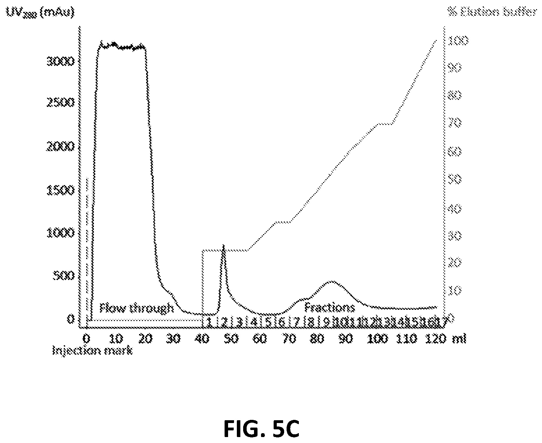

**FIG. 5C** illustrates the elution profile of the purification of BAH (SEQ ID NO: 76) from *E. coli* cells grown in 500 mL shake flask culture by a single-step of nickel-charged 5 mL HiTrap chelating HP column chromatography. mAU, milli absorption unit; 100% B = 0.5 M imidazole.

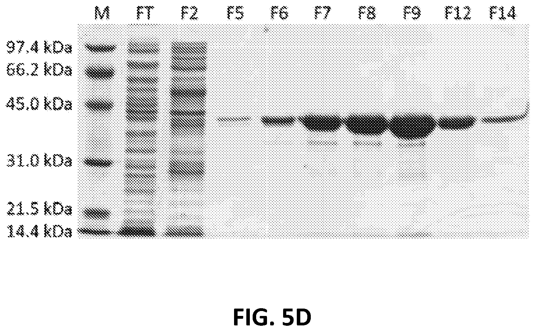

**FIG. 5D** illustrates the SDS-PAGE analysis of the column fractions from nickel affinity chromatography for BAH (SEQ ID NO: 76). M, SDS-PAGE molecular weight markers, low range (Bio-Rad); FT, flow through; F2-F14, eluted fractions from the column.

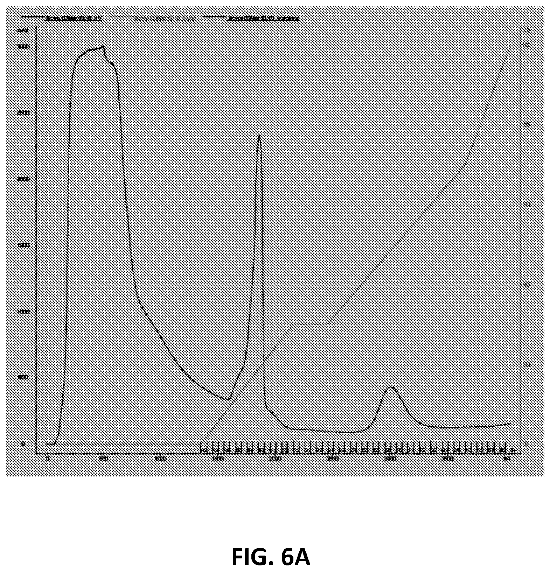

**FIG. 6A** illustrates the chromatogram showing the large scale purification of engineered BCA (S161C)-His (SEQ ID NO: 89) using XK50 nickel affinity column with 196 mL bed volume. Similar to the small scale purification, a four-segment elution gradient was adopted and the elution profile was monitored by absorbance at 280 nm.

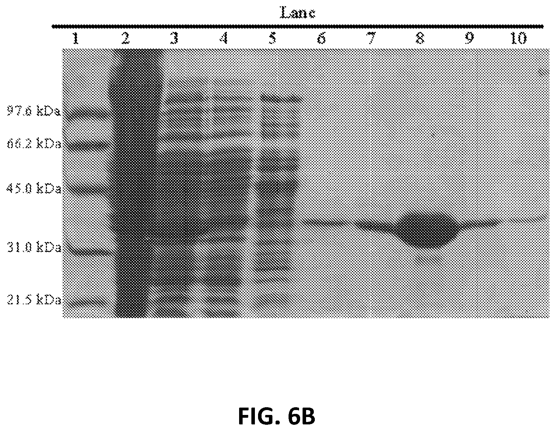

**FIG. 6B** illustrates the SDS-PAGE analysis of the selected fractions from the large scale purification of engineered BCA (S161C)-His (SEQ ID NO: 89). Lane 1 is the low-range molecular weight marker. Lane 2 is the total protein from cell lysate. Lane 3 represents the soluble proteins collected after heat treatment. Lane 4 shows the protein flow through from the XK50 nickel affinity column. Lane 5 is the non-specific bound proteins (Pooled fractions from A6-C3). Lanes 6 to 10 represent fraction E2, E3, F7, G3 and A'5, respectively.

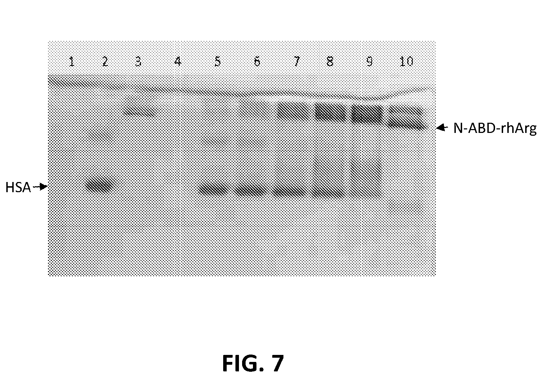

**FIG. 7** illustrates the binding between the N-ABD-rhArg fusion protein (SEQ ID NO: 49) and HSA on non-denaturing PAGE. Thirty picomoles of HSA was mixed with 7.5, 15, 30, 60 and 120 picomoles of N-ABD-rhArg (SEQ ID NO: 49) as shown in Lanes 5-9, respectively. The mobility of 30 picomoles of HSA and N-ABD-rhArg (SEQ ID NO: 49) on the non-denaturing PAGE are shown in Lanes 2 and 3, respectively. Lane 1 is empty or blank. Lane 10 shows 120 picomoles of N-ABD-rhArg (SEQ ID NO: 49).

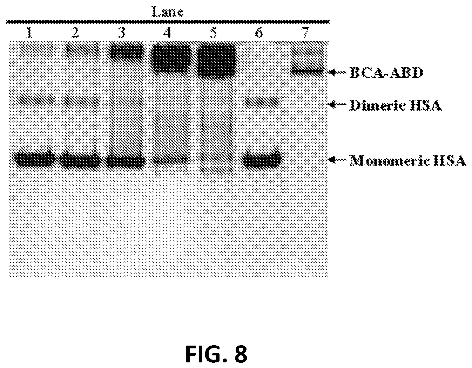

**FIG. 8** illustrates the binding between the BHA fusion protein (SEQ ID NO: 75) and HSA on non-denaturing PAGE. Sixty picomoles of HSA was mixed with 6, 12, 60, 300 and 600 picomoles of BHA fusion protein (SEQ ID NO: 75) as shown in Lanes 1-5, respectively. The mobility of 60 picomoles of HSA and BHA fusion protein (SEQ ID NO: 75) on the non-denaturing PAGE are shown in Lanes 6 and 7, respectively. The band labeled "BCA-ABD" is BHA (SEQ ID NO: 75).

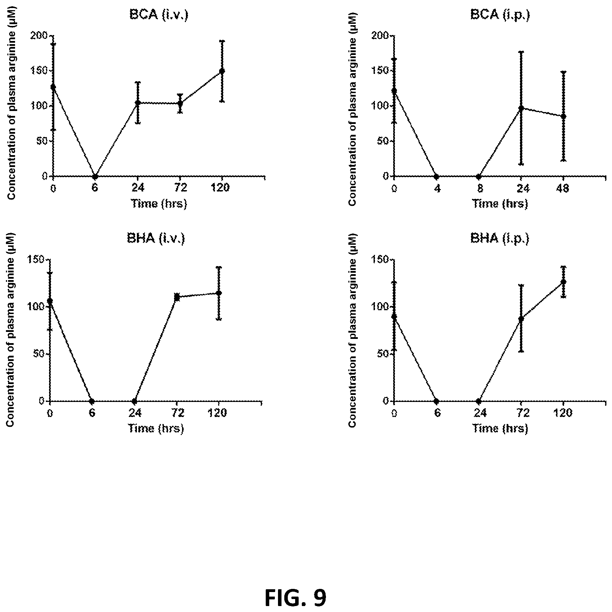

**FIG. 9** illustrates the pharmacodynamics of BCA (S161C)-His (SEQ ID NO: 89) and the BHA fusion protein (SEQ ID NO: 75) on plasma arginine in mice. Each BALB/c mouse was administered via intravenous injection (i.v.) or intraperitoneal injection (i.p.) with 250 U BCA (S161C)-His (SEQ ID NO: 89) or BHA fusion protein (SEQ ID NO: 75). Plasma was collected at the indicated time points after injection. Time 0 refers to the plasma sample collected prior to the injection. The amount of arginine in each sample is determined by the Biochrom 30 Amino Acid Analyzer. Arginine concentrations below the detection limit (3 $\mu$M) are regarded as 0 $\mu$M in these plots. Each point represents mean $\pm$ SD of three mice.

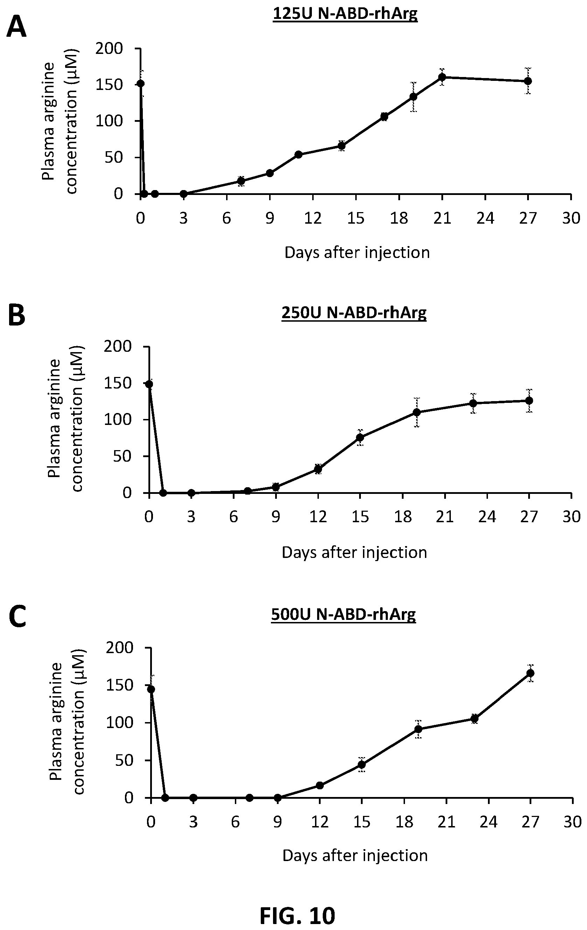

**FIG. 10** illustrates plasma arginine concentrations, measured using the Biochrom 30 Amino Acid Analyzer, of BALB/c mice after receiving intraperitoneal injection of a single dose of **(A)** 125U; **(B)** 250U; and (C) 500U of N-ABD-rhArg (SEQ ID NO: 49). N-ABD-rhArg (SEQ ID NO: 49) was injected at day 0.

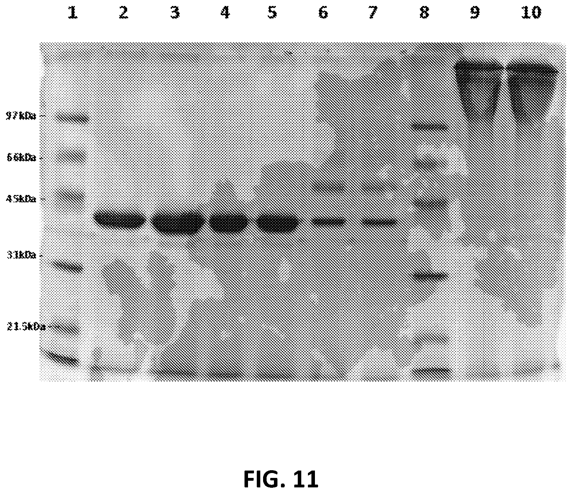

**FIG. 11** illustrates the SDS-PAGE analysis of the production of PEGylated His-rhArg (SEQ ID NO: 101). Lanes 1 and 8: SDS-PAGE low range molecular weight marker (Bio-rad); Lanes 2 and 3: His-rhArg (SEQ ID NO: 101); Lanes 4 and 5: 60% elution; Lanes 6 and 7: 30% elution; Lanes 9 and 10: PEGylated His-rhArg (SEQ ID NO: 101). The molecular weight of the PEG is 5,000. The PEG used is methoxypolyethylene glycol-succinimidyl propionate (mPEG-SPA 5,000). The protein surface lysine residues of His-rhArg (SEQ ID NO: 101) are covalently attached via a propionamidyl (SPA) linker to PEG (5,000 amu) described in U.S. 8,679,810

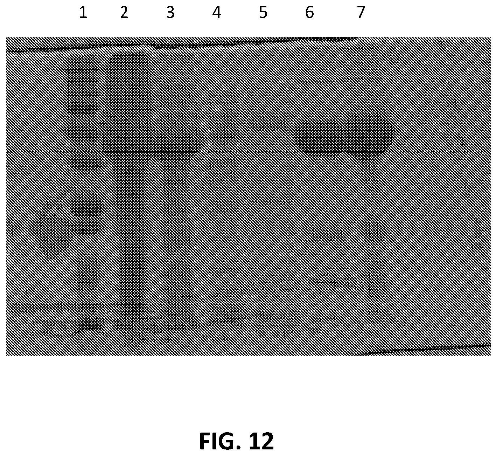

**FIG. 12** illustrates the results of a preparation of N-ABD094-rhArg (SEQ ID NO: 50). Lane 1: SDS-PAGE molecular weight marker; Lane 2: cell lysate; Lane 3: after heat-treatment; Lane 4: flow through; Lane 5: 30% elution; Lane 6: 60% elution; Lane 7: After tangential flow filtration (TFF). The major protein band in lane 6 or lane 7 is the purified N-ABD094-rhArg (SEQ ID NO: 50).

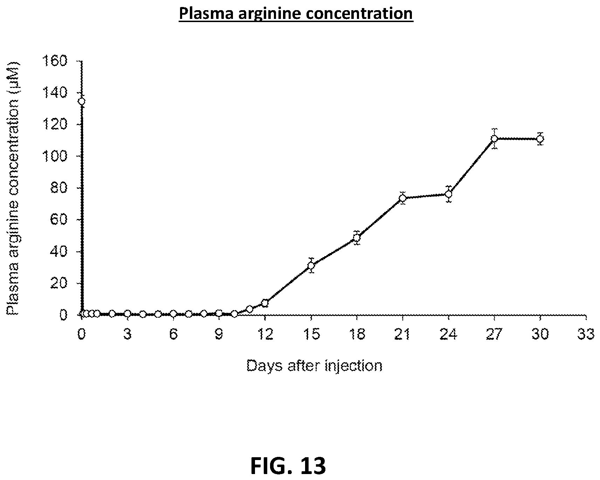

**FIG. 13** illustrates the plasma arginine concentrations in C57BL/6J male mice fed a chow diet, measured using the Agilent 6460 Liquid Chromatography/Electrospray Ionization Triple Quadrupole Mass Spectrometer with a detection limit of 0.3 $\mu$M arginine, at different time points after receiving intraperitoneal injection of a single dose of 500U N-ABD094-rhArg (SEQ ID NO: 50) in saline demonstrated that plasma arginine concentrations can be depleted to < 1 $\mu$M for 10 days. Each time point represents mean $\pm$ SEM of 5 mice.

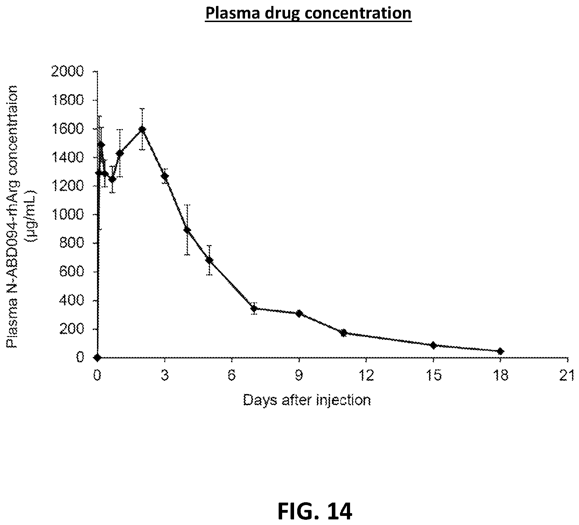

**FIG. 14** illustrates the plasma concentrations of N-ABD094-rhArg (SEQ ID NO: 50) at different time points after being injected into C57BL/6J male mice at a single dose of 500U, measured by ELISA to detect human arginase (n = 5). The circulatory half-life of the drug is around 4 days. Each time point represents mean $\pm$ SEM of 5 mice.

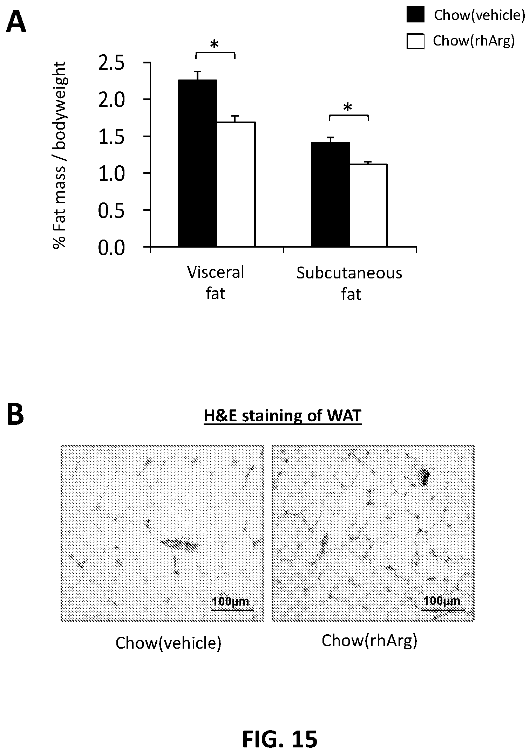

**FIG. 15** illustrates a significant fat loss in normal lean C57BL/6J male mice fed a chow diet and treated with N-ABD094-rhArg (SEQ ID NO: 50) [Chow(rhArg) group]. **(A)** Significant reduction in visceral and subcutaneous fat mass after treatment with 500U N-ABD094-rhArg (SEQ ID NO: 50) at 10-day intervals for 4 weeks in comparison to the vehicle (saline)-treated control [Chow(vehicle) group]. *P < 0.05, independent t-test. Data are expressed as ± SEM, n = 5 for each group. **(B)** Haematoxylin and eosin (H&E) stained paraffin sections of visceral white adipose tissue (WAT) in N-ABD094-rhArg (SEQ ID NO: 50) treated mice showed a prominent reduction in the size of adipocytes.

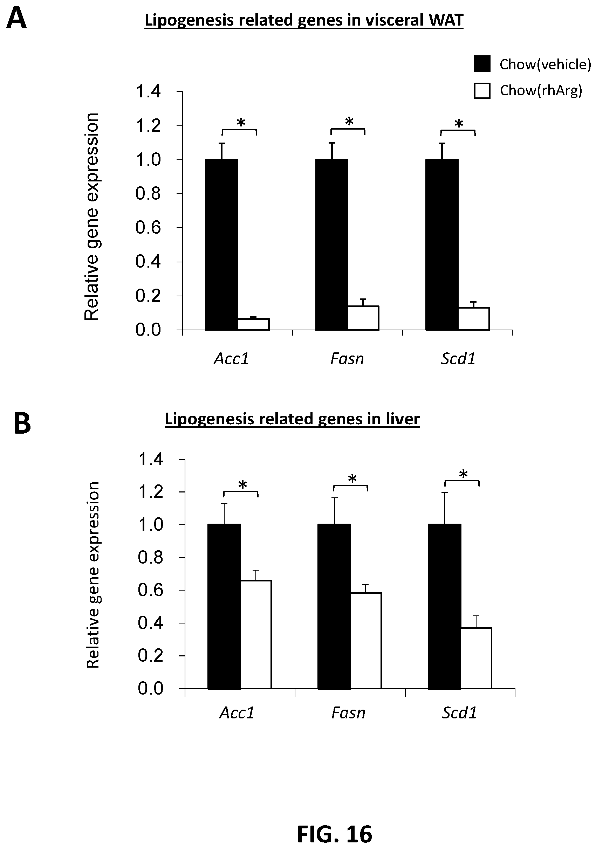

**FIG. 16** illustrates the expression levels of several crucial lipogenesis related genes in various tissues measured using real-time qRT-PCR. Significant downregulation of lipogenesis related genes in **(A)** visceral white adipose tissue (WAT) and **(B)** liver after treatment with 500U N-ABD094-rhArg (SEQ ID NO: 50) [Chow(rhArg) group] at 10-day intervals for 4 weeks in comparison to the vehicle-treated control [Chow(vehicle) group]. Gene expression levels are expressed relative to the vehicle-treated mice [Chow(vehicle) group], which is set as 1.*P < 0.05, independent t-test. Data are expressed as mean ± SEM, n = 5 for each group.

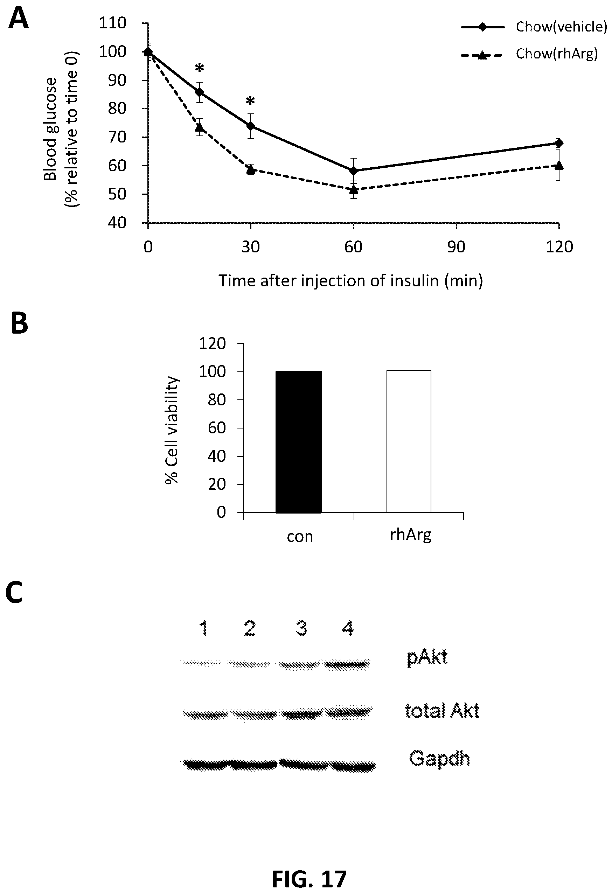

**FIG. 17** shows that treatment with N-ABD094-rhArg (SEQ ID NO: 50) significantly improves insulin sensitivity. **(A)** Insulin tolerance test showed that mice injected with 500U N-ABD094-rhArg (SEQ ID NO: 50) [Chow(rhArg) group] at 10-day intervals demonstrated a significant increase in sensitivity to blood glucose-lowering effect of insulin as soon as 2 weeks after treatment in comparison to the vehicle-treated control [Chow(vehicle) group]. *P < 0.05 vs. Chow(rhArg) group, independent t-test. Data are expressed as mean ± SEM, n = 5 for each group. **(B)** MTT assay showed that treatment of mouse primary hepatocytes with 5U/mL N-ABD094-rhArg (SEQ ID NO: 50) for 48 hr did not affect cell viability in comparison to the control without any treatment (con). **(C)** Western blot analysis of protein levels of phosphorylated Akt (pAkt) (marker of insulin signaling) in mouse primary hepatocytes subjected to arginine depletion with 5U/mL N-ABD094-rhArg (SEQ ID NO: 50) for 24 hr prior to exposure to 100nM insulin for 20 min. Hepatocytes treated with N-ABD094-rhArg (SEQ ID NO: 50) showed a higher level of phosphorylation of Akt upon stimulation by insulin suggested an enhancement in insulin signaling. Lane 1: con; Lane 2: 5U/mL N-ABD094-rhArg (SEQ ID NO: 50); Lane 3: 100nM insulin; Lane 4: 5U/mL N-ABD094-rhArg (SEQ ID NO: 50) + 100nM insulin.

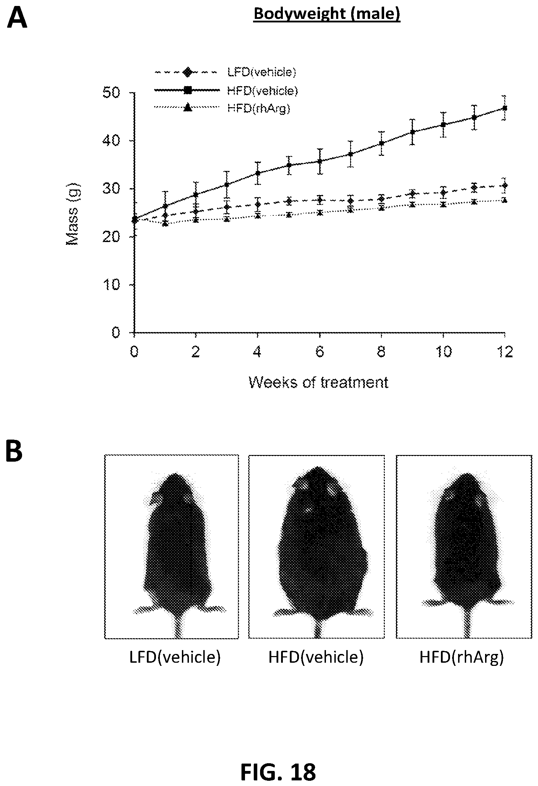

**FIG. 18** illustrates that while vehicle-treated C57BL/6J male mice fed a high fat diet (HFD) containing 60 kcal% fat (from 8 week old for 12 weeks) [HFD(vehicle) group] showed prominent gain in **(A)** bodyweight and **(B)** size, concurrent treatment of male mice fed a HFD (from 8 week old for 12 weeks) with 300U N-ABD094-rhArg (SEQ ID NO: 50) [HFD(rhArg) group] once a week could effectively prevent weight gain. Their bodyweight remained similar to the vehicle-treated lean control mice fed a matched low fat diet (LFD) containing 10 kcal% fat [LFD(vehicle) group]. Data are expressed as mean ± SEM, n = 5 for each group.

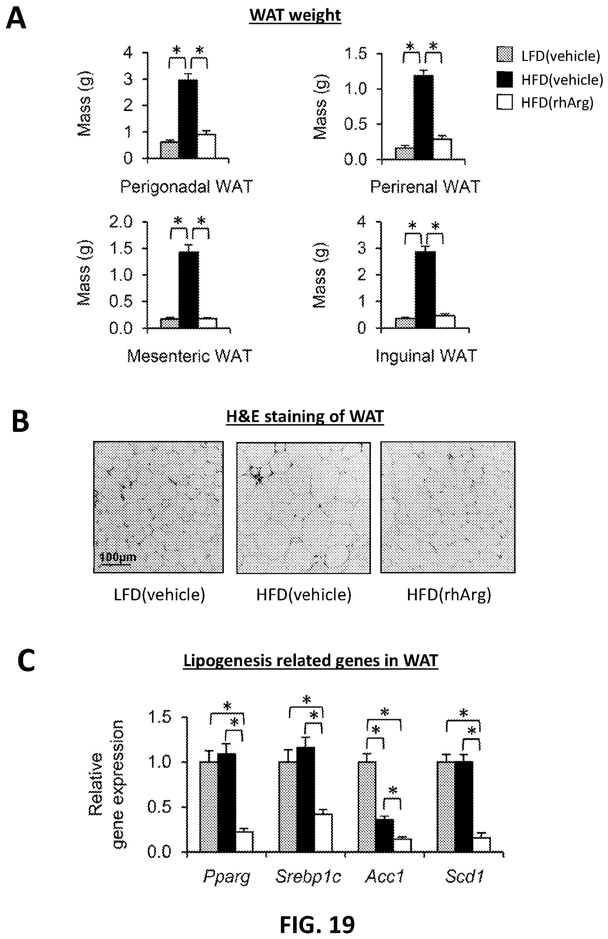

**FIG. 19** illustrates that concurrent treatment of male mice fed a HFD with 300U N-ABD094-rhArg (SEQ ID NO: 50) [HFD(rhArg) group] once a week for 12 weeks could effectively prevent HFD-induced expansion of white adipose tissue (WAT). **(A)** Weight of fat pad at main visceral (perigonadal, perirenal and mesenteric) and subcutaneous (inguinal) depots. **(B)** H&E stained sections of visceral WAT showed that concurrent treatment with N-ABD094-rhArg prevented HFD-induced hypertrophy of white adipocytes. **(C)** Expression levels of several crucial lipogenesis related genes in WAT expressed relative to the lean control [LFD(vehicle) group], which is set as 1. These genes were dramatically downregulated in HFD-fed mice treated with N-ABD094-rhArg. *P < 0.05, one-way ANOVA followed by Fisher's LSD. Data are expressed as mean ± SEM, n = 5 for each group.

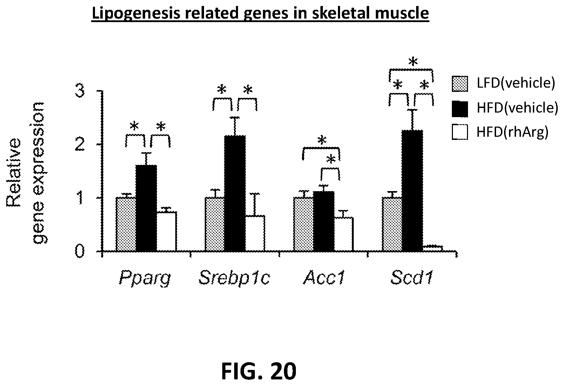

**FIG. 20** illustrates suppression of HFD-induced upregulation of transcriptional regulators of lipogenesis (*Pparg* and *Srebp1c*), with concomitant downregulation of crucial lipogenic enzymes (*Acc1* and *Scd1*) in skeletal muscle of mice treated with 300U N-ABD094-rhArg (SEQ ID NO: 50) [HFD(rhArg) group] once a week for 12 weeks. *P < 0.05, one-way ANOVA followed by Fisher's LSD. Data are expressed as mean ± SEM, n = 5 for each group].

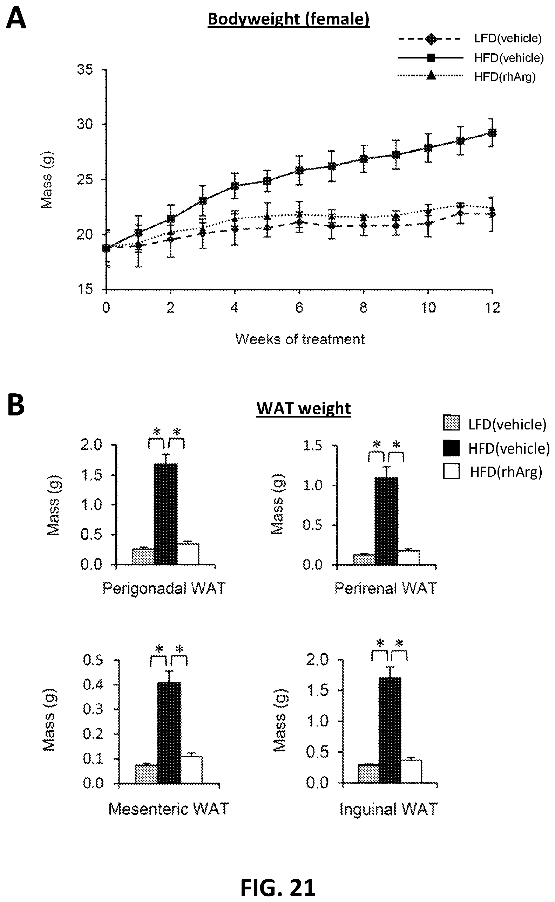

**FIG. 21** illustrates that concurrent treatment with 300U N-ABD094-rhArg (SEQ ID NO: 50) [HFD(rhArg) group] once a week could effectively prevent HFD-induced obesity in C57BL/6J female mice. **(A)** Bodyweight of mice fed a HFD from 8 week old for 12 weeks, and currently treated with N-ABD094-rhArg or vehicle. Vehicle-treated female mice fed a LFD served as the lean control [LFD(vehicle) group]. **(B)** Weight of fat pad at main visceral (perigonadal, perirenal and mesenteric) and subcutaneous (inguinal) depots. *P < 0.05, one-way ANOVA followed by Fisher's LSD. Data are expressed as mean ± SEM, n = 5 for each group.

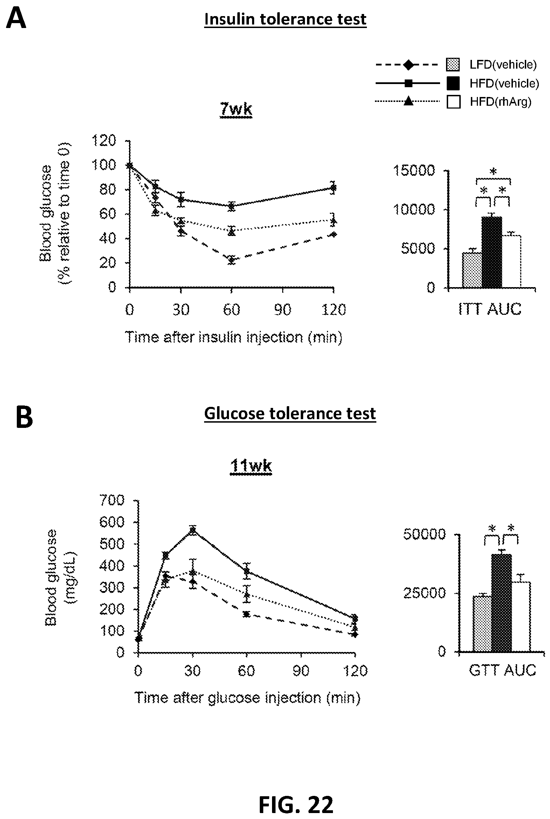

**FIG. 22** illustrates that concurrent treatment with 300U N-ABD094-rhArg (SEQ ID NO: 50) [HFD(rhArg) group] once a week could effectively prevent HFD-induced insulin resistance and glucose intolerance in C57BL/6J female mice. **(A)** Insulin tolerance test (ITT) conducted at 7 week after feeding a HFD. **(B)** Glucose tolerance test (GTT) conducted at 11 week after feeding a HFD. ITT and GTT results are expressed as area under the curve (AUC). *P < 0.05, one-way ANOVA followed by Fisher's LSD. Data are expressed as mean ± SEM, n = 5 for each group.

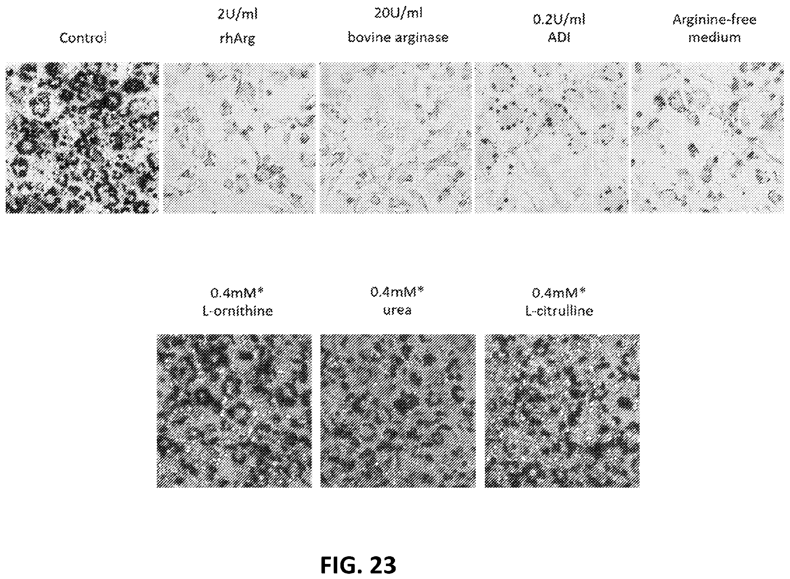

**FIG. 23** illustrates that arginine depletion by adding N-ABD094-rhArg (SEQ ID NO: 50) (rhArg), bovine arginase or native arginine deiminase (ADI) to the culture medium, or using arginine-free medium inhibited the differentiation of mouse 3T3-L1 preadipocytes into adipocytes. Addition of arginase metabolic product L-ornithine or urea, or ADI metabolic product L-citrulline to the culture medium did not have inhibitory effects on adipogenesis and lipogenesis. (*The culture medium contains 0.4mM L-arginine and can be converted into 0.4mM L-ornithine and 0.4mM urea by arginase, or 0.4mM L-citrulline by ADI.). Cells grown in culture medium without addition of arginine depleting agents or metabolic products of arginase/ADI served as the control. Lipids in cells were stained with oil red O at 6 days after exposure to specific differentiation-inducing factors added to the medium.

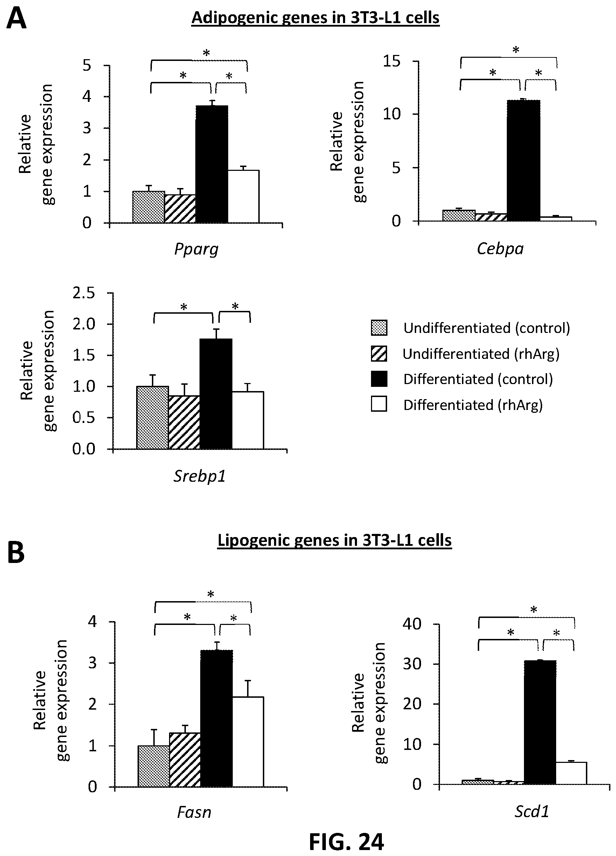

**FIG. 24** illustrates that N-ABD094-rhArg (SEQ ID NO: 50) could suppress adipogenic and lipogenic genes in 3T3-L1 preadipocytes. Mouse 3T3-L1 preadipocytes were cultured for 6 days in medium supplemented with specific factors to induce differentiation into adipocytes (differentiated). Cells grown in medium without these factors remained undifferentiated. The medium was added with 2U/mL N-ABD094-rhArg (SEQ ID NO: 50) (rhArg). Cells grown in medium without the addition of N-ABD094-rhArg served as the control. The mRNA expression levels of several crucial **(A)** transcriptional regulators of adipogenesis, and **(B)** enzymes in lipogenic pathways were determined by real-time qRT-PCR, and expressed relative to the undifferentiated (control) group, which is set as 1. *$P < 0.05$, one-way ANOVA followed by Fisher's LSD. Data are expressed as mean $\pm$ SEM, n = 3 for each group.

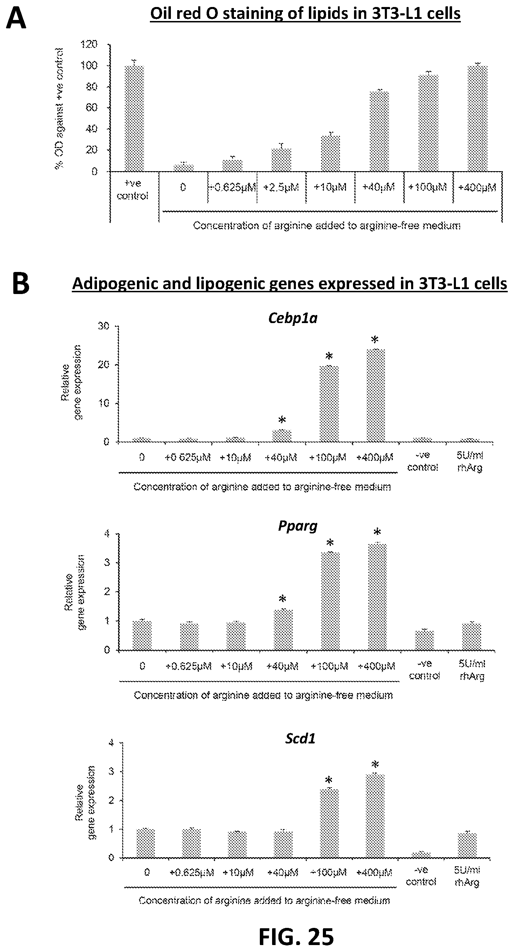

**FIG. 25** illustrates the response of 3T3-L1 preadipocytes to various concentrations of arginine. Mouse 3T3-L1 preadipocytes were cultured in arginine-free medium with or without addition of varying concentrations of L-arginine for 6 days. Cells cultured in normal arginine-containing medium supplemented with differentiation inducing factors served as the positive (+ve) control of differentiation, while cells cultured in normal arginine-containing medium without supplementation of differentiation inducing factors served as the negative (-ve) control of differentiation. One group of cells was cultured in normal arginine-containing medium supplemented with differentiation inducing factors and treated with 5U/mL N-ABD094-rhArg (SEQ ID NO: 50) (rhArg) to inhibit differentiation. **(A)** Cells were stained for lipids with oil red O, and staining intensity was quantified by measuring optical density, which is expressed as the percentage against the +ve control group. **(B)** The mRNA levels of several crucial lipogenesis related genes are expressed relative to cells cultured in arginine-free medium, which is set as 1. Data are expressed as mean $\pm$ SEM, n = 3 for each group.

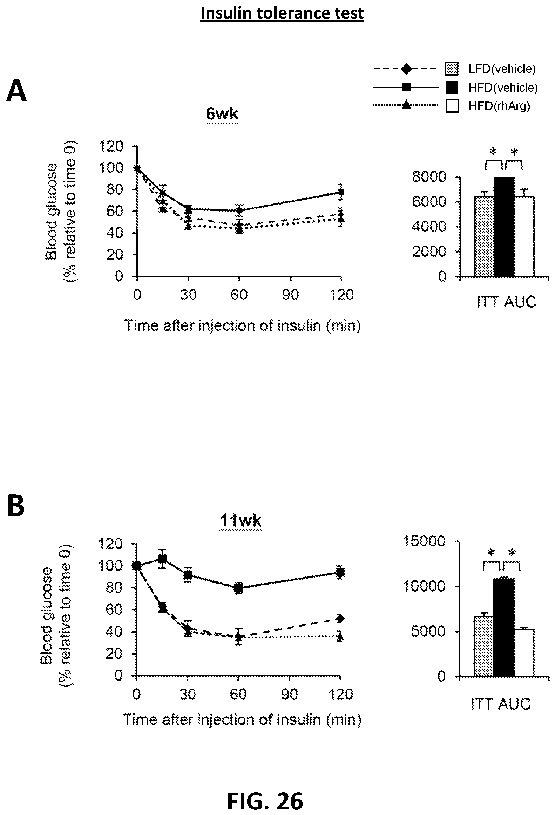

**FIG. 26** illustrates that concurrent treatment of male mice fed a HFD with 300U N-ABD094-rhArg (SEQ ID NO: 50) [HFD(rhArg) group] once a week could effectively prevent HFD-induced insulin resistance. Insulin tolerance test (ITT) was conducted at **(A)** 6 week and **(B)** 11 week after feeding a HFD, and results are expressed as area under the curve (AUC). *$P < 0.05$, one-way ANOVA followed by Fisher's LSD. Data are expressed as mean $\pm$ SEM, n = 5 for each group.

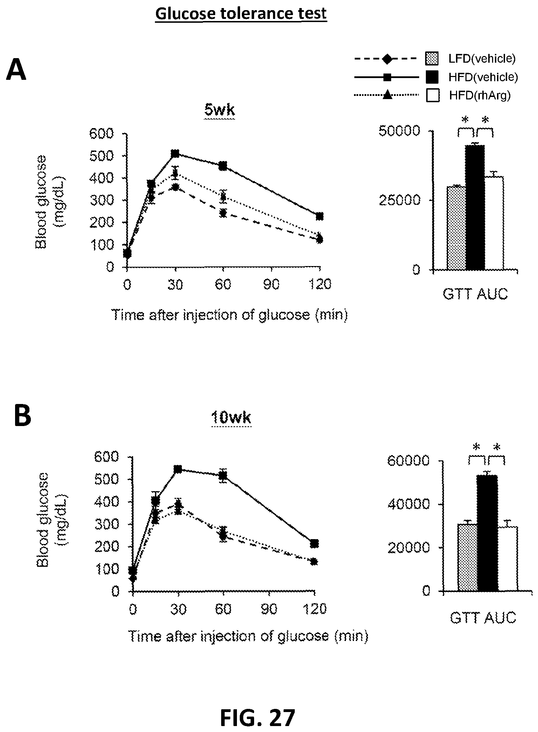

**FIG. 27** illustrates that concurrent treatment of male mice fed a HFD with 300U N-ABD094-rhArg (SEQ ID NO: 50) [HFD(rhArg) group] once a week could effectively prevent HFD-induced glucose intolerance. Glucose tolerance test (GTT) was conducted at **(A)** 5 week and **(B)** 10 week after feeding a HFD, and results are expressed as area under the curve (AUC). *$P < 0.05$, one-way ANOVA followed by Fisher's LSD. Data are expressed as mean $\pm$ SEM, n = 5 for each group.

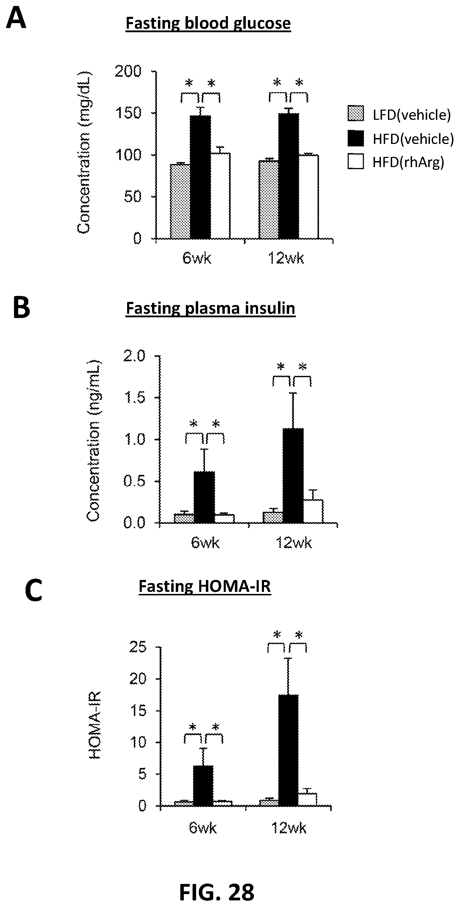

**FIG. 28** illustrates that concurrent treatment of male mice fed a HFD with 300U N-ABD094-rhArg (SEQ ID NO: 50) [HFD(rhArg) group] once a week could effectively prevent HFD-induced elevation of fasting **(A)** blood glucose measured using a glucometer, and **(B)** plasma concentrations of insulin measured using ELISA. **(C)** HOMA-IR score, calculated by standard formula, as a measurement of insulin resistance showed that concurrent treatment with N-ABD094-rhArg prevented the development of HFD-induced insulin resistance. *$P < 0.05$, one-way ANOVA followed by Fisher's LSD. Data are expressed as mean $\pm$ SEM, n = 5 for each group.

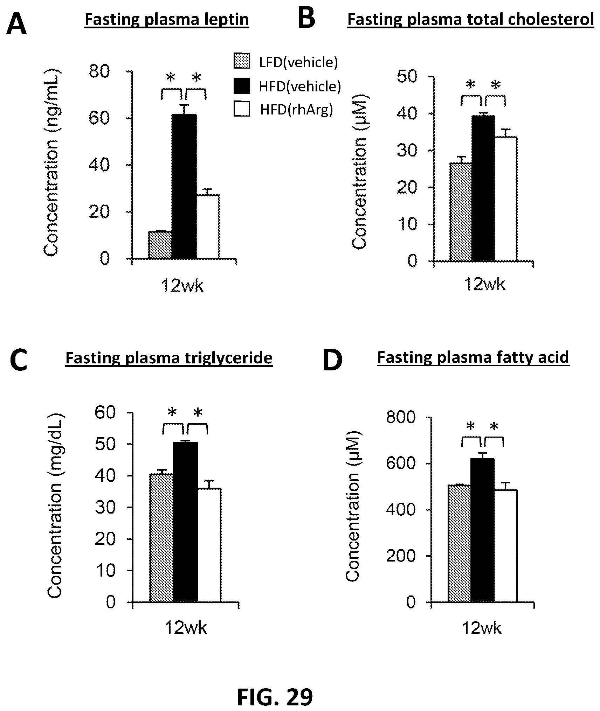

**FIG. 29** illustrates that concurrent treatment of male mice fed a HFD with 300U N-ABD094-rhArg (SEQ ID NO: 50) [HFD(rhArg) group] once a week prevented/ameliorated HFD-induced elevation of fasting plasma **(A)** leptin, **(B)** total cholesterol, **(C)** triglyceride, and **(D)** free fatty acids levels. *$P < 0.05$, one-way ANOVA followed by Fisher's LSD. Data are expressed as mean $\pm$ SEM, n = 5 for each group.

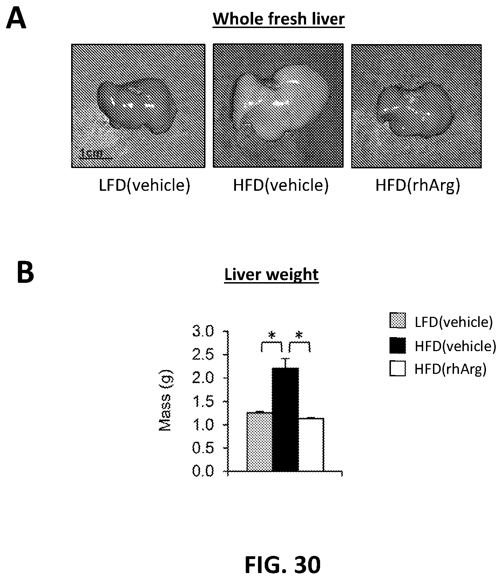

**FIG. 30** illustrates that concurrent treatment of male mice fed a HFD with 300U N-ABD094-rhArg (SEQ ID NO: 50) [HFD(rhArg) group] once a week for 12 weeks prevented HFD-induced enlargement of liver. **(A)** Representative images of whole fresh liver. **(B)** Weight of liver. *$P < 0.05$, one-way ANOVA followed by Fisher's LSD. Data are expressed as mean $\pm$ SEM, n = 5 or each group.

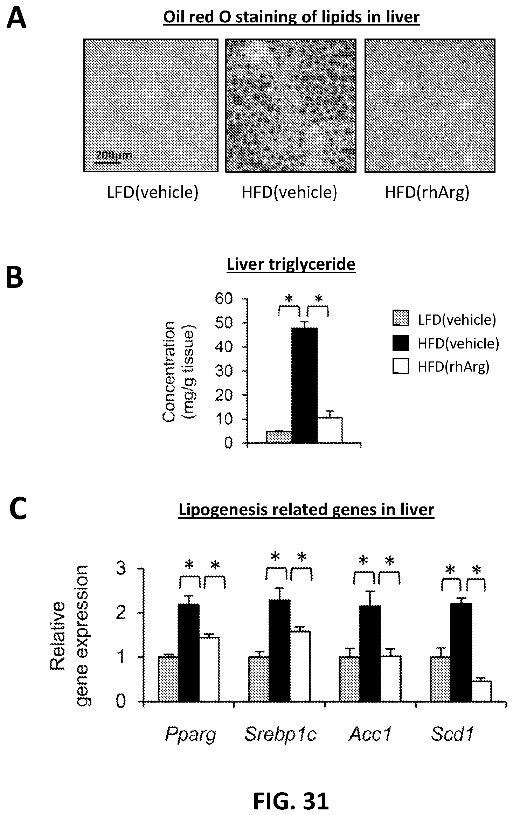

**FIG. 31** illustrates that concurrent treatment of male mice fed a HFD with 300U N-ABD094-rhArg (SEQ ID NO: 50) [HFD(rhArg) group] once a week for 12 weeks could effectively prevent HFD-induced hepatic steatosis. **(A)** Staining of liver sections with oil red O showed extensive accumulation of lipids in vehicle-treated mice fed a HFD [HFD(vehicle) group], which was absent in mice treated with N-ABD094-rhArg. **(B)** N-ABD094-rhArg treatment prevented elevation in triglyceride concentrations in the liver of HFD-fed mice. **(C)** N-ABD094-rhArg suppressed HFD-induced upregulation of lipogenesis related genes in the liver. *$P < 0.05$, one-way ANOVA followed by Fisher's LSD. Data are expressed as mean $\pm$ SEM, n = 5 for each group.

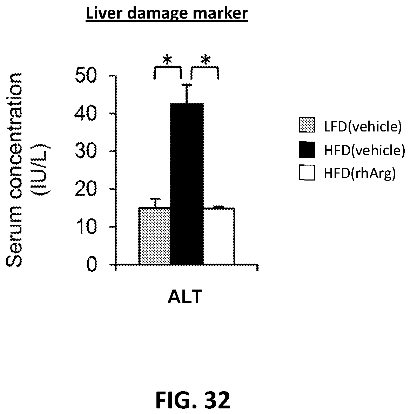

**FIG. 32** illustrates that concurrent treatment of male mice fed a HFD with 300U N-ABD094-rhArg (SEQ ID NO: 50) [HFD(rhArg) group] once a week for 12 weeks prevented HFD-induced elevation of serum levels of alanine aminotransferase (ALT) commonly measured as biomarker for liver damage. *$P < 0.05$, one-way ANOVA followed by Fisher's LSD. Data are expressed as mean $\pm$ SEM, n = 5 for each group.

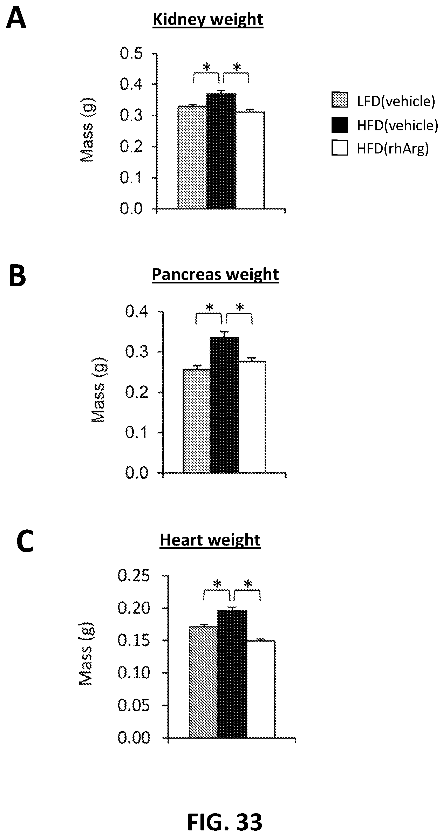

**FIG. 33** illustrates that concurrent treatment of male mice fed a HFD with 300U N-ABD094-rhArg (SEQ ID NO: 50) [HFD(rhArg) group] once a week for 12 weeks prevented HFD-induced increase in the weight of **(A)** kidney, **(B)** pancreas, and **(C)** heart. *$P < 0.05$, one-way ANOVA followed by Fisher's LSD. Data are expressed as mean $\pm$ SEM, n = 5 for each group.

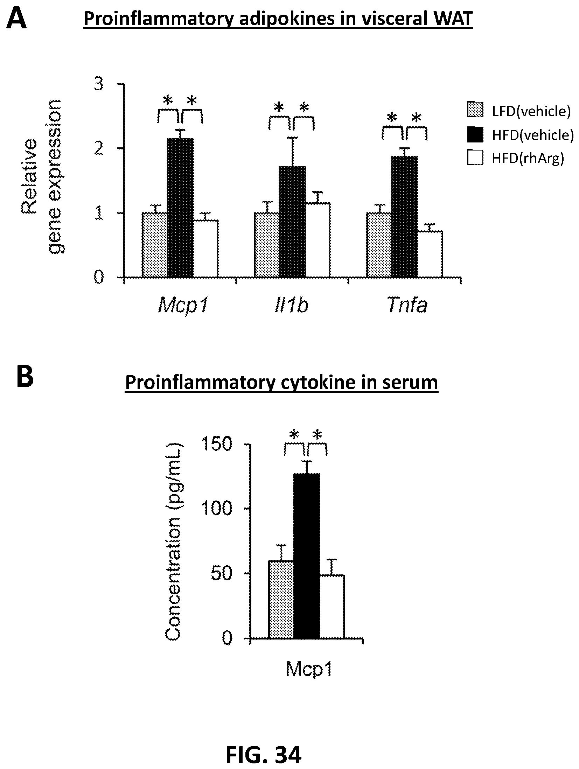

**FIG. 34** illustrates that concurrent treatment of male mice with 300U N-ABD094-rhArg (SEQ ID NO: 50) [HFD(rhArg) group] once a week for 12 weeks could effectively **(A)** suppress HFD-induced upregulation of mRNA levels of proinflammatory adipokines in visceral WAT, expressed relative to the lean control mice [LFD(vehicle) group], which is set as 1, and **(B)** suppress HFD-induced elevation in serum levels of proinflammatory cytokine. *$P < 0.05$, one-way ANOVA followed by Fisher's LSD. Data are expressed as mean $\pm$ SEM, n = 5 for each group.

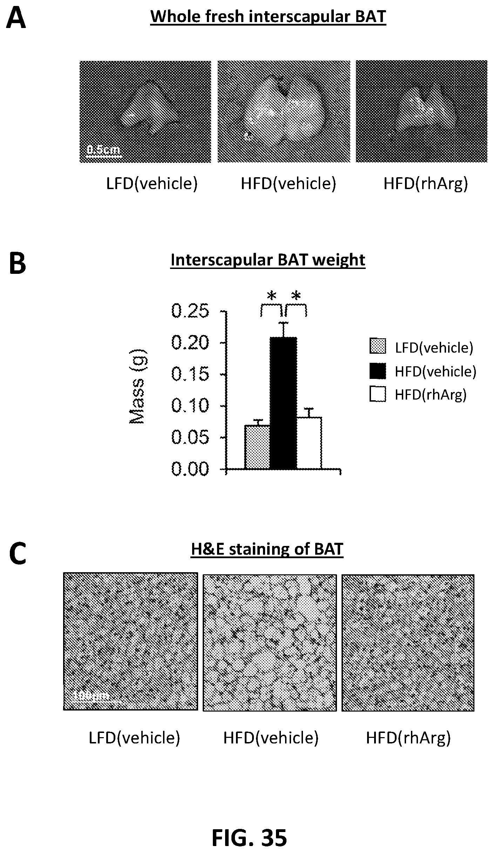

**FIG. 35** illustrates that concurrent treatment of male mice with 300U N-ABD094-rhArg (SEQ ID NO: 50) [HFD(rhArg) group] once a week for 12 weeks could effectively prevent HFD-induced whitening of brown adipose tissue (BAT). **(A)** Representative images of whole fresh interscapular BAT. **(B)** Weight of the interscapular BAT. **(C)** H&E stained paraffin sections revealed that cells with a large single hole (lipid globule), which are typical histological features of white adipocytes, were plentiful in BAT of HFD-fed mice treated with vehicle [HFD(vehicle) group], but were rarely found in BAT of HFD-fed mice treated with N-ABD094-rhArg. *$P < 0.05$, one-way ANOVA followed by Fisher's LSD. Data are expressed as mean $\pm$ SEM, n = 5 for each group.

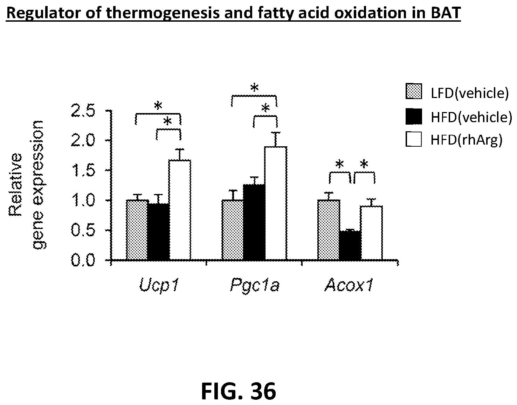

**FIG. 36** illustrates that concurrent treatment of male mice with 300U N-ABD094-rhArg (SEQ ID NO: 50) [HFD(rhArg) group] once a week for 12 weeks significantly upregulated the mRNA levels of key regulators of thermogenesis (*Ucp1* and *Pgc1a*) in BAT, and suppressed the HFD-induced downregulation of fatty acid oxidation gene *Acox1* in BAT. *$P < 0.05$, one-way ANOVA followed by Fisher's LSD. Data are expressed as mean $\pm$ SEM, n = 5 for each group.

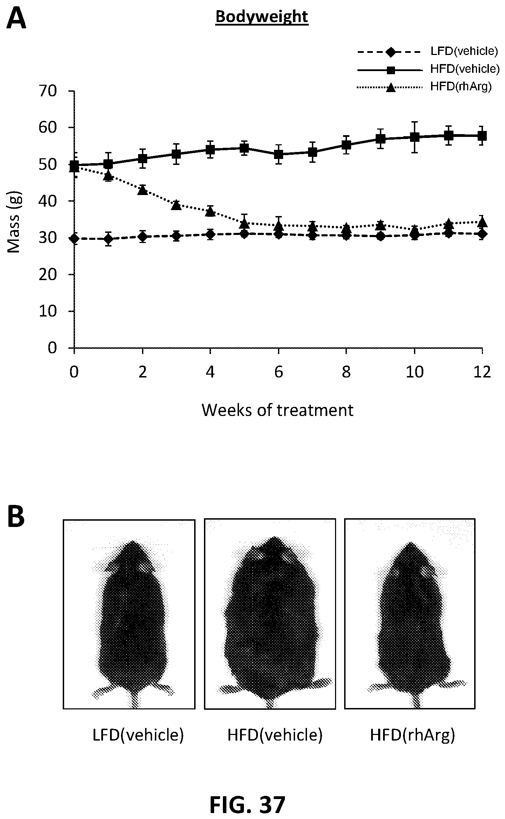

**FIG. 37** illustrates that C57BL/6J male mice with pre-existing obesity induced by feeding a HFD from 5 week old for 12 weeks showed prominent reduction in bodyweight after treatment with N-ABD094-rhArg (SEQ ID NO: 50). **(A)** Bodyweight of mice treated with 600U N-ABD094-rhArg [HFD(rhArg) group] once a week for 12 weeks. **(B)** Representative images of mice after 12 weeks of treatment. Data are expressed as mean $\pm$ SEM, n = 5 for each group.

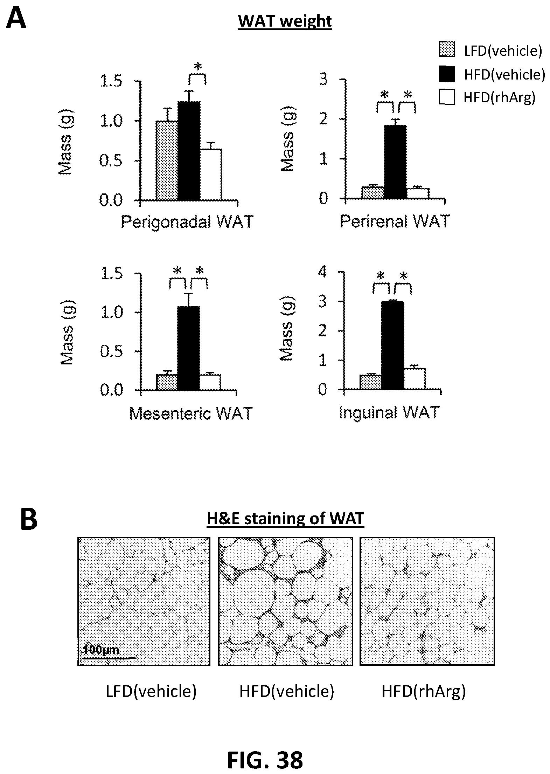

**FIG. 38** illustrates that administration of 600U N-ABD094-rhArg (SEQ ID NO: 50) once a week for 12 weeks to male mice with pre-existing HFD-induced obesity [HFD(rhArg) group] could effectively reduce white adipose tissue. **(A)** Weight of fat pad at main visceral (perigonadal, perirenal and mesenteric) and subcutaneous (inguinal) depots of obese mice was highly similar to lean control mice [LFD(rhArg) group] after treatment with N-ABD094-rhArg. *$P < 0.05$, one-way ANOVA followed by Fisher's LSD. Data are expressed as mean $\pm$ SEM, n = 5 for each group. **(B)** H& E stained sections of visceral WAT showed that treatment with N-ABD094-rhArg markedly reduced adipocyte cell size of obese mice.

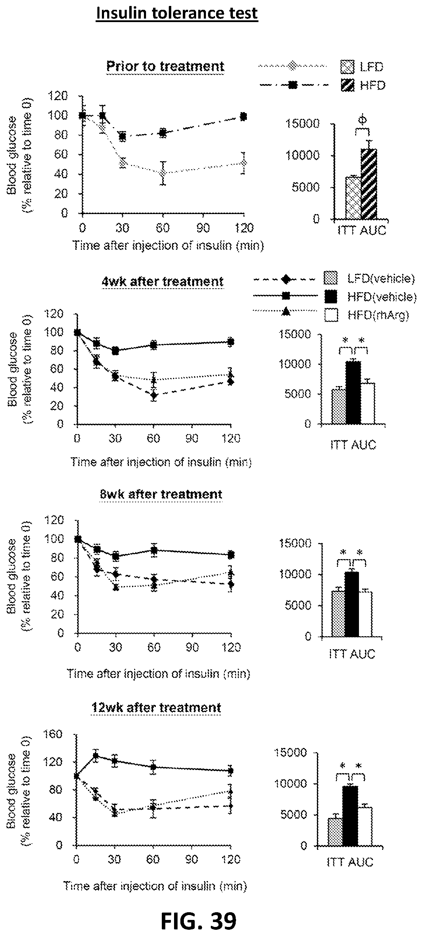

**FIG. 39** illustrates that administration of 600U N-ABD094-rhArg (SEQ ID NO: 50) once a week to male mice with pre-existing HFD-induced obesity [HFD(rhArg) group] could effectively reverse insulin resistance. Insulin tolerance test (ITT) was conducted prior to and at 4, 8 and 12 weeks after treatment with N-ABD094-rhArg. Results of ITT are expressed as area under the curve (AUC). *$P < 0.05$, one-way ANOVA followed by Fisher's LSD. Data are expressed as mean $\pm$ SEM, n = 5 for each group.

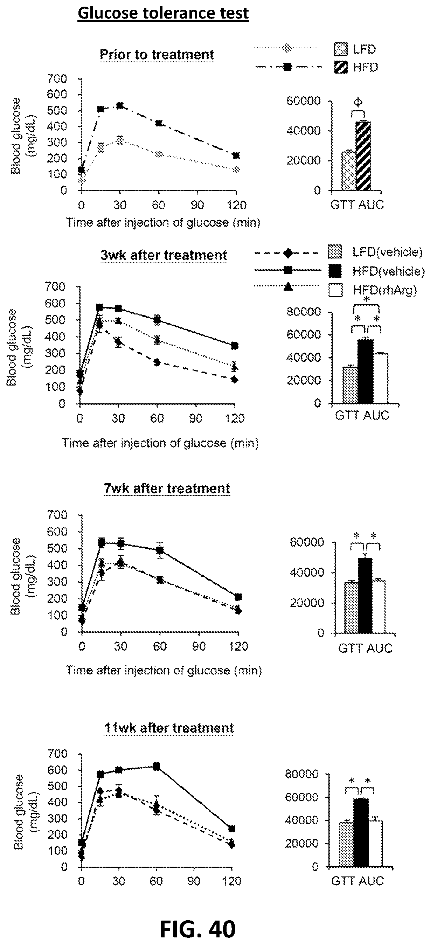

**FIG. 40** illustrates that administration of 600U N-ABD094-rhArg (SEQ ID NO: 50) once a week to male mice with pre-existing HFD-induced obesity [HFD(rhArg) group] could effectively reverse glucose intolerance. Glucose tolerance test (GTT) was conducted prior to and at 3, 7 and 11 weeks after treatment with N-ABD094-rhArg. Results of GTT are expressed as area under the curve (AUC). *$P < 0.05$, one-way ANOVA followed by Fisher's LSD. Data are expressed as mean $\pm$ SEM, n = 5 for each group.

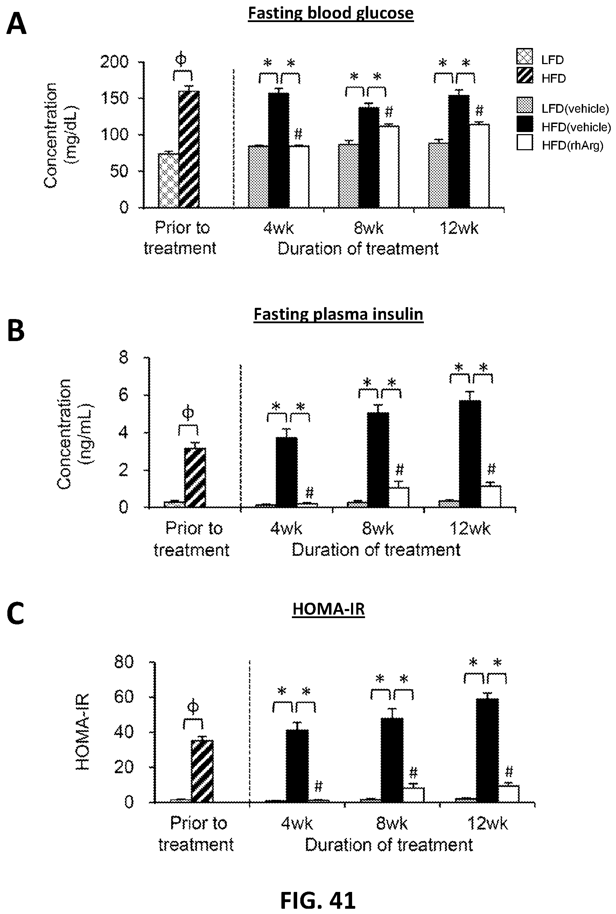

**FIG. 41** illustrates that administration of 600U N-ABD094-rhArg (SEQ ID NO: 50) once a week to male mice with pre-existing HFD-induced obesity [HFD(rhArg) group] could effectively reverse hyperglycemia, hyperinsulinemia and insulin resistance. **(A)** Fasting blood glucose, **(B)** fasting plasma insulin and **(C)** HOMA-IR score of obese mice were corrected to near-normal levels following treatment with N-ABD094-rhArg. *$P < 0.05$, one-way ANOVA followed by Fisher's LSD. Data are expressed as mean $\pm$ SEM, n = 5 for each group.

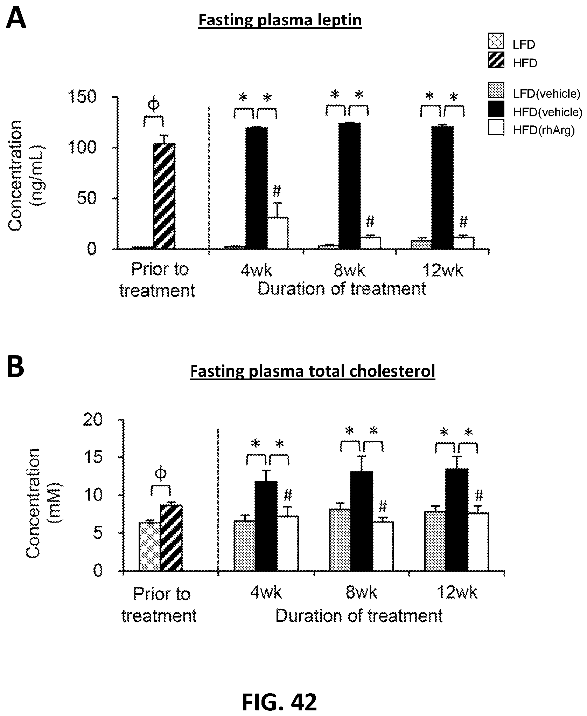

**FIG. 42** illustrates that administration of 600U N-ABD094-rhArg (SEQ ID NO: 50) once a week to male mice with pre-existing HFD-induced obesity [HFD(rhArg) group] could effectively reverse hyperleptinemia and hypercholesterolemia. **(A)** Fasting plasma leptin, and **(B)** fasting plasma total cholesterol of obese mice were corrected to near-normal levels following treatment with N-ABD094-rhArg. *$P < 0.05$, one-way ANOVA followed by Fisher's LSD. Data are expressed as mean $\pm$ SEM, n = 5 for each group.

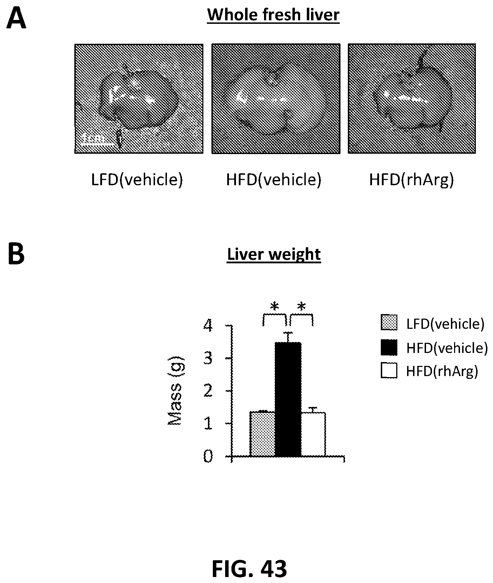

**FIG. 43** illustrates that administration of 600U N-ABD094-rhArg (SEQ ID NO: 50) once a week for 12 weeks to male mice with pre-existing HFD-induced obesity [HFD(rhArg) group] could effectively reverse enlargement of liver. **(A)** Representative images of whole fresh liver showed that the liver of obese mice had returned to normal size following treatment of N-ABD094-rhArg. **(B)** The liver weight of obese mice was dramatically reduced following treatment with N-ABD094-rhArg and was similar to the lean control mice [LFD(rhArg) group]. *$P < 0.05$, one-way ANOVA followed by Fisher's LSD. Data are expressed as mean $\pm$ SEM, n = 5 for each group.

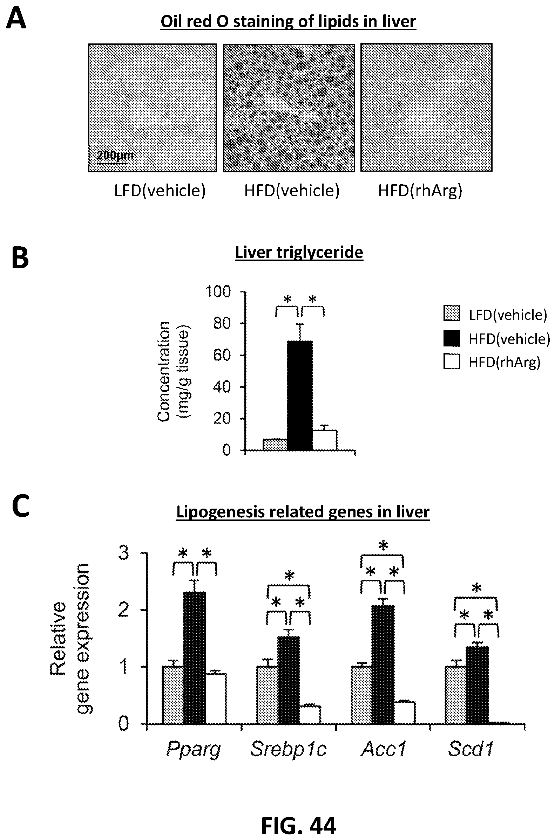

**FIG. 44** illustrates that administration of 600U N-ABD094-rhArg (SEQ ID NO: 50) once a week for 12 weeks to male mice with pre-existing HFD-induced obesity [HFD(rhArg) group] could effectively reverse hepatic steatosis. In the liver of obese mice treated with N-ABD094-rhArg, **(A)** oil red O staining of liver sections showed a dramatic clearance of lipids, and **(B)** triglyceride concentrations were reduced to levels comparable with the lean control mice [LFD(vehicle)], and **(C)** the mRNA levels of several crucial lipogenesis related genes were significantly downregulated The expression levels are expressed relative to the lean control, which is set as 1. *P < 0.05, one-way ANOVA followed by Fisher's LSD. Data are expressed as mean $\pm$ SEM, n = 5 for each group.

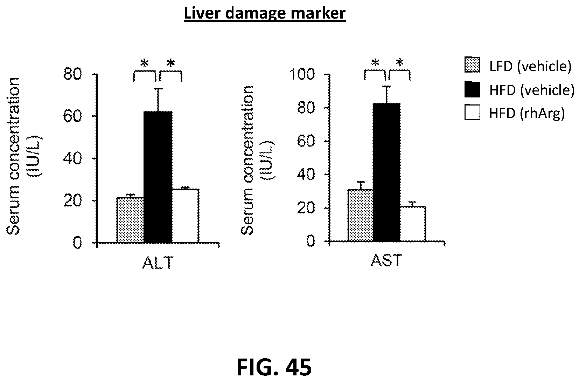

**FIG. 45** illustrates that administration of 600U N-ABD094-rhArg (SEQ ID NO: 50) once a week for 12 weeks to male mice with pre-existing HFD-induced obesity [HFD(rhArg) group] could significantly reduce serum levels of alanine transaminase (ALT) and aspartate transaminase (AST), which are common biomarkers of liver damage. *P < 0.05, one-way ANOVA followed by Fisher's LSD. Data are expressed as mean $\pm$ SEM, n = 5 for each group.

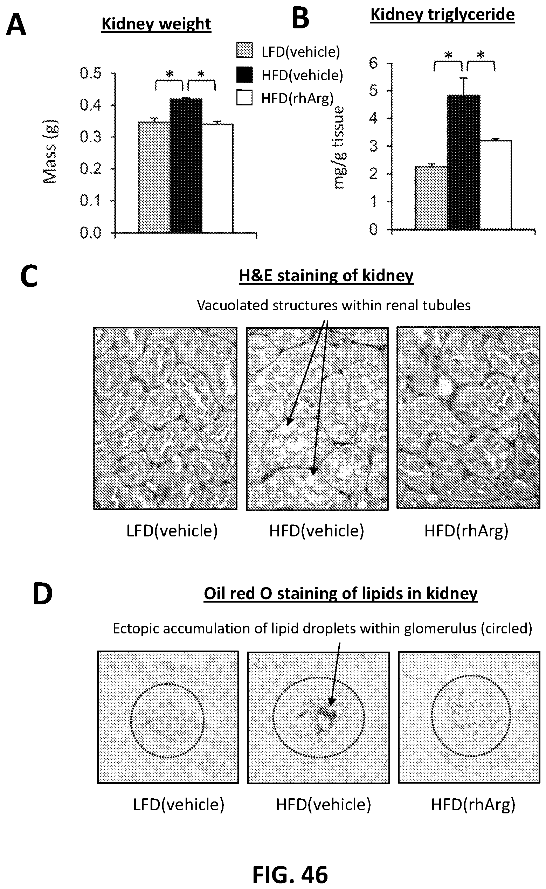

**FIG. 46** illustrates that administration of 600U N-ABD094-rhArg (SEQ ID NO: 50) once a week for 12 weeks to male mice with pre-existing HFD-induced obesity [HFD(rhArg) group] could reverse renal steatosis. In obese mice treated with N-ABD094-rhArg: **(A)** the weight of the kidney; **(B)** triglyceride concentrations in the kidney; **(C)** vacuolated structures in the renal tubules of H&E-stained kidney sections, and **(D)** ectopic accumulation of lipid droplets in the glomeruli of oil red O-stained kidney sections were prominently reduced. *P < 0.05, one-way ANOVA followed by Fisher's LSD. Data are expressed as mean $\pm$ SEM, n = 5 for each group.

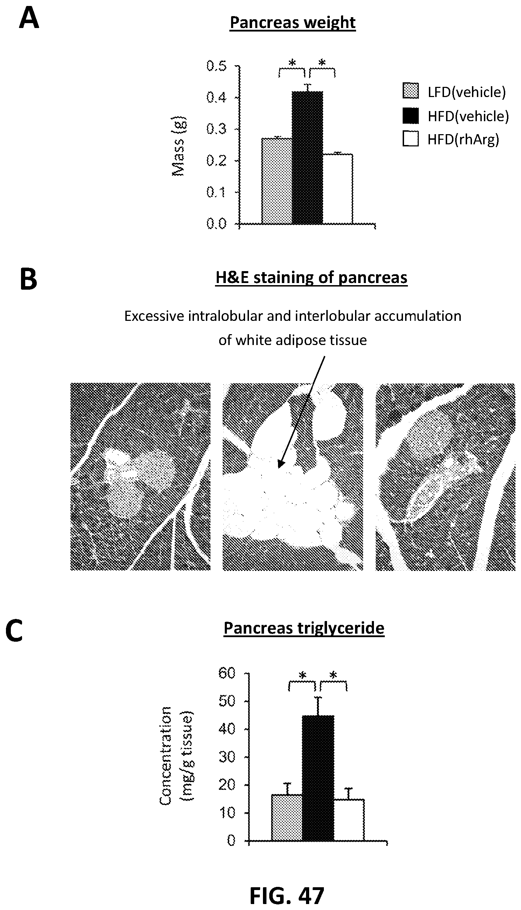

**FIG. 47** illustrates that administration of 600U N-ABD094-rhArg (SEQ ID NO: 50) once a week for 12 weeks to male mice with pre-existing HFD-induced obesity [HFD(rhArg) group] could reverse pancreatic steatosis. In obese mice treated with N-ABD094-rhArg: **(A)** the weight of the pancreas; **(B)** excessive interlobular and intralobular accumulation of white adipose tissue shown in H&E-stained sections of the pancreas; and **(C)** triglyceride concentrations in the pancreas were prominently reduced. *P < 0.05, one-way ANOVA followed by Fisher's LSD. Data are expressed as mean $\pm$ SEM, n = 5 for each group.

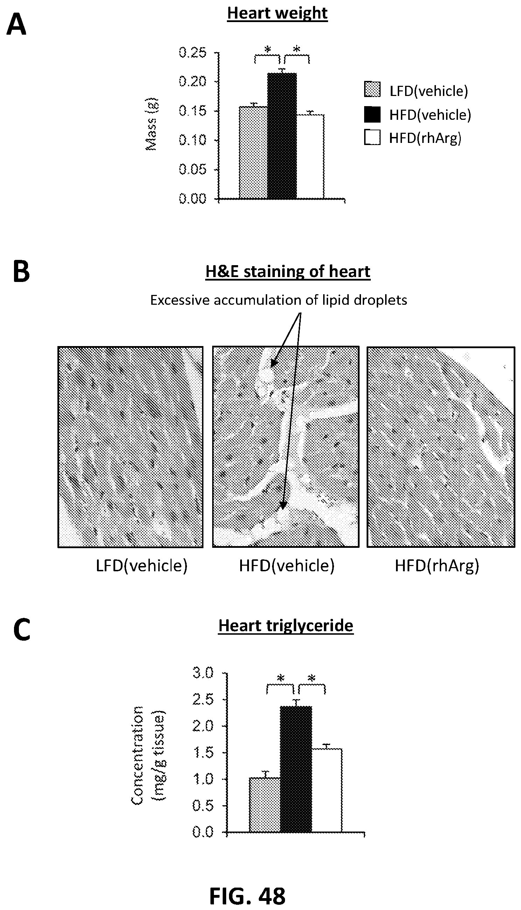

**FIG. 48** illustrates that administration of 600U N-ABD094-rhArg (SEQ ID NO: 50) once a week for 12 weeks to male mice with pre-existing HFD-induced obesity [HFD(rhArg) group] could reverse cardiac steatosis. In obese mice treated with N-ABD094-rhArg: **(A)** the weight of the heart; **(B)** excessive accumulation of lipid droplets in the heart; and **(C)** triglyceride concentrations in the heart were prominently reduced. *P < 0.05, one-way ANOVA followed by Fisher's LSD. Data are expressed as mean $\pm$ SEM, n = 5 for each group.

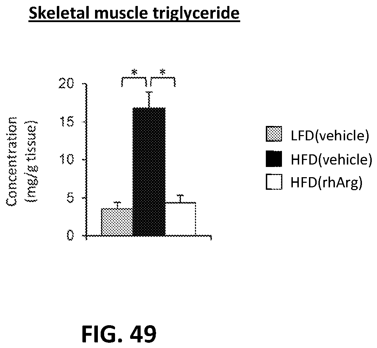

**FIG. 49** illustrates that administration of 600U N-ABD094-rhArg (SEQ ID NO: 50) once a week for 12 weeks to male mice with pre-existing HFD-induced obesity [HFD(rhArg) group] could significantly reduce triglyceride concentrations in the skeletal muscle. *P < 0.05, one-way ANOVA followed by Fisher's LSD. Data are expressed as mean $\pm$ SEM, n = 5 for each group.

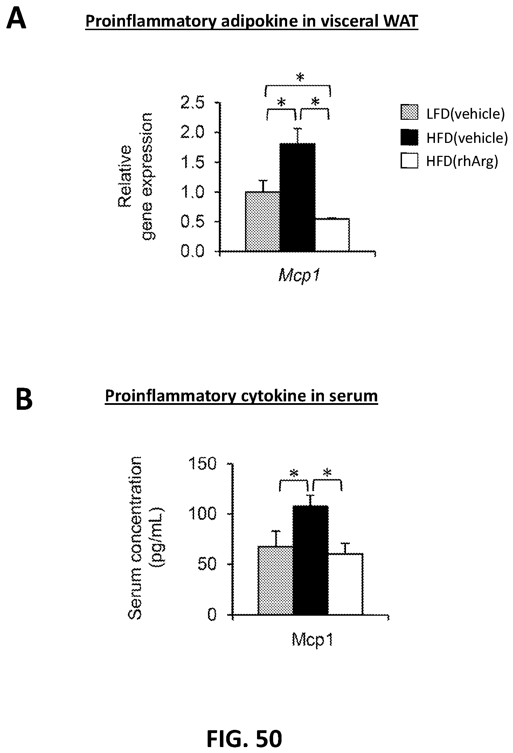

**FIG. 50** illustrates that administration of 600U N-ABD094-rhArg (SEQ ID NO: 50) once a week for 12 weeks to male mice with pre-existing HFD-induced obesity [HFD(rhArg) group] could significantly **(A)** downregulate the mRNA levels of proinflammatory adipokine *Mcp1* in visceral WAT, expressed relative to the lean control mice [LFD(vehicle) group], which is set as 1, and **(B)** reduce the serum concentration of proinflammatory cytokine Mcp1. *P < 0.05, one-way ANOVA followed by Fisher's LSD. Data are expressed as mean $\pm$ SEM, n = 5 for each group.

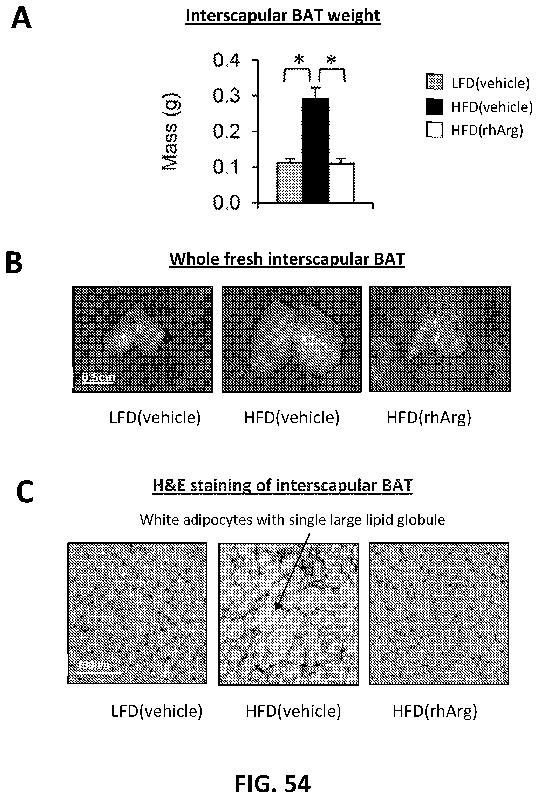

**FIG. 54** illustrates that administration of 600U N-ABD094-rhArg (SEQ ID NO: 50) once a week for 12 weeks to male mice with pre-existing HFD-induced obesity [HFD(rhArg) group] could effectively reverse whitening of brown fat (BAT). In the obese mice treated with N-ABD094-rhArg, **(A)** the weight of interscapular BAT; **(B)** the morphology and size of the interscapular BAT; and **(C)** histological features shown in the H&E stained section of BAT were similar to the lean control mice. *P < 0.05, one-way ANOVA followed by Fisher's LSD. Data are expressed as mean $\pm$ SEM, n = 5 for each group.

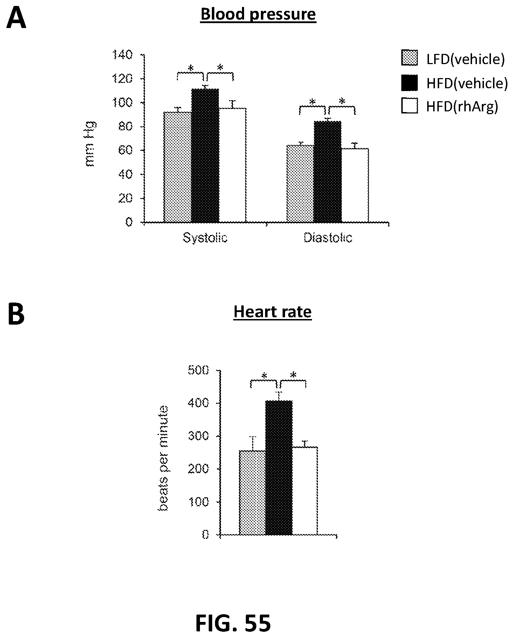

**FIG. 55** illustrates that administration of 600U N-ABD094-rhArg (SEQ ID NO: 50) once a week to male mice with pre-existing HFD-induced obesity [HFD(rhArg) group] could significantly lower **(A)** the systolic and diastolic blood pressure, and **(B)** the heart rate of obese mice to a level similar to the lean control mice [LFD(vehicle) group] by 5 weeks after drug treatment. *P < 0.05, one-way ANOVA followed by Fisher's LSD. Data are expressed as mean $\pm$ SEM, n = 10 for each group.

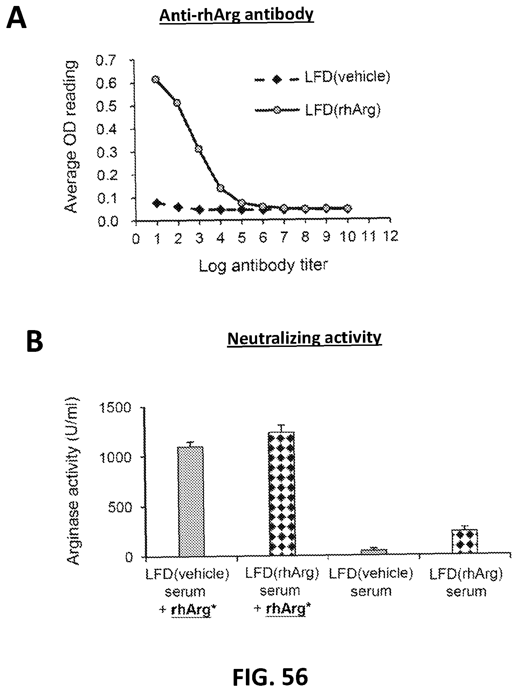

**FIG. 56** illustrates that C57BL/6J male mice fed a LFD and received an intraperitoneal injection of 500U N-ABD094-rhArg (SEQ ID NO: 50) [LFD(rhArg) group] once a week for 8 months starting from 5 week old had developed non-neutralizing anti-drug antibody. **(A)** Using an ELISA assay, anti-rhArg antibodies, with an average antibody titer of $10^8$, could be detected in the plasma of LFD-fed mice treated with N-ABD094-rhArg. **(B)** The plasma was incubated with 1,000U/mL N-ABD094-rhArg (rhArg*) for 1 hr followed by measurement of arginase activity. There was no reduction in arginase activity, showing that the antibodies generated in the animal against the drug were not neutralizing antibodies. Data are expressed as mean $\pm$ SEM, n = 3 for each group.

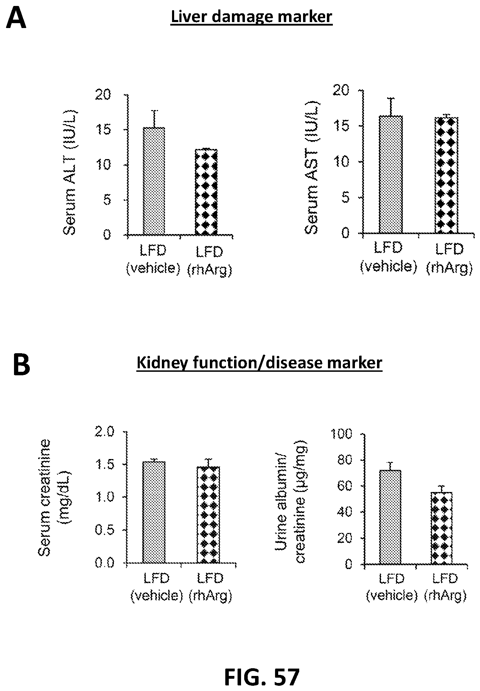

**FIG. 57** illustrates that administration of 500U N-ABD094-rhArg (SEQ ID NO: 50) to C57BL/6J male mice fed a LFD [LFD(rhArg) group] once a week for 8 months starting from 5 week old did not induce adverse effects on the liver and kidney. LFD-fed mice treated with N-ABD094-rhArg did not show any significant difference from LFD-fed mice treated with vehicle [LFD(vehicle) group] in **(A)** serum concentrations of alanine aminotransferase (ALT) and aspartate aminotransferase (AST), commonly used as biomarkers to assess liver functions or diseases; and **(B)** serum concentrations creatinine and urine albumin/creatinine, commonly used as biomarkers to assess kidney functions or diseases. Data are expressed as mean $\pm$ SEM, n = 3 for each group.

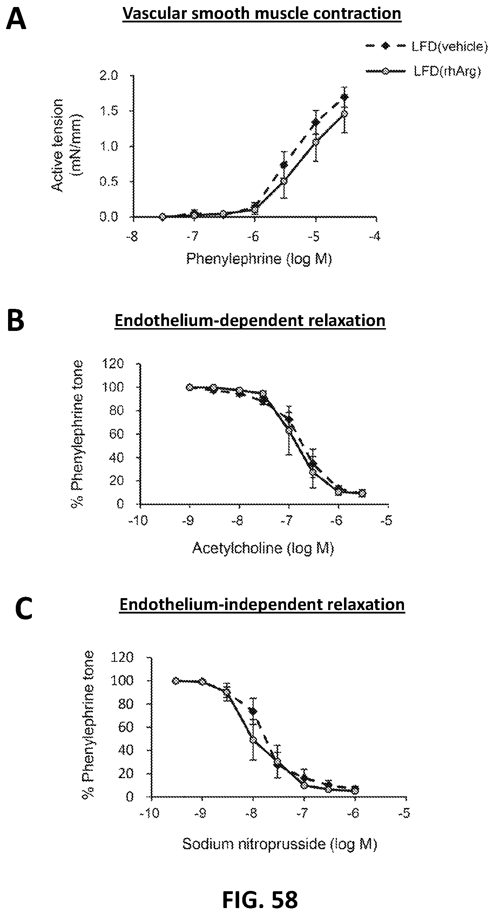

**FIG. 58** illustrates that administration of 500U N-ABD094-rhArg (SEQ ID NO: 50) to C57BL/6J male mice fed a LFD [LFD(rhArg) group] once a week for 8 months starting from 5 week old did not induce adverse vascular effects. LFD-fed mice treated with N-ABD094-rhArg did not show any significant difference from LFD-fed mice treated with vehicle [LFD(vehicle) group] in **(A)** vascular smooth muscle contraction evoked by phenylephrine; **(B)** acetylcholine-induced endothelium-dependent relaxation; and **(C)** endothelium-independent relaxation in response to the nitric oxide donor sodium nitroprusside. Data are expressed as mean $\pm$ SEM, n = 3 for each group.

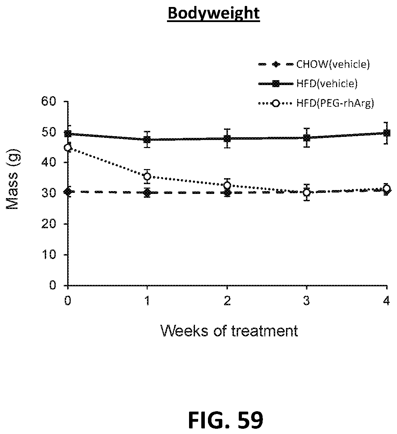

**FIG. 59** illustrates that administration of 300U PEGylated His-rhArg (SEQ ID NO: 101) once a week for 4 weeks to C57BL/6J male mice with pre-existing HFD-induced obesity [HFD(PEG-rhArg) group] could effectively reduce the bodyweight of mice to a weight similar to vehicle-treated lean control mice fed a chow diet [Chow(vehicle) group]. Data are expressed as mean $\pm$ SEM, n = 6 for each group..

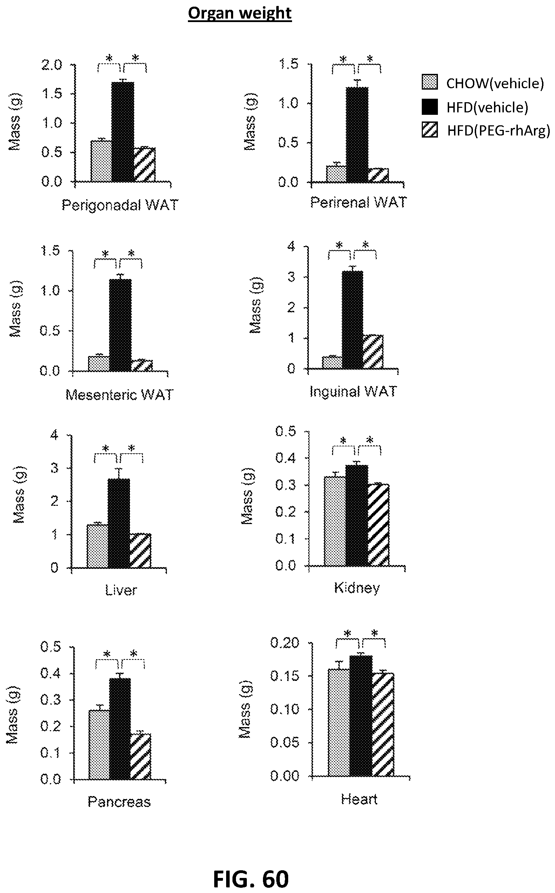

**FIG. 60** illustrates that administration of 300U PEGylated His-rhArg (SEQ ID NO: 101) once a week for 4 weeks to male mice with pre-existing HFD-induced obesity [HFD(PEG-rhArg) group] could effectively reduce the fat mass at the main visceral (perigondal, perirenal, mesenteric) and subcutaneous (inguinal) depots, and reduce the weight of the liver, kidney, pancreas and heart. $*P < 0.05$, one-way ANOVA followed by Fisher's LSD. Data are expressed as mean $\pm$ SEM, n = 4 for each group.

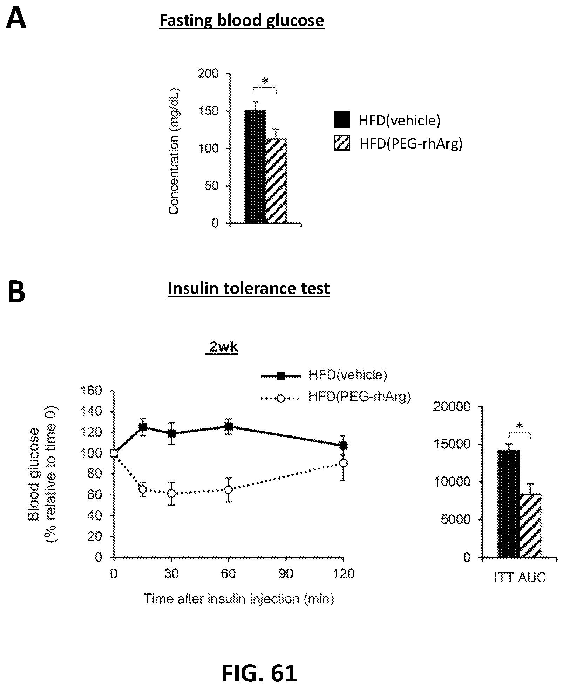

**FIG. 61** illustrates that administration of 300U PEGylated His-rhArg (SEQ ID NO: 101) once a week to male mice with pre-existing HFD-induced obesity [HFD(PEG-rhArg) group] could significantly **(A)** reduce fasting blood glucose, and **(B)** increase insulin sensitivity in the insulin tolerance test (ITT) conducted at 2 week after drug treatment. Results of ITT are expressed as area under the curve (AUC). $*P < 0.05$, one-way ANOVA followed by Fisher's LSD. Data are expressed as mean $\pm$ SEM, n = 6 for each group.

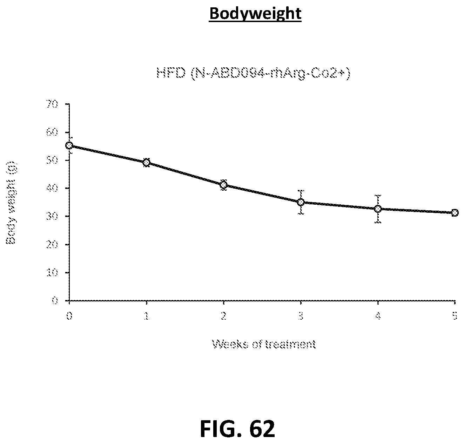

**FIG. 62** illustrates that administration of 25-100U N-ABD094-rhArg-Co$^{2+}$ [SEQ ID NO: 50 (cobalt substituted)] once a week for 5 weeks to C57BL/6J male mice with pre-existing HFD-induced obesity could effectively reduce the bodyweight of mice. Data are expressed as mean $\pm$ SEM,n = 3.

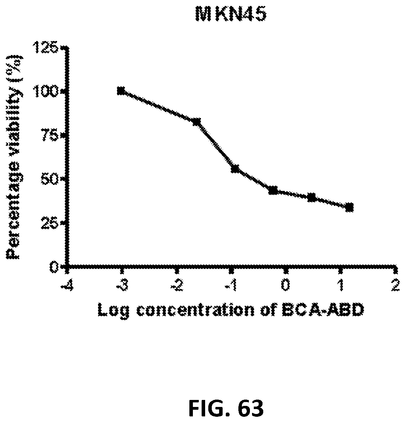

**FIG. 63** illustrates the anti-cancer effect of BHA fusion protein (SEQ ID NO: 75) on MKN45 gastric cancer cell line. The growth of MKN45 was inhibited in a dose-dependent manner. The viability was determined by MTT endpoint colorimetric assay. Data expressed as mean $\pm$ SD,n=3.

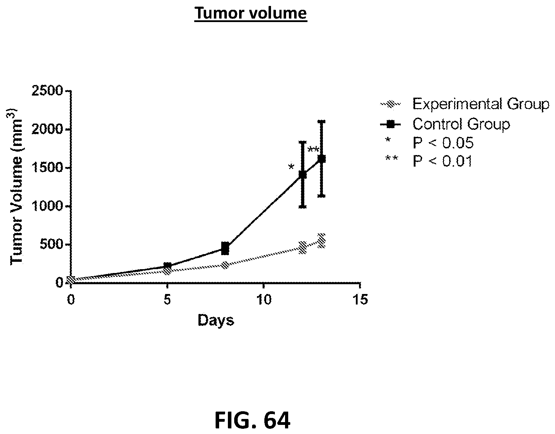

**FIG. 64** illustrates the effect of N-ABD-rhArg (SEQ ID NO: 49) on tumor volume in 4T1 breast cancer xenograft nude mice. The curve for "Experimental Group" represents the experimental group that received 500U N-ABD-rhArg (SEQ ID NO: 49) injection once per week; the curve for "Control Group" represents the control group that received PBS injection once per week. $*P < 0.05$, $**P < 0.01$ vs Experimental Group, t-test, n = 4-6.

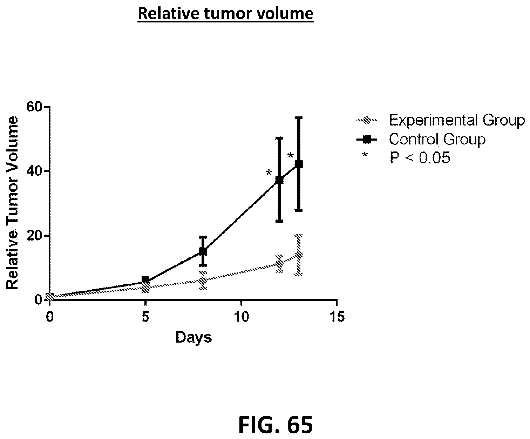

**FIG. 65** illustrates the effect of N-ABD-rhArg (SEQ ID NO: 49) on relative tumor volume in 4T1 breast cancer xenograft nude mice. The curve for "Experimental Group" represents the experimental group that received 500U N-ABD-rhArg (SEQ ID NO: 49) injection once per week. The curve for "Control group" represents the control group that received PBS once per week. $* P < 0.05$ vs Experimental Group, t-test, n = 4-6.

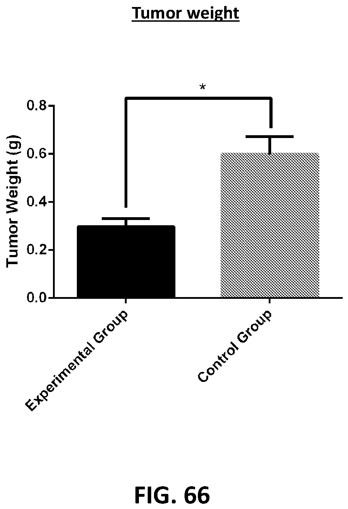

**FIG. 66** illustrates the significant differences of tumor weight between the experimental group and the control group. The experimental group received 500U N-ABD-rhArg (SEQ ID NO: 49) injection once per week. The control group received PBS injection once per week. $*P < 0.05$ vs Experimental Group, t-test, n = 4-6.

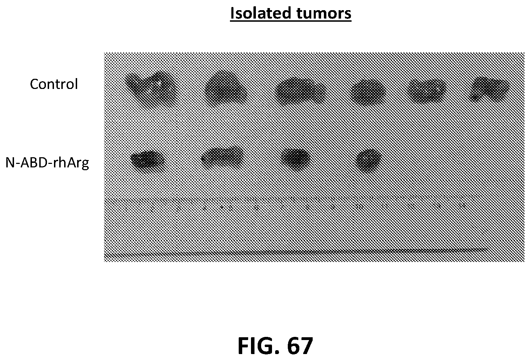

**FIG. 67** illustrates the photograph of the tumors collected from the nude mice of the control group and the N-ABD-rhArg (SEQ ID NO: 49) treated group.

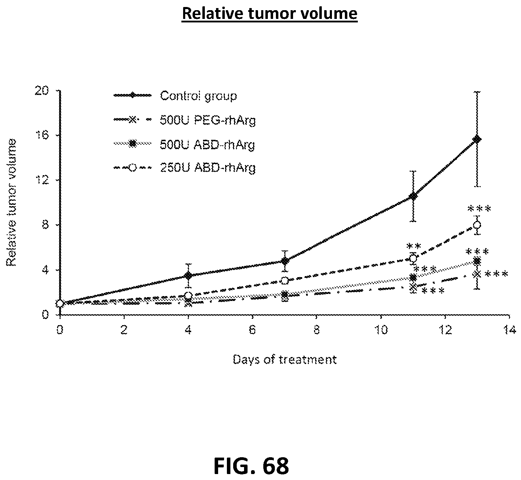

**FIG. 68** illustrates the effect of N-ABD-rhArg (SEQ ID NO: 49) on relative tumor volume in 4T1 breast cancer allografts. The curve for "500U ABD-rhArg" represents the experimental group that received 500U N-ABD-rhArg (SEQ ID NO: 49) injection once per week. The curve for "250U ABD-rhArg" represents the experimental group that received 250U N-ABD-rhArg (SEQ ID NO: 49) injection once per week. The curve for "500U PEG-rhArg" represents the experimental group that received 500U PEGylated His-rhArg (SEQ ID NO: 101) injection once per week. The curve for "Control group" represents the control group that received PBS once per week. $**P < 0.01$, $***P < 0.001$ vs Experimental Group, t-test, n = 6-8.

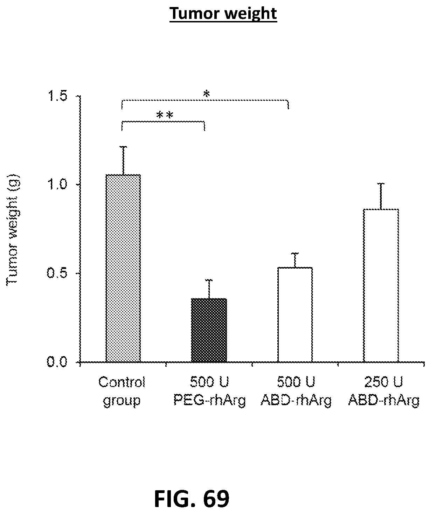

**FIG. 69** illustrates the significant differences of tumor weight between the experimental groups and the control group. The data for "500U ABD-rhArg" represents the group that received 500U N-ABD-rhArg (SEQ ID NO: 49) injection once per week. The data for "250U ABD-rhArg" represents the group that received 250U N-ABD-rhArg (SEQ ID NO: 49) injection once per week. The data for "500U PEG-rhArg" represents the group that received 500U PEGylated His-rhArg (SEQ ID NO: 101) injection once per week. The data for "Control group" represents the control group that received PBS once per week. $*P < 0.05$, $**P < 0.01$ vs Experimental Group, t-test, n = 6-8.

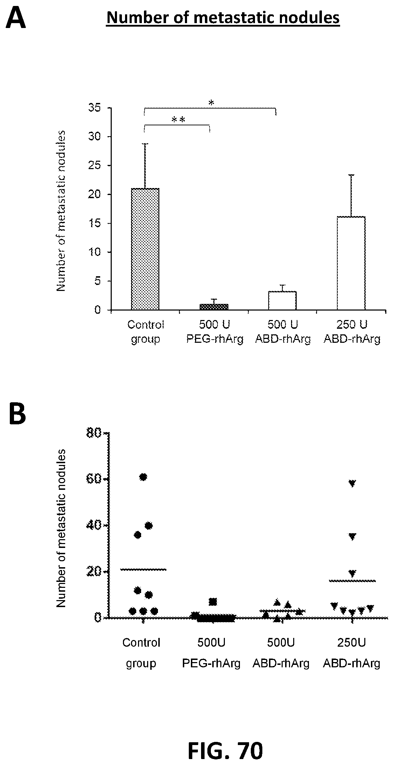

**FIG. 70** illustrates the average number of metastatic nodules from the lungs of 4T1 allografts. **(A)** The data for "500U ABD-rhArg" represents the group that received 500U N-ABD-rhArg (SEQ ID NO: 49) injection once per week. The data "250U ABD-rhArg" represents the group that received 250U N-ABD-rhArg (SEQ ID NO: 49) injection once per week. The data for "500U PEG-rhArg" represents the group that received 500U PEGylated His-rhArg (SEQ ID NO: 101) injection once per week. The data for "Control group" represents the control group that received PBS once per week. $*P < 0.05$, $**P < 0.01$ vs Experimental Group, t-test, $n = 6$-8. **(B)** Scatter plot of the number of metastatic nodules from the lungs of 4T1 allografts shown in FIG. 70A.

## DETAILED DESCRIPTION

The present disclosure generally relates to an arginine depleting agent for use in treating obesity, metabolic disorders, and related complications and comorbidities in a subject in need thereof by depleting the level of arginine in the subject. Depletion of arginine in the subject results in reduction of weight and fat mass, improvement of glucose tolerance and insulin responsiveness, normalization of blood glucose levels, endocrine and metabolic profiles, and alleviation of steatosis. Any agent capable of depleting arginine in a subject can be used in the methods of treating obesity, metabolic disorders, and related complications and comorbidities described herein.

## Definition of Terms

The definitions of terms used herein are meant to incorporate the present state-of-the-art definitions recognized for each term in the field of biotechnology. Where appropriate, exemplification is provided. The definitions apply to the terms as they are used throughout this specification, unless otherwise limited in specific instances, either individually or as part of a larger group.

As used herein, the term "half-life" or "½-life" refers to the time that would be required for the concentration of an agent, e.g., a fusion protein or arginine depleting agent as described herein, to fall by half *in vitro* or *in vivo,* for example, after injection into a mammal. In certain instances, the concentration of plasma arginine, after injection, is used herein as a proxy indicator of the half-life of the agent. In such instances, the term "therapeutic duration" is used to refer to the length of time a specified dosage of the arginine depleting agent is able to maintain the plasma concentration of arginine below a specified threshold concentration that a desired therapeutic effect is observed. In certain embodiments, the threshold concentration of plasma arginine is below 50 $\mu$M, below 40 $\mu$M, below 30 $\mu$M, below 20 $\mu$M, below 10 $\mu$M, below 5 $\mu$M, below 3 $\mu$M, or at a concentration below the detection limit of conventional analytical instrumentation. For example, depletion of plasma arginine to concentrations below the detection limit of the Biochrom 30 Amino Acid Analyzer (detection limit is 3 $\mu$M) for 7 days upon injection of an arginine catabolic enzyme described herein indicates a therapeutic duration of 7 days and a half-life, e.g., on the order of around 7 days.

The term "attach" or "attached" as used herein, refers to connecting or uniting by a bond or non-bonding interaction in order to keep two or more compounds together, which encompasses either direct or indirect attachment such that for example where a first polypeptide is directly bound to a second polypeptide or other molecule, and the embodiments wherein one or more intermediate compounds (e.g., a linker), such as a polypeptide, is disposed between the first polypeptide and the second polypeptide or other molecule.

The term "protein" or "polypeptide" as used herein indicates an organic polymer composed of two or more amino acid monomers and/or analogs thereof. The term "polypeptide" includes amino acid polymers of any length including full length proteins and peptides, as well as analogs and fragments thereof. A polypeptide of three or more amino acids is also called an oligopeptide. As used herein, the term "amino acid", "amino acidic monomer", or "amino acid residue" refers to any of the twenty naturally occurring amino acids including synthetic amino acids with unnatural side chains and including both D and L optical isomers. The term "amino acid analog" refers to an amino acid in which one or more individual atoms have been replaced, either with a different atom, isotope, or with a different functional group but is otherwise identical to its natural amino acid analog.

As used herein, the term "unnatural amino acid" refers to any amino acid, modified amino acid, and/or amino acid analogue that is not one of the 20 common naturally occurring amino acids, seleno cysteine or pyrrolysine.

As used herein, the term "fusion protein" refers to a chimeric protein containing proteins or functional protein fragments (e.g., arginase or variants thereof) having different origins that are covalently linked, e.g., by an amide, ester, urea, carbamate, ether, and/or disulfide bond.

As used herein, the term "variant" refers to a polynucleotide or nucleic acid differing from a reference nucleic acid or polypeptide, but retaining essential properties thereof. Generally, variants are overall closely similar, and, in many regions, identical to the reference nucleic acid or polypeptide.

A variant can, for example, comprise the amino acid sequence of the parent polypeptide sequence with at least one conservative amino acid substitution. Alternatively or additionally, the variant can comprise the amino acid sequence of the parent polypeptide sequence with at least one non-conservative amino acid substitution. In this case, it is preferable for the non-conservative amino acid substitution to not interfere with or inhibit the biological activity of the functional variant. The non-conservative amino acid substitution may enhance the biological activity of the variant, such that the biological activity of the variant is increased as compared to the parent polypeptide.

The term "functional fragment" when used in reference to a polypeptide refers to any part or portion of the subject polypeptide, which part or portion retains the biological activity of the polypeptide of which it is a part (the parent polypeptide). The functional fragment can be any fragment comprising contiguous amino acids of the polypeptide of which it is a part, provided that the functional fragment still exhibits at least 40%, 50%, 60%, 70%, 80%, 90%, 95%, or 99% or has substantially the same or even higher biological activity of the parent polypeptide. In reference to the parent polypeptide, the functional fragment can comprise, for instance, about 10%, 20%, 25%, 30%, 40%, 50%, 60%, 70%, 80%, 90%, 95%, 98% or more, of the parent polypeptide.

The functional fragment can comprise additional amino acids at the amino or carboxy terminus, or at both termini, e.g., amino acids not found in the amino acid sequence of the parent polypeptide.

Amino acid substitutions of the described polypeptides can be conservative amino acid substitutions. Conservative amino acid substitutions are known in the art, and include amino acid substitutions in which one amino acid having certain physical and/or chemical properties is exchanged for another amino acid that has the same or similar chemical or physical properties. For instance, the conservative amino acid substitution can be an acidic/negatively charged polar amino acid substituted for another acidic/negatively charged polar amino acid (e.g., Asp or Glu), an amino acid with a nonpolar side chain substituted for another amino acid with a nonpolar side chain (e.g., Ala, Gly, Val, Ile, Leu, Met, Phe, Pro, Trp, Cys, Val, etc.), a basic/positively charged polar amino acid substituted for another basic/positively charged polar amino acid (e.g. Lys, His, Arg, etc.), an uncharged amino acid with a polar side chain substituted for another uncharged amino acid with a polar side chain (e.g., Asn, Gln, Ser, Thr, Tyr, etc.), an amino acid with a beta-branched side-chain substituted for another amino acid with a beta-branched side-chain (e.g., Ile, Thr, and Val), an amino acid with an aromatic side-chain substituted for another amino acid with an aromatic side chain (e.g., His, Phe, Trp, and Tyr), etc.

The terms "percentage homology" and "percentage sequence identity", when used in reference to a polypeptide or polynucleotide sequence, are used interchangeably herein to refer to comparisons among polynucleotides and polypeptides, and are determined by comparing two optimally aligned sequences over a comparison window, wherein the portion of the polynucleotide or polypeptide sequence in the comparison window may comprise additions or deletions (i.e., gaps) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison and multiplying the result by 100 to yield the percentage of sequence identity. Homology is evaluated using any of the variety of sequence comparison algorithms and programs known in the art. Such algorithms and programs include, but are by no means limited to, TBLASTN, BLASTP, FASTA, TFASTA, and CLUSTALW [Pearson and Lipman, 1988, Proc. Natl. Acad. Sci. USA 85(8):2444-2448; Altschul et al., 1990, J. Mol. Biol. 215(3):403-410; Thompson et al., 1994, Nucleic Acids Res. 22(2):4673-4680; Higgins et al. 1996, Methods Enzymol. 266:383-402; Altschul et al., 1990, J. Mol. Biol. 215(3):403-410; Altschul et al., 1993, Nature Genetics 3:266-272]. In certain embodiments, protein and nucleic acid sequence homologies are evaluated using the Basic Local Alignment Search Tool ("BLAST") which is well known in the art (see, e.g., Karlin and Altschul, 1990, Proc. Natl. Acad. Sci. USA 87:2267-2268; Altschul et al., 1990, J. Mol. Biol. 215:403-410; Altschul et al., 1993, Nature Genetics 3:266-272; Altschul et al., 1997, Nuc. Acids Res. 25:3389-3402).

As used herein, the terms "treat", "treating", "treatment", and the like refer to reducing or ameliorating a disorder/disease and/or symptoms associated therewith. It will be appreciated, although not precluded, treating a disorder or condition does not require that the disorder, condition, or symptoms associated therewith be completely eliminated. In certain embodiments, treatment includes prevention of a disorder or condition, and/or symptoms associated therewith. The term "prevention" or "prevent" as used herein refers to any action that inhibits or at least delays the development of a disorder, condition, or symptoms associated therewith. Prevention can include primary, secondary and tertiary prevention levels, wherein: a) primary prevention avoids the development of a disease; b) secondary prevention activities are aimed at early disease treatment, thereby increasing opportunities for interventions to prevent progression of the disease and emergence of symptoms; and c) tertiary prevention reduces the negative impact of an already established disease by restoring function and reducing disease-related complications.

As used herein the term "catabolism" or "catabolic" refers to the chemical reaction of a molecule into other, e.g., smaller, molecules. For example, arginine catabolic enzymes refer to any enzyme capable of reacting with arginine thereby transforming it into other molecules, such as ornithine, citrulline, and agmatine.

As used herein, the term "subject" refers to any animal (e.g., a mammal), including, but not limited to, humans, non-human primates, canines, felines, and rodents.

As used herein the term "body mass index" or "BMI" means the ratio of weight in Kg divided by the height in meters, squared.

As used herein the term "overweight" refers to a BMI between 25 and 30 in adult humans. For people under 20 "overweight" is defined as a BMI between the 85th and 95th percentile compared to people of the same age.

As used herein the term "obesity" refers to a BMI between 30 and 40 in adult humans. For people under 20 "obesity" is defined as a BMI above the 95th percentile compared to people of the same age. As used herein, the term can include both obesity and morbid obesity.

As used herein the term "morbid obesity" refers to a BMI greater than 40 in adult humans.

As used herein, the term "steatosis" refers to the condition in which fat accumulates in tissues, such as liver tissue, kidney tissue, pancreas tissue, heart muscle tissue or other muscle tissues.

As used herein, the term "fibrosis" refers to the condition in which there is excess deposition of fibrous connective tissue, including collagen, in an organ or tissue, such as liver tissue, kidney tissue, pancreas tissue or heart tissue.

The term "hypercholesterolemia", as used herein, refers to a condition in which levels of blood cholesterol are elevated in comparison to the normal average level of blood cholesterol in a respective reference subject of the same ethnic background, age, and gender. With regard to humans the term in particular refers to blood levels of total cholesterol above about 200, in particular above about 240 mg/dL.

The terms "dyslipidemia" and "hyperlipidemia" as used herein, refers to a condition in which the level of lipids/fats including cholesterol, cholesterol esters, phospholipids, triglycerides and/or lipoproteins in the blood stream are respectively abnormal and elevated in comparison to the normal average level of blood lipids/fats in a respective reference subject of the same ethnic background, age, and gender.

Provided herein is an arginine depleting agent for use in treating obesity and/or a metabolic disorder in a subject in need thereof comprising the step of administering a therapeutically effective amount of an arginine depleting agent to the subject.

The arginine depleting agent can be any arginine depleting agent known in the art that is capable of reducing plasma and/or cellular levels of arginine in a subject. The arginine depleting agent can be a small molecule or protein.

The protein can be a fusion protein and/or a chemically modified protein, such as a PEGylated protein. Exemplary proteins include those that are capable of catalyzing the catabolism of arginine to other products, such as proteins having arginase, arginine deiminase, or arginine decarboxylase activity.

The arginase can be any arginase known in the art, such as those produced by bacteria, fungi, fish, human, bovine, swine, rabbit, rodent, primate, sheep and goat. For example, *Bacillus caldovelox* arginase, *Thermus thermophilus* arginase, *Capra hircus* arginase I, *Heterocephalus glaber* arginase I, *Bos taurus* arginase I, *Sus scrofa* arginase I, *Plecoglossus altivelis* arginase I, *Salmo salar* arginase I, *Oncorhynchus mykiss* arginase I, *Osmerus mordax* arginase I, *Hyriopsis cumingii* arginase I, *Rattus norvegicus* arginase I, *Mus musculus* arginase I, *Homo sapiens* (human) arginase I, *Pan troglodytes* arginase I, *Oryctolagus cuniculus* arginase I, *Rattus norvegicus* arginase II, *Mus musculus* arginase II, *Homo sapiens* (human) arginase II, *Bostaurus* arginase II, *Heterocephalus glaber* arginase II, *Pan troglodytes* arginase II, *Oryctolagus cuniculus* arginase II, *Delftia* arginase, *Bacillus coagulans* arginase, *Hoeflea phototrophica* arginase and *Roseiflexus castenholzii* arginase. Other examples include arginases from *Bacillus methanolicus, Bacillus sp. NRRL B-14911, Planococcus donghaensis, Paenibacillus dendritiformis, Desmospora sp., Methylobacter tundripaludum, Stenotrophomonas sp., Microbacterium laevaniformans, Porphyromonas uenonis, Agrobacterium sp., Octadecabacter arcticus, Agrobacterium tumefaciens, Anoxybacillus flavithermus, Bacillus pumilus, Geobacillus thermoglucosidasius, Geobacillus thermoglucosidans, Brevibacillus laterosporus, Desulfotomaculum ruminis, Geobacillus kaustophilus, Geobacillus thermoleovorans, Geobacillus thermodenitrificans, Staphylococcus aureus, Halophilic archaeon DL31, Halopigerxanaduensis, Natrialba magadii, Plasmodium falciparum, Helicobacter pylori,* and the like.

The arginine deiminase can be any arginine deiminase known in the art, such as those produced from Mycoplasma, Lactococcus, Pseudomonas, Steptococcus, Escherichia, Mycobacterium or Bacillus microorganisms. Exemplary arginine deiminase include, but are not limited, to those produced by *Mycoplasma hominis, Mycoplasma arginini, Mycoplasma arthritidis, Clostridium perfringens, Bacillus licheniformis, Borrelia burgdorferi, Borrelia afzellii, Enterococcus faecalis, Lactococcus lactis, Bacillus cereus, Streptococcus pyogenes, Steptococcus pneumoniae, Lactobacillus sake, Giardia intestinalis, Mycobacterium tuberculosis, Pseudomonas plecoglossicida, Pseudomonas putida, Pseudomonas aeruginosa,* and the like.

The arginine decarboxylase can be any arginine decarboxylase known in the art, such as those produced *by Escherichia coli., Salmonella typhimurium, Chlamydophila pneumoniae, Methanocaldococcus jannaschii, Paramecium bursaria Chlorella virus 1, Vibrio vulnificus YJ016, Campylobacter jejuni subsp., Trypanosoma cruzi, Sulfolobus solfataricus, Bacillus licheniformis, Bacillus cereus, Carica papaya, Nicotianatobacum, Glycine max, Lotus coniculata, Vibrio vulnificus, Vibrio cholerae, Mus musculus, Thermotoga, Rattus norvegicus, Homo sapiens, Bos taurus, Susscrofa, Thermus thermophiles, Thermus parvatiensis, Thermus aquaticus, Thermus thermophilus, Thermus islandicus, Arabidopsis thaliana, Avena sativa,* and the like.

The arginine decarboxylase, arginine deiminase, and arginase can be the full protein or a functional fragment and/or variant thereof. The arginine decarboxylase, arginine deiminase, and arginase can be modified to improve their pharmacokinetic properties, such as by fusion of the protein or functional fragment and/or variant thereof with human serum albumin, an albumin binding domain, an Fc region of immunoglobulin, a PEG group, or a combination thereof.

The arginine catabolic enzymes described herein can be engineered to include specific sites on the enzyme where PEG can be selectively attached. The selected PEGylation sites are preferably located at a site removed from the active site of the enzyme, and generally exposed to solvent to allow reaction with PEGylation reagents.

For example, Cys$^{45}$-human arginase I (HAI) and Cys$^{161}$-*Bacillus caldovelox* arginase (BCA) can be produced to react with thiol-specific PEG molecules. Conjugation between the single, free cysteine residue of the modified arginase and a maleimide group (MAL) attached to a PEG compound can result in a covalent bond between the PEG compound and the free cysteine of the modified arginase. SEQ ID NOs: 102 and 104 include mutant (C168S/C303S) designed for Cys[45] site-directed PEGylation and thus can optionally be PEGylated. SEQ ID NO: 89 also includes mutant (S161C) designed for Cys[161] site-directed PEGylation and thus can optionally be PEGylated.

In certain embodiments the arginase can comprise SEQ ID NO: 101, SEQ ID NO: 102, SEQ ID NO: 103, or SEQ ID NO: 104, wherein SEQ ID NO: 102 and SEQ ID NO: 104 optionally comprise a polyethylene glycol group (PEG).

Any PEGylation reagent known in the art can be used to covalently attach PEG to the arginine catabolic enzymes described herein. Exemplary PEGylation reagents include, but are not limited to mPEG-ALD (methoxypolyethylene glycol-propionaldehyde); mPEG-MAL (methoxypolyethylene glycol-maleimide); mPEG-NHS (methoxypolyethylene glycol-N-hydroxy-succinimide); mPEG-SPA (methoxypolyethylene glycol-succinimidyl propionate); and mPEG-CN (methoxypolyethylene glycol-cyanuric chloride).

The PEG group can have a molecular weight of about 5,000 to about 20,000 amu, about 5,000 to about 15,000 amu, about 5,000 to about 12,000 amu, about 7,000 to about 12,000 amu, or about 7,000 to about 10,000 amu. In certain embodiments, the PEG group has a molecular weight of about 2,000 amu to 10,000 amu. In certain embodiments, the PEG group is PEG4,000, PEG5,000, PEG6,000, or PEG7,000.

The PEG group can be covalently attached directly to the arginase or via a linker. In certain embodiments, the arginase is covalently attached via a propionic acid linker to PEG. In other embodiments, the arginase is covalently attached via a C2-C10, C2-C9, C2-C8, C2-C7, C2-C6, C2-C5, or C2-C4 straight or branched chain carboxylic acid linker to PEG.

In certain embodiments, the arginine depleting agent is a fusion protein comprising an arginase polypeptide. The arginase polypeptide can be derived from an arginase protein expressed by any organism that expresses arginase. Exemplary arginases include those that are produced by bacteria, such as bacilli, agrobacteria, cyanobacteria, and mycobacteria, and mammals, such as bovine, porcine, sheep, goat, rodents and humans. When the arginase polypeptide is derived from human arginase, it can be arginase type 1 (ARG1) or arginase type 2 (ARG2).

The arginase polypeptide can comprise a full length arginase polypeptide or a functional fragment and/or variant thereof.

Arginase is a manganese-containing enzyme. As demonstrated in the Example 32, when one or more of the manganese ions present in the fusion are replaced with one or more divalent cationic metal, such as $Co^{2+}$ or $Ni^{2+}$, the catalytic activity of the fusion protein can increase. Accordingly, in certain embodiments, the fusion proteins comprise one or more divalent metals, other than manganese, such as $Co^{2+}$ or $Ni^{2+}$. In certain embodiments, the fusion protein comprises one or more metals selected from $Co^{2+}$ and $Ni^{2+}$. In certain embodiments, the fusion protein comprises two $Co^{2+}$ ions or two $Ni^{2+}$ ions. In other embodiments, the fusion protein comprises two $Mn^{2+}$ ions.

In certain embodiments, the arginase polypeptide is wild type human ARG1. In certain embodiments, the arginase polypeptide comprises a sequence with at least 95% sequence homology to SEQ ID NO: 69. For example, the arginase polypeptide can comprise at a polypeptide sequence with at least 96%, 97%, 98%, 99%, 99.1%, 99.4% or 99.7% homology to SEQ ID NO: 69. In certain embodiments, the sequence of the arginase polypeptide can differ from SEQ ID NO: 69 by 1, 2, 3, 4, 5, 6, 7, 8, 9, 10, 15, 20, 25, or 30 amino acid modifications (e.g., insertion, substitution, deletion, etc.). In certain embodiments, the arginase polypeptide comprises a polypeptide with conservative amino acid replacements, non-conservative amino acid replacements, or a combination thereof.

In certain embodiments, the arginase polypeptide is *Bacillus caldovelox* arginase (BCA). In certain embodiments, the arginase polypeptide comprises a sequence with at least 95% sequence homology to SEQ ID NO: 70. For example, the arginase polypeptide can comprise at a polypeptide sequence with at least 96%, 97%, 98%, 99%, 99.3%, or 99.7% homology to SEQ ID NO: 70. In certain embodiments, the sequence of the arginase polypeptide can differ from SEQ ID NO: 70 by 1, 2, 3, 4, 5, 6, 7, 8, 9, 10, 15, 20, 25, or 30 amino acid modifications (e.g., insertion, substitution, deletion, etc.). In certain embodiments, the arginase polypeptide comprises a polypeptide with conservative amino acid replacements, non-conservative amino acid replacements, or a combination thereof.

In certain embodiments, the arginase polypeptide is BCA, wherein serine 161 is replaced by a cysteine as presented in SEQ ID NO: 71 and SEQ ID NO: 72. The substitution of serine with a cysteine allows for the sited directed incorporation of chemical moieties that can further improve the properties of the fusion protein. For example, the side chain of cysteine 161 can be reacted with an appropriately activated PEG moiety thereby forming a PEGylated arginase, which can be incorporated into the resulting fusion protein. PEGylation of the fusion protein has the ability to further enhance the retention time of the fusion proteins by further protecting them against various degrading mechanisms active inside a tissue or cell, which consequently improves their therapeutic potential. Accordingly, in certain embodiments the arginase polypeptide comprises a sequence with at least 95% sequence homology to SEQ ID NO: 71 or SEQ ID NO: 72. For example, the arginase polypeptide can comprise at a polypeptide sequence with at least 96%, 97%, 98%, 99%, 99.3%, or 99.7% homology to SEQ ID NO: 71 or SEQ ID NO: 72. In certain embodiments, the sequence of the arginase polypeptide can differ from SEQ ID NO: 71 or SEQ ID NO: 72 by 1, 2, 3, 4, 5, 6, 7, 8, 9, 10, 15, 20, 25, or 30 amino acid modifications (e.g., insertion, substitution, deletion, etc.). In certain embodiments, the arginase polypeptide comprises a polypeptide with conservative amino acid replacements, non-conservative amino acid replacements, or a combination thereof.

In instances where the fusion proteins are PEGylated, the PEG group can have a molecular weight of about 5,000 to about 20,000 amu, about 5,000 to about 15,000 amu, about 5,000 to about 12,000 amu, about 7,000 to about 12,000 amu, or about 7,000 to about 10,000 amu. In certain embodiments, the PEG group has a molecular weight of about 4,000 amu to 10,000 amu. In certain embodiments, the PEG group is PEG4,000 or PEG7,000.

The PEG group can be covalently attached directly to the fusion protein or via a linker. PEG group can be covalently attached to the fusion protein by reaction of a cysteine or lysine side chain present on the protein with a PEGylation reagent. Alternatively, the PEG group can be covalently attached to the N-terminal amine of the protein.

In certain embodiments, the fusion protein is covalently attached via a propionic acid linker to PEG. In other embodiments, the fusion protein is covalently attached via a C2-C10, C2-C9, C2-C8, C2-C7, C2-C6, C2-C5, or C2-C4 straight or branched chain carboxylic acid linker to PEG. In certain embodiments, the PEG group is attached to the arginase polypeptide.

The fusion proteins can also comprise an albumin binding domain (ABD) polypeptide. A number of studies have demonstrated the potential of albumin binding to achieve longer half-lives of therapeutic proteins. However, the design of fusion proteins including ABD polypeptides can be challenging, because fusion of the ABD polypeptide to the protein therapeutic has the potential to affect both the efficacy of the protein therapeutic, the binding affinity of the ABD polypeptide, and the solubility of the fusion protein. Accordingly, the selection of the ABD polypeptide, its site of attachment, and the construction of any necessary linkers is not a straight forward process and often times requires trial and error in order to arrive at a fusion property with the desired properties. For example, Figs 9, 10 and 13 demonstrate that the therapeutic duration (and half-life) of N-ABD094-rhArg (SEQ ID NO: 50) is unexpectedly much longer than BHA and BAH. While all of BHA, BAH, and N-ABD094-rhArg exhibit surprisingly high therapeutic duration (and half-life), it could not have been predicted that N-ABD094-rhArg would have such long therapeutic duration (and half-life).

Longer action of a drug is generally desirable. The linker type, length, flexibility, and fusion of the bioactive peptide or protein to the C or N terminus of the half-life-extension module, can have profound effects on the activity of a fusion protein. In the present disclosure, various arginases were fused to ABD molecules via a suitable linker so that both the arginase enzymatic activity and the albumin binding ability of ABD can be retained. Good stability and solubility are also essential. This is very difficult and challenging to achieve. Unlike the common HSA or Fc fusions, very little is known about the ABD fusions. The present disclosure provides examples of the linker design that can be used to generate functional arginase-ABD fusions. The activity of the engineered arginase fusion proteins (N-ABD094-rhArg, N-ABD-rhArg, etc) have been optimized in the present disclosure either through linker engineering or the position of the bioactive protein (arginase) with respect to the half-life-extension module (ABD), or both.

In many cases, protein engineering can be used to overcome the loss of activity. In one example, linker engineering was used to regain activity lost upon fusion of IFN-$\alpha$ 2b to HSA [Prot Exp Purif. 2008; 61:73-7]. A direct fusion of IFN-$\alpha$2b to HSA resulted in an unstable protein with very little biological activity. In a fusion format, the effects of various linkers on the activity of IFN-$\alpha$2b were tested. Peptide linkers are known to have an influence on the expression, activity, and pharmacokinetics of fusion proteins [Adv Drug Deliv Rev. 2013; 65:1357-69]. The two major types of peptide linkers are: (i) flexible linkers (e.g., (G4S)n, where n = 1-4); (ii) rigid linkers such as the $\alpha$-helical linker [A(EAAAK)nA]x (where n = 2-4 and x = 1 or 2), and XPn (where X is either A, K or E). For flexible linkers, one advantage is that the flexibility may be required to obtain proper orientation of the bioactive portion of the molecule with respect to its cognate receptor. However, flexible linkers do not give a lot of space between the fusion partner and the bioactive protein. On the other hand, rigid linkers provide more space but lack the flexibility. In the case of the IFN-$\alpha$2b-HSA fusion protein, the flexible linker resulted in approximately 39% activity as compared with that of native IFN-$\alpha$2b, whereas the rigid XP linker and the $\alpha$-helical linker resulted in 68 and 115% of the activity of native IFN-$\alpha$2b, respectively [Prot Exp Purif. 2008;61:73-7].

Certain linkers can have a negative impact on fusion protein properties. For example, granulocyte colony-stimulating factor (G-CSF) was fused to transferrin (Tf). The use of a short leucine-glutamate (LE) linker resulted in only approximately 10 % of the activity of native G-CSF. Insertion of a $(G_4S)_3$ or $\alpha$-helical [A(EAAAK)$_n$A]$_m$ (n = 2-4, m = 1 or 2) linker significantly increased the activity of the fusion proteins over that of G-CSF-LE-Tf. The fusion protein constructed with the linker (A(EAAAK)$_4$ALEA-(EAAAK)$_4$A) resulted in biological activity near to that of native G-CSF [Pharm Res. 2006;23:2116-21]. In another example, while the C-terminus of the Fc moiety may be directly linked to the N-terminus of the IFN-$\alpha$ moiety via a peptide bond, Gillies et al. [US 7,670,595 B2] additionally connects the Fc moiety and the IFN-$\alpha$ moiety via a linker peptide. The linker peptide is located between the C-terminus of the Fc moiety and the N-terminus of the mature IFN-$\alpha$ moiety. In this case, the linker peptide is preferably composed of serine and glycine residues such as the amino acid sequence G$_4$SG$_4$SG$_3$SG. All these findings demonstrate the importance of testing linker technology for the success of fusion protein research and development programs.

Using a different approach, the importance of fusion position for activity has been demonstrated [Curr Pharmaceut Biotechnol. 2014;15:856-63]. The brain natriuretic peptide (BNP) was fused to either the N or C terminus of HSA in various formats. The results showed that BNP-HSA, BNP2-HSA (two copies of BNP), and BNP4-HSA, all fused to the N terminus of HSA, were not active. However, HSA-BNP2, fused to the C-terminus of HSA, was as active as native BNP and long-lasting.

The albumin binding protein is a three-helical protein domain found in various surface proteins expressed by Gram positive bacteria. The albumin binding protein derived from Streptococcal protein G has 214 amino acids and contains three albumin binding domains (ABD1-3), which are used to bind to human serum albumin and evade the immune system of a host. ABD3 corresponds to a 46 amino acid sequence, which has been demonstrated to bind to human serum albumin and has been the subject of a number of studies and affinity maturation for human serum albumin to develop ABD polypeptides with differing properties, such as binding affinity and binding selectivity. Such studies have generated a substantial number of ABD polypeptides with widely varying properties.

Albumin binding proteins are found in other bacteria. For example, naturally occurring albumin binding proteins include certain surface proteins from Gram positive bacteria, such as Streptococcal M proteins (e.g. Ml/Emml, M3 Emm3, M12/Emml2, EmmL55/Emm55, Emm49/EmmL49 and Protein H), streptococcal proteins G, MAG and ZAG, and PPL and PAB from certain strains of *Finegoldia magna.*

In certain embodiments, the fusion proteins comprise an ABD polypeptide that is derived from a Streptococcal protein G albumin binding domain. In certain embodiments, the ABD polypeptide is the full Streptococcal protein G albumin binding domain 3 or a functional fragment and/or variant thereof.

In certain embodiments, the fusion protein comprises an ABD polypeptide comprising a polypeptide sequence having at least 93% sequence homology with SEQ ID NO: 66. For example, the ABD polypeptide can comprise at a polypeptide sequence with at least 94%, 96%, or 98% homology to SEQ ID NO: 66. In certain embodiments, the sequence of the ABD polypeptide can differ from SEQ ID NO: 66 by 1, 2, 3, 4, 5, 6, 7, 8, 9, or 10 amino acid modifications (e.g., insertion, substitution, deletion, etc.). In certain embodiments, the ABD polypeptide comprises a polypeptide with conservative amino acid replacements, non-conservative amino acid replacements, or a combination thereof.

In certain embodiments, the fusion protein comprises an ABD polypeptide comprising a polypeptide sequence having at least 93% sequence homology with SEQ ID NO: 67. For example, the ABD polypeptide can comprise at a polypeptide sequence with at least 94%, 96%, or 98% homology to SEQ ID NO: 67. In certain embodiments, the sequence of the ABD polypeptide can differ from SEQ ID NO: 67 by 1, 2, 3, 4, 5, 6, 7, 8, 9, or 10 amino acid modifications (e.g., insertion, substitution, deletion, etc.). In certain embodiments, the ABD polypeptide comprises a polypeptide with conservative amino acid replacements, non-conservative amino acid replacements, or a combination thereof.

In certain embodiments, the fusion protein comprises an ABD polypeptide comprising a polypeptide sequence having at least 93% sequence homology with SEQ ID NO: 68. For example, the ABD polypeptide can comprise a polypeptide sequence with at least 93%, 95%, or 97% homology to SEQ ID NO: 68. In certain embodiments, the sequence of the ABD polypeptide can differ from SEQ ID NO: 68 by 1, 2, 3, 4, 5, 6, 7, 8, 9, or 10 amino acid modifications (e.g., insertion, substitution, deletion, etc.). In certain embodiments, the ABD polypeptide comprises a polypeptide with conservative amino acid replacements, non-conservative amino acid replacements, or a combination thereof.

The relative position of the ABD polypeptide and arginase polypeptide can vary. For example, the ABD polypeptide can precede the arginase polypeptide (e.g., the arginase polypeptide can be attached either directly or indirectly from the C-terminal of the ABD polypeptide) or the arginase polypeptide can precede the ABD polypeptide (e.g., the ABD polypeptide can be attached either directly or indirectly from the C-terminal of the arginase polypeptide).

In certain embodiments, the fusion protein can include one or more arginase polypeptides and/or one or more ABD polypeptides. For example, the fusion protein can have the general structure ABD-rhArg-ABD, ABD094-rhArg-ABD094, ABD-BCA-ABD, ABD094-BCA-ABD094, rhArg-ABD-rhArg, rhArg-ABD094-rhArg, BCA-ABD-BCA, or BCA-ABD094-BCA.

The ABD polypeptide and the arginase polypeptide can be attached by direct covalent attachment or indirectly attached via a peptide linker.

The peptide linker or linker is a polypeptide typically ranging from about 1, 2, 3, 4, 5, 6, 7, 8, 9, 10, 11, 12, 13, 14, 15, 16, 17, 18, 19, 20 or more amino acids in length, which is designed to facilitate the functional connection of the ABD polypeptide and arginase polypeptide into a linked fusion protein. The term functional connection denotes a connection that facilitates proper folding of the polypeptides into a three dimensional structure that allows the linked fusion protein to exhibit some or all of the functional aspects or biological activities of the protein(s) from which its polypeptide constituents are derived.

The polypeptide linker can be disposed between the N-terminal of the ABD polypeptide and the C-terminal of the arginase polypeptide or alternatively disposed between the N-terminal of the arginase polypeptide and the C-terminal of the ABD polypeptide.

The peptide linker can comprise naturally occurring amino acids, unnatural amino acids, and combinations thereof.

In certain embodiments, the peptide linker can comprise glycine, serine, asparagine, or a combination thereof. Exemplary peptide linkers include linkers containing poly-glycine, $(GS)_n$, and $(GGS)_n$, wherein n is 1-30 Additional exemplary peptide linkers include flexible linkers (e.g., $(G_4S)_n$, wherein n = 1-4), or rigid linkers (e.g., the alpha-helical linker $[A(EAAAK)_nA]_x$, wherein n = 2-4 and x = 1 or 2; and XPn, wherein X is either A, K or E and n = 1-10). In certain embodiments, the peptide linker is (A(EAAAK)$_4$ALEA-(EAAAK)$_4$A) (SEQ ID NO: 105), G$_4$SG$_4$SG$_3$SG (SEQ ID NO: 106), GS(N)$_n$GSG, where n = 1-10, and GS(Q)$_n$GSG, where n = 1-10, and the like. In certain embodiments, the peptide linker comprises a polypeptide sequence having at least 90% sequence homology with SEQ ID NO: 73. For example, peptide linker can comprise a polypeptide having at least a 91%, 92%, 93%, 94%, 95%, 96%, 97%, 98%, or 99% sequence homology or is identical to SEQ ID NO: 73.

In certain embodiments, the peptide linker can comprise glycine, serine, asparagine, or a combination thereof. In certain embodiments, the peptide linker comprises a polypeptide sequence having at least 90% sequence homology with SEQ ID NO: 74. For example, peptide linker can comprise a polypeptide having at least a 91%, 92%, 93%, 94%, 95%, 96%, 97%, 98%, or 99% sequence homology or is identical to SEQ ID NO: 74.

Purification tags can be used to improve the ease of purifying the fusion protein, such as by affinity chromatography. A well-known purification tag is the hexa-histidine (6x His) tag, which is a sequence of six histidine residues. Thus, in certain embodiments, the fusion protein further comprises a poly-histidine comprising 4-8 histidine amino acids, e.g., the 6x His tag. The poly-histidine can be present at the C-terminal of the fusion protein, the N-terminal of the fusion protein, or disposed in between ABD polypeptide and the arginase polypeptide.

When the poly-histidine is disposed in between the ABD polypeptide and the arginase polypeptide it can act as a peptide linker or can be included in addition to the peptide linker. For example, the fusion protein of SEQ ID NO 75 includes a six histidine polypeptide linker at position 300-305, which serves to link the ABD polypeptide and the arginase polypeptide and advantageously can be used to purify the fusion protein by affinity chromatography.

The poly-histidine tag can be optionally removed after purification is complete using techniques generally known in the art. For example, exopeptidases can be used to remove N-terminal poly-histidine tags (e.g., Qiagen TAGZyme) and C-terminal poly-histidine tags can be preceded by a suitable amino acid sequence that facilitates a removal of the poly-histidine-tag using endopeptidases. Thus, fusion proteins excluding the N-terminal and/or C-terminal poly-histidine tag are encompassed within the scope of this disclosure.

In certain embodiments, the ABD polypeptide comprises a polypeptide sequence having at least 93% sequence homology with SEQ ID NO: 66, SEQ ID NO: 67, or SEQ ID NO: 68 and the arginase polypeptide comprises a polypeptide sequence having at least 95% homology with SEQ ID NO: 69, SEQ ID NO: 70, SEQ ID NO: 71, or SEQ ID NO: 72. For example, the ABD polypeptide comprises a polypeptide sequence having at least 94%, 95%, 96%, 97%, 98%, or 99% sequence homology or is identical with SEQ ID NO: 66, SEQ ID NO: 67, or SEQ ID NO: 68 and the arginase polypeptide comprises a polypeptide sequence having at least 96%, 97%, 98%, or 99% sequence homology or is identical with SEQ ID NO: 69, SEQ ID NO: 70, SEQ ID NO: 71, or SEQ ID NO: 72.

In certain embodiments, the ABD polypeptide comprises a polypeptide sequence having at least 93%, 94%, 95%, 96%, 97%, 98%, or 99% sequence homology or is identical with SEQ ID NO: 66 and the arginase polypeptide comprises a polypeptide sequence having at least 95%, 96%, 97%, 98%, or 99% sequence homology or is identical with SEQ ID NO: 69.

In certain embodiments, the fusion protein further comprises a peptide linker comprising a polypeptide sequence having at least 90%, 91%, 92%, 93%, 94%, 95% , 96%, 97%, 98%, or 99% sequence homology or identical with SEQ ID NO: 73 or SEQ ID NO: 74.

Exemplary fusion proteins include fusion proteins having at least 95%, 96%, 97%, 98%, or 99% homology or are identical with SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 75, and SEQ ID NO: 76.

As demonstrated in the experiments below, arginine depletion can dramatically reduce adiposity. Here we have investigated the therapeutic potential of arginine deprivation using ABD arginase fusion proteins or PEGylated arginase, such as N-ABD094-rhArg (SEQ ID NO: 50), PEGylated His-rhArg (SEQ ID NO: 101), and N-ABD094-rhArg-Co$^{2+}$ [SEQ ID NO: 50 (cobalt substituted)] in treating obesity and associated metabolic disorders. The data presented herein demonstrates that arginine depletion with, e.g., N-ABD094-rhArg (SEQ ID NO: 50), can significantly reduce fat mass, suppress lipogenesis and increase insulin sensitivity in normal lean mice and obese mice. It was found that arginine deprivation, e.g., with N-ABD094-rhArg (SEQ ID NO: 50), can prevent and reduce obesity, metabolic disorders, and related complications and comorbidities, including insulin resistance.

The effects of arginine depletion presented herein are surprising and unexpected, because numerous studies have shown that increasing dietary intake of some amino acids (AAs), including arginine, has significant effects on glucose and lipid metabolism and AA supplementation could be used to reduce body weight. Moreover, since arginine is a semi-essential amino acid, its depletion does not result in deleterious effects on the subject.

The high-fat diet-induced obesity (DIO) mouse model was employed. Lean mice starting a high-fat diet were concurrently treated with N-ABD094-rhArg (SEQ ID NO: 50) to test its efficacy on preventing development of DIO and associated metabolic disorders. Mice with pre-existing DIO were treated with N-ABD094-rhArg (SEQ ID NO: 50) to test its efficacy on reducing obesity, metabolic disorders, and related complications and comorbidities.

As demonstrated in the examples below, treatment with N-ABD-rhArg (SEQ ID NO: 50) resulted in reduction of weight and fat mass, improvement of glucose tolerance and insulin responsiveness, normalization of endocrine and metabolic profiles, and alleviation of inflammation, steatosis, and prevent or reverse whitening of brown fat.

An arginine depleting agent for use in treating at least one condition selected from the group consisting of obesity, a metabolic disorder, and a related complication and/or comorbidity can also include the prevention of at least one condition selected from the group consisting of obesity, a metabolic disorder, and a related complication and/or comorbidity.

The metabolic disorder can include obesity, hypercholesterolemia, dyslipidemia, steatosis, insulin resistance, glucose intolerance, hyperglycemia, and diabetes or combinations thereof and related complications and/or comorbidities can include diabetic nephropathy, diabetic vasculopathy, diabetic neuropathy, steaohepatitis, hypertension, cardiovascular disease, and whitening of brown fat or a combination thereof.

In certain embodiments, the metabolic disorder is the treatment and/or prevention of insulin resistance, e.g., in subjects having type I or particularly type II diabetes. As the examples demonstrate below, arginine depletion results in an increase in insulin sensitivity of a subject.

The method of treating obesity can include reducing fat mass or preventing the gain of fat mass in a subject. Treatment of a subject with an arginine depleting agent can result in the reduction of mRNA expression levels of several adipogenic transcription factor and lipogenic enzymes in visceral white adipose tissue (WAT), liver and skeletal muscle, such as *Pparg, Srebp1c, Acc,* and *Scd1* as compared with untreated subjects on a high fat diet. In certain embodiments, the expression level of the reduction of mRNA expression levels of regulators of lipogensis is reduced by, e.g., at least about 10%, about 20%, about 30%, about 40%, about 50%, about 60%, about 70%, about 80%, or about 90% of corresponding mRNA expression levels in an untreated subject on a high fat diet. In certain embodiments, treatment of a subject with an arginine depleting agent results in the reduction of mRNA expression of one or more genes selected from the group consisting of *Pparg, Srebp1c, Acc,* and *Scd1* in the WAT, liver and/or skeletal muscles of the subject.

Obesity can be the result of and/or associated with a metabolic disorder (such as, hyperglycemia, hyperinsulinemia) and/or other factors, such as over eating, lack of physical exercise, etc.

The metabolic disorder can also include the treatment and/or prevention of steatosis in a subject, such as cardiac steatosis, hepatic steatosis, renal steatosis, pancreatic steatosis, and muscular steatosis.

The metabolic disorder may also involve the treatment of hypercholesterolemia, dyslipidemia, e.g., by the reduction of plasma total cholesterol, LDL cholesterol, apolipoprotein B and/or triglyceride levels.

Treatment of a subject with an arginine depleting agent can reduce the concentration of plasma leptin, e.g., at least about 10%, about 20%, about 30%, about 40%, about 50%, about 60%, or about 70% of the corresponding concentration of plasma leptin in an untreated subject on a HFD. In certain embodiments, the concentration of plasma leptin can be reduced by about 10% to about 70%, about 10% to about 60%, about 10% to about 50%, about 10% to about 40%, about 10% to about 30% of the corresponding concentration of plasma leptin in an untreated subject on a HFD.

Treatment of a subject with an arginine depleting agent can reduce the concentration of plasma triglycerides by, e.g., at least about 20%, about 30%, or about 40% of the corresponding concentration of plasma triglycerides in an untreated subject on a HFD. In certain embodiments, the concentration of plasma triglycerides can be reduced by about 5% to about 40%, about 5% to about 30%, or about 5% to about 20% of the corresponding concentration of plasma triglycerides in an untreated subject on a HFD.

Treatment of a subject with an arginine depleting agent can reduce the concentration of plasma total cholesterol by, e.g., at least about 5%, about 10%, or about 15% of the corresponding concentration of plasma total cholesterol in an untreated subject on a HFD. In certain embodiments, the concentration of plasma total cholesterol can be reduced by about 5% to about 15%, about 5% to about 10%, or about 10% to about 15% of the corresponding concentration of plasma total cholesterol in an untreated subject on a HFD.

Treatment of a subject with an arginine depleting agent can reduce the concentration of plasma free fatty acids by, e.g., at least about 5%, about 10%, about 15%, or about 20% of the corresponding concentration of plasma free fatty acids in an untreated subject on a HFD. In certain embodiments, the concentration of plasma free fatty acids can be reduced by about 5% to about 20%, about 5% to about 15%, or about 5% to about 10% of the corresponding concentration of plasma free fatty acids in an untreated subject on a HFD.

The concentration of plasma arginine in the subject needed to observe a therapeutic effect can vary based on numerous factors, including the condition of the subject and the type and severity of the disease and/or medical condition and/or diet composition. The selection of the target plasma arginine levels is well within the skill of a person of ordinary skill in the art. In certain embodiments, the concentration of plasma arginine is below about 100 $\mu$M, about 90 $\mu$M, about 80 $\mu$M, about 70 $\mu$M, about 60 $\mu$M, about 50 $\mu$M, about 40 $\mu$M, about 30 $\mu$M, about 20 $\mu$M, about 10 $\mu$M, or about 5 $\mu$M. In certain embodiments, the concentration of plasma arginine is about 0.1 $\mu$M to about 100 $\mu$M, about 0.1 $\mu$M to about 90 $\mu$M, about 0.1 $\mu$M to about 80 $\mu$M, about 0.1 $\mu$M to about 70 $\mu$M, about 0.1 $\mu$M to about 60 $\mu$M, about 0.1 $\mu$M to about 50 $\mu$M, about 0.1 $\mu$M to about 40 $\mu$M, about 0.1 $\mu$M to about 30 $\mu$M, about 0.1 $\mu$M to about 20 $\mu$M, or about 0.1 $\mu$M to about 10 $\mu$M. In certain embodiments, the level of arginine is below the detection limit of the Biochrom 30 Amino Acid Analyzer (e.g., below about 3 $\mu$M) and/or below the detection limit of the Agilent 6460 Liquid Chromatography/Electrospray Ionization Triple Quadrupole Mass Spectrometer (e.g., lower than about 0.3 $\mu$M).

The determination of the duration of treatment, e.g., the duration of time the plasma arginine concentrations are maintained in a depleted state in the subject, is well within the skill of a person of ordinary skill in the art. In certain embodiments, the duration of treatment is about 1, about 2, about 3, about 4, about 8, about 12, about 16, about 20, about 24, about 28, about 32, about 36, about 40, about 44, about 48, about 52, about 56 weeks, or longer.

Depletion of arginine has been shown to be an effective treatment of a number of conditions, including but not limited to viral infections (US 8,507,245 ), multiple sclerosis, rheumatoid arthritis, autoimmune diseases (US 9,789,169), congenital hyperargininemia (ClinicalTrials.gov Identifier: NCT02488044), graft-versus-host disease (GvHD) (Hallet W., et al., Exploiting arginase to prevent GVHD, Blood 2010 116:5440-5441), inflammation (Sahin E., et al., Macrophage PTEN regulates expression and secretion of arginase I modulating innate and adaptive immune responses, J Immunol. 2014 Aug 15;193(4):1717-27), and retinal neurovascular degeneration (Fouda AY. et al., Arginase 1 promotes retinal neurovascular protection from ischemia through suppression of macrophage inflammatory responses Cell Death Dis. 2018 Sep 25;9(10):1001).

Additional objects, advantages, and novel features of this disclosure will become apparent to those skilled in the art upon examination of the following examples thereof, which are not intended to be limiting.

**Example 1: Construction of ABD and ABD094 fusion genes**

The gene of N-ABD for N-terminal fusion was constructed by mixing primers 01-08 (SEQ ID NO: 1-8) listed in **Table 1** in a PCR reaction with each primer to a concentration of 20 nM, together with 1X reaction buffer of iProof DNA polymerase (Bio-rad), 0.2 mM dNTP and 0.5 unit iProof DNA polymerase (Bio-rad) in a 50 μL reaction volume. PCR program was set as: (1) 98 °C, 10 s; (2) 50 °C, 20 s; (3) 72 °C, 15 s; (4) repeat (1) to (3) 35 times. Then 1 μL of PCR reaction product was added to a new PCR reaction together with 1X reaction buffer of iProof DNA polymerase (Bio-rad), 0.2 mM dNTP, 0.2 μM primer (N-ABDNde-F): 5'-GATCTTAAGCATATGCATCATCACCATC-3' (SEQ ID NO: 9), primer (N-ABDHind-R): 5'-ACAGCTAAAAGCTTATCAGCCTAGGATCCGCCGCTACCATTG-3' (SEQ ID NO: 93) and 0.5 unit iProof DNA polymerase in a 50 μL reaction volume. PCR program was set as: (1) 98 °C, 10 s; (2) 50 °C, 20 s; (3) 72 °C, 15 s; (4) repeat (1) to (3) 35 times; (5) 72 °C, 5 minutes. The construction of N-ABD094 gene was the same as the procedures for the N-ABD gene construction except that primers 09-16 (SEQ ID NO: 9-16) listed in **Table 1** were used instead of primers 01-08 (SEQ ID NO: 1-8) listed in **Table 1.**

The gene of C-ABD for C-terminal fusion was constructed by mixing primers 17-24 listed in **Table 1** in a PCR reaction with each primer to a concentration of 20 nM, together with 1X reaction buffer of iProof DNA polymerase, 0.2 mM dNTP and 0.5 unit iProof DNA polymerase in a 50 μL reaction volume. PCR program was set as: (1) 98 °C, 10 s; (2) 50 °C, 20 s; (3) 72 °C, 15 s; (4) repeat (1) to (3) 35 times. Then 1 μL of PCR reaction product was added to a new PCR reaction together with 1X reaction buffer of iProof DNA polymerase, 0.2 mM dNTP, 0.2 μM primer (C-ABDNde-F): 5'-TAGCTG ATCATATGTTATGCGATGGATCCAGTAACAAC-3' (SEQ ID NO: 91), 0.2 μM primer (C-ABDHind-R): 5'-GACCCTAAA AGCTTAATGGTGATGGTGATGATG-3' (SEQ ID NO: 92) and 0.5 unit iProof DNA polymerase in a 50 μL reaction volume. PCR program was set as: (1) 98 °C, 10 s; (2) 50 °C, 20 s; (3) 72 °C, 15 s; (4) repeat (1) to (3) 35 times; (5) 72 °C, 5 minutes. The construction of C-ABD094 gene was the same as the procedures for the C-ABD gene construction except that primers 25-32 (SEQ ID NO: 25-32) listed in **Table 1** were used instead of primers 17-24 (SEQ ID NO: 17-24) listed in **Table 1.**

Either the PCR fragment N-ABD, N-ABD094, C-ABD or C-ABD094 was digested with restriction enzymes Nde I and Hind III and ligated into vector pRSET-lac pre-digested with the same enzymes and transformed into *Escherichia coli* Top10 to generate plasmid pN-ABD, pN-ABD094, pC-ABD and pC-ABD094 respectively. Plasmid pRSET-lac was modified from pRSET-A (Invitrogen) by replacing the T7 promoter with lac promoter from pGEMT-Easy Vector (Promega).

N-ABD or N-ABD094 fusion of the human arginase I gene was constructed by mixing 0.2 μM primer HARGBam-F (5'-TTAGCTGGGGATCCGCCAAGTCCAGAACCATAGG-3') (SEQ ID NO: 93), 0.2 μM primer HARGHind-R (5'-GAG ATCAAAGCTTACTTAGGTGGGTTAAGGTAGTC-3') (SEQ ID NO: 94) together with 1X reaction buffer of iProof DNA polymerase, 0.2 mM dNTP, 100 ng cDNA of human arginase I and 0.5 unit iProof DNA polymerase in a 50 μL reaction volume. PCR program was set as: (1) 98 °C, 10 s; (2) 50 °C, 20 s; (3) 72 °C, 30 s; (4) repeat (1) to (3) 35 times; (5) 72 °C, 5 minutes. The PCR fragment human arginase I gene was digested with restriction enzymes BamH I and Hind III and ligated into vector pN-ABD or pN-ABD094 pre-digested with the same enzymes and transformed into *Escherichia coli* ER2566 (NEB) to generate plasmid pN-ABD-rhArg and pN-ABD094-rhArg respectively.

C-ABD or C-ABD094 fusion of the human arginase I gene was constructed by mixing 0.2 μM primer HARGNde-F (5'-TAGGCTGCATATGAGCGCCAAGTCCAGAACCATAG-3') (SEQ ID NO: 95), 0.2 μM primer HARGBam-R (5'-ATC AGCTAGGATCCCTTAGGTGGGTTAAGGTAGTCAATAGG-3') (SEQ ID NO: 96) together with 1X reaction buffer of iProof DNA polymerase (Bio-rad), 0.2 mM dNTP, 100 ng cDNA of human arginase I and 0.5 unit iProof DNA polymerase (Bio-rad) in a 50 μL reaction volume. PCR program was set as: (1) 98 °C, 10 s; (2) 50 °C, 20 s; (3) 72 °C, 30 s; (4) repeat (1) to (3) 35 times; (5) 72 °C, 5 minutes. The PCR fragment human arginase I gene was digested with restriction enzymes Nde I and BamH I and ligated into vector pC-ABD or pC-ABD094 pre-digested with the same enzymes and transformed into *Escherichia coli* ER2566 (NEB) to generate plasmid pC-ABD-rhArg and pC-ABD094-rhArg respectively.

N-ABD or N-ABD094 fusion of the *Bacillus caldovelox* arginase (BCA) gene was constructed by mixing 0.2 µM primer BCABam-F (5'-ATGCTAGTGGATCCATGAAGCCAATTTCAATTATCG -3') (SEQ ID NO: 97), 0.2 µM primer BCAHind-R (5'-TCAGCCTAAAGCTTACATGAGTTTTTCACCAAACAACG-3') (SEQ ID NO: ) together with 1X reaction buffer of iProof DNA polymerase, 0.2 mM dNTP, 100 ng genomic DNA of *Bacillus caldovelox* and 0.5 unit iProof DNA polymerase in a 50 µL reaction volume. PCR program was set as: (1) 98 °C, 10 s; (2) 50 °C, 20 s; (3) 72 °C, 30 s; (4) repeat (1) to (3) 35 times; (5) 72 °C, 5 minutes. The PCR fragment *Bacillus caldovelox* arginase gene was digested with restriction enzymes BamH I and Hind III and ligated into vector pN-ABD or pN-ABD094 pre-digested with the same enzymes and transformed into *Escherichia coli* ER2566 (NEB) to generate plasmid pN-ABD-BCA and pN-ABD094-BCA respectively.

C-ABD or C-ABD094 fusion of the *Bacillus caldovelox* arginase (BCA) gene was constructed by mixing 0.2 µM primer BCANde-F (5'-ATGCTAGCCATATGAAGCCAATTTCAATTATCGGG -3') (SEQ ID NO: 99), 0.2 µM primer BCABam-R (5'-TCAGCTAAGGATCCCATGAGTTTTTCACCAAACAACG -3') (SEQ ID NO: 100) together with 1X reaction buffer of iProof DNA polymerase 0.2 mM dNTP, 100 ng genomic DNA of *Bacillus caldovelox* and 0.5 unit iProof DNA polymerase in a 50 µL reaction volume. PCR program was set as: (1) 98 °C, 10 s; (2) 50 °C, 20 s; (3) 72 °C, 30 s; (4) repeat (1) to (3) 35 times; (5) 72 °C, 5 minutes. The PCR fragment *Bacillus caldovelox* arginase gene was digested with restriction enzymes Nde I and BamH I and ligated into vector pC-ABD or pC-ABD094 pre-digested with the same enzymes and transformed into *Escherichia coli* ER2566 (NEB) to generate plasmid pC-ABD-BCA and pC-ABD094-BCA respectively.

**Table 1.** DNA primers used in ABD and ABD094 gene synthesis.

| Number | Sequences |
|--------|-----------|
| 01 | 5'-GGAGATGGACATATGCATCATCACCATCACCATGATGAAGCC GTGGATGC-3' (SEQ ID NO: 1) |
| 02 | 5'-GCTAAGACTTTAGCTTCAGCTAAGGAATTCGCATCCACGGCT TCATCATG-3' (SEQ ID NO: 2) |
| 03 | 5'-CCTTAGCTGAAGCTAAAGTCTTAGCTAACAGAGAACTTGAC AAATATGGA-3' (SEQ ID NO: 3) |
| 04 | 5'-TAGGTTCTTGTAATAGTCACTTACTCCATATTTGTCAAGTTCTC TGTTAG-3' (SEQ ID NO: 4) |
| 05 | 5'-GGAGTAAGTGACTATTACAAGAACCTAATCAACAATGCCAA AACTGTTGA-3' (SEQ ID NO: 5) |
| 06 | 5'-AAATTTCATCTATCAGTGCTTTTACACCTTCAACAGTTTTGG CATTGTTG-3' (SEQ ID NO: 6) |
| 07 | 5'-GTAAAAGCACTGATAGATGAAATTTTAGCTGCATTACCTTC GGGTAGTAA-3' (SEQ ID NO: 7) |
| 08 | 5'-TCCGCCGCTACCATTGTTATTATTGTTGTTACTACCCGAAG GTAATGCAG-3' (SEQ ID NO: 8) |
| 09 | 5'-GATCTTAAGCATATGCATCATCACCATCACCATGATGAAGCG GTGGA-3' (SEQ ID NO: 9) |

(continued)

| Number | Sequences |
|---|---|
| 10 | 5'-GCTTCTTTCGCTTCCGCCAGGCTGTTCGCATCCACCGCTTCATCATG-3' (SEQ ID NO: 10) |
| 11 | 5'-GCGGAAGCGAAAGAAGCGGCGAACGCGGAACTGGATAGCTATGGCGTGAG-3' (SEQ ID NO: 11) |
| 12 | 5'-TTATCAATCAGGCGTTTATAAAAATCGCTCACGCCATAGCTATCCAGTTC-3' (SEQ ID NO: 12) |
| 13 | 5'-GATTTTTATAAACGCCTGATTGATAAAGCGAAAACCGTGGAAGGCGTGGA-3' (SEQ ID NO: 13) |
| 14 | 5'-CGGCAGCGCCGCCAGAATCGCATCTTTCAGCGCTTCCACGCCTTCCACGGT-3' (SEQ ID NO: 14) |
| 15 | 5'-TTCTGGCGGCGCTGCCGTCGGGTAGTAACAACAATAATAACAATGGTAGC-3' (SEQ ID NO: 15) |
| 16 | 5'-GGATCCGCCGCTACCATTGTTATTATTGTTGTTACTACC-3' (SEQ ID NO: 16) |
| 17 | 5'-CATATGTTAGCTGATGGATCCAGTAACAACAATAATAACAATGGTAGCGG-3' (SEQ ID NO: 17) |
| 18 | 5'-AATTCGCATCCACGGCTTCATCACCGCCGCTACCATTGTTATTATTGTTG-3' (SEQ ID NO: 18) |
| 19 | 5'-GAAGCCGTGGATGCGAATTCCTTAGCTGAAGCTAAAGTCTTAGCTAACAG-3' (SEQ ID NO: 19) |
| 20 | 5'-CTTACTCCATATTTGTCAAGTTCTCTGTTAGCTAAGACTTTAGCTTCAGC-3' (SEQ ID NO: 20) |
| 21 | 5'-GAGAACTTGACAAATATGGAGTAAGTGACTATTACAAGAACCTAATCAAC-3'(SEQ ID NO: 21) |
| 22 | 5'-TACACCTTCAACAGTTTTGGCATTGTTGATTAGGTTCTTGTAATAGTCAC-3' (SEQ ID NO: 22) |

(continued)

| Number | Sequences |
|---|---|
| 23 | 5'-GCCAAAACTGTTGAAGGTGTAAAAGCACTGATAGATGAAA TTTTAGCTGC-3' (SEQ ID NO: 23) |
| 24 | 5'-ATGGTGATGGTGATGATGAGGTAATGCAGCTAAAATTTCA TCTATCAGTG-3' (SEQ ID NO: 24) |
| 25 | 5'-CATATGTTATGCGATGGATCCAGTAACAACAATAATAACAA TGGTAGCGG-3' (SEQ ID NO: 25) |
| 26 | 5'-TGTTCGCATCCACCGCTTCATCACCGCCGCTACCATTGTTATT ATTGTTG-3' (SEQ ID NO: 26) |
| 27 | 5'-GAAGCGGTGGATGCGAACAGCCTGGCGGAAGCGAAAGAA GCGGCGAACGC-3' (SEQ ID NO: 27) |
| 28 | 5'-AAAATCGCTCACGCCATAGCTATCCAGTTCCGCGTTCGCCGCT TCTTTCG-3' (SEQ ID NO: 28) |
| 29 | 5'-CTATGGCGTGAGCGATTTTTATAAACGCCTGATTGATAAAGC GAAAACCG-3' (SEQ ID NO: 29) |
| 30 | 5'-TCGCATCTTTCAGCGCTTCCACGCCTTCCACGGTTTTCGCTTT ATCAATC-3' (SEQ ID NO: 30) |
| 31 | 5'-GAAGCGCTGAAAGATGCGATTCTGGCGGCGCTGCCGCATCAT CACCATCA-3' (SEQ ID NO: 31) |
| 32 | 5'-CTAAAAGCTTAATGGTGATGGTGATGATGCGGCAG-3' (SEQ ID NO: 32) |

**Example 2: Expression and purification of ABD-rhArg or ABD094-rhArg fusion protein by fermentation**

To produce N-ABD-rhArg (SEQ ID NO: 49) fusion protein, *Escherichia coli* ER2566 transformed with pN-ABD-rhArg was grown in 50 mL seed medium (1.5 g yeast extract, 0.25 g NaCl and 5 mg ampicillin) at 30 °C in an orbital shaker at 250 rpm for 16 hours. The seed culture was then transferred to the 1 L fermentation medium (10 g yeast extract, 16 g tryptone, 6.7 g $Na_2HPO_4.7H_2O$, 3.41 g $KH_2PO_4$, 2.41 g $NH_4Cl$, 0.67 g $(NH_4)_2SO_4$, 10 g glycerol, 1 g glucose, $MgSO_4.7H_2O$, 1 mM $CaCl_2$ and 0.1 g ampicillin) and grown at 28°C supplied with 1L/min air and with a minimum rate of 500 rpm, pH was maintained at 7.4. The stir rate was increased automatically in order to maintain the minimum dissolved oxygen $pO_2$ at least 20 %. When the absorbance at 600 nm wavelength of the culture reached an optical density (OD) of 15, 500 mL medium (10 g yeast extract, 16 g tryptone, 2.41 g $NH_4Cl$, 0.67 g $(NH_4)_2SO_4$, 20 g glycerol) was fed at the rate of 31.25 mL/hr for a continuous 16 hours. The cells were collected by centrifugation and the protein was purified by a Ni-IDA sepharose column. Usually more than 1 g/L of culture of purified protein was obtained. Endotoxin was then removed from the purified protein by Triton X-114 method [Aida Y. and Pabst M.J. (1990) Journal of Immunological Methods. 132: 191-195,]. The procedures for the production of fusion proteins N-ABD094-rhArg (SEQ ID NO: 50), C-ABD-rhArg (SEQ ID NO: 51), C-ABD094-rhArg (SEQ ID NO: 52), N-ABD-BCA (SEQ ID NO: 53), N-ABD094-BCA (SEQ ID NO: 54), C-ABD-BCA (SEQ ID NO: 55) and C-ABD094-BCA (SEQ ID NO: 56) were the same as the production procedures for N-ABD-rhArg (SEQ ID NO: 49) except that the cells were transformed with the respective plasmid accordingly. FIG. 1 and **Table 2** shows an example of the protein purification of the above-mentioned albumin binding domain recombinant human arginase (rhArg), *i.e.* N-ABD094-rhArg (SEQ ID NO: 50).

**Table 2.** The N-ABD094-rhArg protein was prepared and 990 mg pure protein was obtained.

|  | Vol. (mL) | Conc. (mg/mL) | Total (mg) | Specific Act. (U/mg) |
|---|---|---|---|---|
| N-ABD094-rhArg | 50 | 19.81 | 990 | 184 |

FIG. 12. shows the results of a preparation. 709 mg of N-ABD094-rhArg (SEQ ID NO: 50) was obtained, with specific activity of 205 U/mg and protein concentration of 10.5 mg/mL.

**Example 3: Construction and cloning of the N-ABD094-rhArg gene (SEQ ID NO: 38)**

The N-ABD094-rhArg (SEQ ID NO: 38) gene was synthesized by overlapping extension PCR based on the N-ABD-rhArg gene (SEQ ID NO: 37). Two PCR reactions were performed. First of all, pRSET/lac plasmid with N-ABD094-rhArg gene (SEQ ID NO: 38) was used. The rhArg region was isolated and amplified in the first PCR reaction by ABD094-0F (SEQ ID NO: 57) and HuArgHinBam-R (SEQ ID NO: 64). The PCR product was isolated and purified by 0.7 % agarose gel (Biorad) with 100 V for 30 min and gel band purification kit (GE Healthcare Life Science). ABD094 was then added to the PCR product via the second PCR reaction. A 20x diluted primers mixture, including ABD094-0F, ABD094-1F, ABD094-2F, ABD094-3F, ABD094-4F and ABD094-5F (SEQ ID NO: 57-62) was first prepared with sterilized milli-Q water. The primer mixture and the abdNde-F overlap to each other. The second PCR reaction was performed by abdNde-F (SEQ ID NO: 63), HuArgHinBam-R (SEQ ID NO: 64), and 20x diluted primers mixture. The PCR product was isolated and purified by same methods described above. The sequences of the primers are shown in **Table 3.** The binding locations of the primers are illustrated in FIG. 2A. The DNA sequence of the N-ABD094-rhArg fusion gene (SEQ ID NO: 38) obtained and its protein sequence is shown in FIG. 2B.

**Table 3.** List of primers for cloning N-ABD094-rhArg gene (SEQ ID NO: 38)

| Primer name | Sequence |
|---|---|
| ABD094-0F | 5'-ATTCTGGCAGCGCTGCCGTCGGGTAG TAACAACAATAATAACA ATGGTAG-3' (SEQ ID NO: 57) |
| ABD094-1F | 5'-AACCGTGGAAGGTGTGGAAGCGCTGAAA GATGCGATTCTGG CAGCGCTGC-3' (SEQ ID NO: 58) |
| ABD094-2F | 5'-GATTTTTATAAACGTCTGATTGATAAAGCGAA AACCGTGGAAGGTGTGGA-3' (SEQ ID NO: 59) |
| ABD094-3F | 5'-CTGGATAGCTATGGCGTGAGCGATTTTTATAAACG TCTGATTGATAAAGC-3' (SEQ ID NO: 60) |
| ABD094-4F | 5'-GCGGAAGCGAAAGAAGCAGCAAACGCGGAACTG GATAGCTATGGCGTGAG-3' (SEQ ID NO: 61) |
| ABD094-5F | 5'-ACCATGATGAAGCCGTGGATGCGAATTCC CTGGCGGAAGCGAAAGAAGCA-3' (SEQ ID NO: 62) |

(continued)

| Primer name | Sequence |
|---|---|
| abdNde-F | 5'-GGAGATATACATATGCATCATCACCATCACCAT GATGAAGCCGTGG-3' (SEQ ID NO: 63) |
| HuArgHinBam-R | 5'-TGACTACCTTAACCCACCTAAGTAAGCTT GGATCCTGTAACG-3' (SEQ ID NO: 64) |

The PCR product and the pRSET/lac vector were double digested by BamHI-HF and NdeI (New England BioLabs) at 37 °C for 2 h. Both insert and vector were isolated and purified by 0.7 % agarose gel (Biorad) with 100 V for 30 min and gel band purification kit (GE Healthcare Life Science). The insert and vector were then ligated by T4 ligase (New England BioLabs) at room temperature for 20 min. The competent cells were transformed by the recombinant DNA and spread on the LB plate with 50 µg/mL kanamycin. The LB plate was incubated at 37 °C overnight. 8 colonies were picked and checked for the presence of N-ABD094-rhArg by PCR with the abdNde-F and HuArgHinBam-R. The colonies with positive result in PCR were further grown in 5 mL 2x TY with 50 µg/mL kanamycin at 30 °C overnight. The bacterial broth was centrifuged down in 1.7 mL microcentrifuge tube at 16,100 x g for 1 min. The bacterial pellets were resuspended in solubilization buffer (50 mM Tris-HCl, 0.1 M NaCl, 1 mM MnCl$_2$, pH 7.4) and broken by repeating freeze and thaw in liquid nitrogen. The cell lysate was centrifuged down to obtain protein solution. Arginase activity assay by DAMO was performed to test the presence of arginase.

**Example 4: Construction of BCA expression vector**

The gene encoding BCA (Pubmed accession no.: U48226) was ordered from GenScript where it was synthesized by *de novo* and inserted into pUC57 cloning vector. The single cysteine residue was introduced by site-directed mutagenesis while the six-histidine tag was added to the C-terminus to facilitate the protein purification process. The engineered BCA gene was subcloned into the pET-3a vector and transformed into BL21(DE3) competent cells for BCA expression. Crystal structure of BCA (PDB: 2CEV) can be retrieved from the protein data bank. The protein sequence of the engineered BCA (S161C)-His (SEQ ID NO: 89) is shown in FIG. 3.

**Example 5: Construction of BCA-ABD expression vector**

A pair of primers encoding the albumin binding domain (ABD) were constructed and amplified by overlap PCR. Briefly, 200 ng of primers was used as template and amplified by 1 unit of iProof High-Fidelity DNA Polymerase (Bio-Rad). The primers were incubated at 98°C for 30 s while 15 cycles of amplification including heat denaturation at 98 °C for 10 s, annealing at 60 °C for 30 s and extension at 72 °C for 15 s.

**5'-Sense-ABD**

GCGCAGCATGATGAAGCCGTGGATGCGAACAGCTTAGCTGAAGCTAAAGTCT TAGCTAACAGAGAACTTGACAAATATGGAGTAAGTGACTATTACAAGAACC (SEQ ID NO: 77)

**5'-Anti-sense-ABD**

TTAAGGTAATGCAGCTAAAATTTCATCTATCAGTGCTTTTACACCTTCAACAG TTTTGGCATTGTTGATTAGGTTCTTGTAATAGTCACTTACTCCAT (SEQ ID NO: 78)

The ABD encoding gene constructed by this pair of primers was amplified by 2 different sets of primers so as to construct BCA-6xHis-ABD (also referred to as BHA) (SEQ ID NO: 75) and BCA-ABD-6xHis (also referred to as BAH) (SEQ ID NO: 76). For the construction of BHA, the ABD was first amplified by the following pair of primers for which the forward primer was designed for the overlap PCR with the BCA via the six-Histidine tag while BamH I restriction site and stop codon were introduced for the ligation to pET-3a vector using the reverse primer:

**Forward primer for cloning ABD** :
5'- GCATCACCATCACCATCACGCGCAGCATGATGAAG-3' (SEQ ID NO: 79)
**Reverse primer for cloning ABD:**
*5'- cgGGATCCTTAAGGTAATGCAGCTAAAATTTCATCTAT-3'* (SEQ ID NO: 80)

The PCR product from the amplification of ABD was subjected to 1.5% agarose gel electrophoresis and the PCR products were purified. While the engineered BCA with an extra mutation (V20P) was cloned using the following pair of primers for which the stop codon was deleted:

**Forward primer for cloning BCA:**
5'-ggaattccCATATGAAGCCAATTTCAATTATCG-3' (SEQ ID NO: 81)
**Reverse primer for cloning BCA:**
5'- GTGATGGTGATGGTGATGCA-3' (SEQ ID NO: 82)

The BCA amplified by the above primers was subjected to 1% agarose gel electrophoresis and the PCR products were purified. The above PCR products of BCA and ABD was tethered by overlap PCR. Similarly, 200 ng of BCA and ABD was used as template and amplified by 1 unit of iProof High-Fidelity DNA Polymerase. The BCA and ABD were incubated at 98 °C for 30 s while 15 cycles of amplification including heat denaturation at 98 °C for 10 s, annealing at 60 °C for 30 s and extension at 72 °C for 30 s. The product from overlap PCR was used as template for further amplification using the following pair of primers:

**Forward primer for cloning BHA:**
5'-ggaattccCATATGAAGCCAATTTCAATTATCG-3' (SEQ ID NO: 83)

**Reverse primer for cloning BHA:**
5'-cgGGATCCTTAAGGTAATGCAGCTAAAATTTCATCTAT-3' (SEQ ID NO: 84)

The PCR product was subjected to 1% agarose gel electrophoresis and the band corresponding to BHA was excised from the gel for purification.
For the construction of BAH, the ABD was first amplified by the following pair of primers for which the forward primer was designed for the overlap PCR directly with the BCA while BamHI restriction site, six-Histidine tag and stop codon were introduced via the reverse primer:

**Forward primer for cloning ABD:**
5'-CGTTGTTTGGTGAAAAACTCATGGCGCAGCATGATGAAG -3' (SEQ ID NO: 85)

**Reverse primer for cloning ABD:**

5'-cg*GGATCC*TTAGTGATGGTGATGGTGATGAGGTAATGCAGCTAAAATTTCA

TCTAT-3' (SEQ ID NO: 86)

The PCR product from the amplification of ABD was subjected to 1.5% agarose gel electrophoresis and the PCR products were purified. The engineered BCA with an extra mutation (V20P) was cloned using the following pair of primers for which the stop codon and six-Histidine tag were deleted:

**Forward primer for cloning BCA:**
5'-ggaattcc*CATAT*GAAGCCAATTTCAATTATCG-3' (SEQ ID NO: 81)

**Reverse primer for cloning BCA:**
5'- GTGATGGTGATGGTGATGCA-3' (SEQ ID NO: 82)

The BCA amplified by the above primer was subjected to 1% agarose gel electrophoresis and the PCR products were purified. The above PCR product of BCA and ABD was tethered by overlap PCR. Similarly, 200 ng of BCA and ABD was used as template and amplified by 1 unit of iProof High-Fidelity DNA Polymerase. The BCA and ABD were incubated at 98 °C for 30 s while 15 cycles of amplification including heat denaturation at 98°C for 10 s, annealing at 60 °C for 30 s and extension at 72 °C for 30 s. The product from overlap PCR was used as template for further amplification using the following pair of primers:

**Forward primer for cloning BAH:**
5'-ggaattcc*CATATG*AAGCCAATTTCAATTATCG-3' (SEQ ID NO: 87)
**Reverse primer for cloning BAH:**

5'-cg*GGATCC*TTAGTGATGGTGATGGTGATGAGGTAATGCAGCTAAAATTTCAT
CTAT-3' (SEQ ID NO: 88)

The PCR product was subjected to 1% agarose gel electrophoresis and the band corresponding to BHA was excised from the gel for purification. The PCR product encoding BHA and BAH and the pET-3a vector were double digested with NdeI and BamHI. The BHA and BAH were ligated to the pET-3a vector and transformed to TOP10 competent *E. coli* cells. The nucleotide sequences of BHA and BAH were confirmed by DNA sequencing. The pET-3a vector containing the BHA or BAH insert was transformed to the BL21(DE3) vector for protein expression.

**Example 6: Protein constructs of BHA and BAH**

The protein sequence of BCA used in this study is the same as that used by Bewley et al. (Structure 7, 435-448, 1999) (PDB ID: 2CEV) except that it contains a 6xHis tag at the C terminus and a S161C mutation. The constructs of both BCA-6xHis-ABD (BHA) and BCA-ABD-6xHis (BAH) were designed and *E. coli* cell stocks can express BHA and BAH. In both BHA and BAH, the BCA portion is a mutated version of BCA with its valine residue at position 20 mutated to proline (V20P), while the ABD portion is the wild-type version of ABD with a linker sequence (AQHDEAVDANS) (Jonsson et al., 2008, Protein Eng. Des. Sel. 21, 515-527). The ABD portion is either located at the very C terminus of the fusion protein (in the case of BHA) or fused in between BCA and 6x histidine tag (in the case of BAH). The alignment of the protein sequences of BCA, BHA, and BAH used in this study is shown in FIG. 4.

BCA (wild-type) amino acid sequence (PDB ID: 2CEV):

MKPISIIGVPMDLGQTRRG<u>V</u>DMGPSAMRYAGVIERLERLHYDIEDLGDIPIGKAE
RLHEQGDSRLRNLKAVAEANEKLAAAVDQVVQRGRFPLVLGGDHSIAIGTLAG
VAKHYERLGVIWYDAHGDVNTAETSPSGNIHGMPLAASLGFGHPALTQIGGY<u>S</u>P
KIKPEHVVLIGVRSLDEGEKKFIREKGIKIYTMHEVDRLGMTRVMEETIAYLKER
TDGVHLSLDLDGLDPSDAPGVGTPVIGGLTYRESHLAMEMLAEAQIITSAEFVEV
NPILDERNKTASVAVALMGSLFGEKLM (SEQ ID NO: 70)

BCA (S161C)-His amino acid sequence:

MKPISIIGVPMDLGQTRRG<u>V</u>DMGPSAMRYAGVIERLERLHYDIEDLGDIPIGKAE
RLHEQGDSRLRNLKAVAEANEKLAAAVDQVVQRGRFPLVLGGDHSIAIGTLAG
VAKHYERLGVIWYDAHGDVNTAETSPSGNIHGMPLAASLGFGHPALTQIGGY<u>C</u>P
KIKPEHVVLIGVRSLDEGEKKFIREKGIKIYTMHEVDRLGMTRVMEETIAYLKER
TDGVHLSLDLDGLDPSDAPGVGTPVIGGLTYRESHLAMEMLAEAQIITSAEFVEV
NPILDERNKTASVAVALMGSLFGEKLMHHHHHH (SEQ ID NO: 71)

**BHA** amino acid sequence:

MKPISIIGVPMDLGQTRRG<u>P</u>DMGPSAMRYAGVIERLERLHYDIEDLGDIPIGKAER
LHEQGDSRLRNLKAVAEANEKLAAAVDQVVQRGRFPLVLGGDHSIAIGTLAGV
AKHYERLGVIWYDAHGDVNTAETSPSGNIHGMPLAASLGFGHPALTQIGGY<u>C</u>PK
IKPEHVVLIGVRSLDEGEKKFIREKGIKIYTMHEVDRLGMTRVMEETIAYLKERT
DGVHLSLDLDGLDPSDAPGVGTPVIGGLTYRESHLAMEMLAEAQIITSAEFVEVN
PILDERNKTASVAVALMGSLFGEKLMHHHHHHAQHDEAVDANSLAEAKVLAN
RELDKYGVSDYYKNLINNAKTVEGVKALIDEILAALP (SEQ ID NO: 75)

**BAH** amino acid sequence:

MKPISIIGVPMDLGQTRRG<u>P</u>DMGPSAMRYAGVIERLERLHYDIEDLGDIPIGKAER
LHEQGDSRLRNLKAVAEANEKLAAAVDQVVQRGRFPLVLGGDHSIAIGTLAGV

AKHYERLGVIWYDAHGDVNTAETSPSGNIHGMPLAASLGFGHPALTQIGGY<u>C</u>PK
IKPEHVVLIGVRSLDEGEKKFIREKGIKIYTMHEVDRLGMTRVMEETIAYLKERT
DGVHLSLDLDGLDPSDAPGVGTPVIGGLTYRESHLAMEMLAEAQIITSAEFVEVN
PILDERNKTASVAVALMGSLFGEKLMAQHDEAVDANSLAEAKVLANRELDKYG
VSDYYKNLINNAKTVEGVKALIDEILAALPHHHHHH (SEQ ID NO: 76)

**Example 7: Production of BHA and BAH**

*E. coli* cell stocks that expressed BHA and BAH were used. To prepare the seed culture, a small amount of *E. coli* BL21 (DE3) glycerol stock was inoculated into 10 mL LB medium supplemented with 100 $\mu$g/mL ampicillin. The seed culture was grown at 37 °C with shaking at 700 rpm for overnight (~16 h). The overnight seed culture at a volume of 2.5 mL was then used to inoculate 250 mL of LB medium with 100 $\mu$g/mL ampicillin. Inoculated cells were grown at 37 °C with shaking at 280 rpm for 2-4 h to an optical density at 600 nm wavelength ($OD_{600}$) of 0.6-0.8. IPTG (0.2 mM) was then added to the culture medium to induce the overexpression of the target protein. Cells were grown for a further 4 h and harvested by centrifugation at 4,500 rpm.

Protein purification by nickel affinity chromatography

*E. coli* cell pellets were resuspended in buffer containing 50 mM Tris-HCl, 100 mM NaCl (pH 7.4) and were disrupted by sonication. Cell debris was then removed by centrifugation at 10,000 rpm for 40 min. Supernatant was applied to a 5 mL HiTrap chelating column (GE Healthcare) that had been charged with $Ni^{2+}$ and equilibrated with buffer A (20 mM sodium phosphate, 0.5 M NaCl, pH 7.4) using an ÄKTA purifier. Proteins were eluted using a gradient of 0-0.5 M imidazole which was achieved by the combination of buffer A with buffer B (0.5 M imidazole in buffer A) at various ratios. Fractions were analyzed by sodium dodecyl sulfate polyacrylamide gel electrophoresis (SDS-PAGE) using 12% polyacrylamide gels, and those containing the target protein were pooled. Pooled fractions were concentrated and the buffer of the concentrated pooled fractions were exchanged with 20 mM sodium phosphate (pH 7.4) using centrifugal filter device (Amicon) or tangential flow filtration (TFF) device (Millipore). The membrane cut-off was 30,000 MW for BHA and BAH. The retentate was then diluted to desired concentration and underwent sterile filtration through a 0.2 $\mu$m syringe filter and stored at 4 °C.

According to the chromatogram of BHA purification, three peaks are observed during the elution process using increasing concentrations of imidazole (Figure 5A). The first two peaks are merged together, succeeded by the third peak that occurs when imidazole concentration reaches ~0.13 M (FIG. 5A). When analyzed by SDS-PAGE, it is found that the third peak mainly consists of a target protein of around 40 kDa (FIG. 5B, lanes F4-F8). This target protein is likely to be BHA since the calculated molecular weight of BHA monomer based on its amino acid sequence is 39.44 kDa. Therefore, it is suggested that BHA has been successfully expressed and purified. Fractions containing relatively pure BHA were pooled together for further processes.

According to the chromatogram of BAH purification, three peaks are observed during the elution process using increasing concentrations of imidazole (FIG. 5C). The second and third peaks are merged together and occur when imidazole concentration reaches ~0.18 M (FIG. 5C). When analyzed by SDS-PAGE, it is found that this merged peak mainly consists of a target protein of around 40 kDa (FIG. 5D, lanes F6-F14) which is likely to be BAH. Compared to BHA which is eluted at ~0.13 M imidazole, BAH seems to bind more tightly to the nickel affinity column. This is probably because the ABD domain, when inserted between BCA and the 6x histidine tag, helps to make the 6x histidine tag more exposed, and hence the BAH can interact with the nickel ions in the column more effectively than BHA. Fractions containing relatively pure BAH were pooled together and exchanged buffer using tangential flow filtration (TFF) device (Millipore) instead of a centrifugal filter device.

One unit of enzyme activity is defined as the amount of enzyme (BCA, BHA or BAH) that catalyzes the production of 1 $\mu$mol urea per min under standard assay conditions. Specific activity of the enzyme is expressed as activity units per mg of protein. Under standard DAMO assay conditions (37 °C, pH 7.4), freshly purified BHA (SEQ ID NO: 75) has a specific activity value of 220.6 $\pm$ 15.8 U/mg, which is close to the value of BCA (S161C)-His (SEQ ID NO: 89) (243.30 U/mg).

For fusion proteins comprising a BCA polypeptide and an ABD polypeptide, extremely high protein yield of about 290 mg protein per L of shake flask culture has been obtained in the case of BHA (SEQ ID NO: 75). The yields of ABD fusion proteins are highly dependent on the expression systems used. It has been reported that the yield of ABD fused with sCR1 ranges from 0.4 to 1.1 $\mu$g per 24 h per mL of Chinese hamster ovary cell culture (Makrides et al., 1996, J. Pharmacol. Exp. Ther. 277, 534-542). Around 2-15 mg single-chain diabody-ABD fusion proteins can be obtained from every L of HEK293 cell culture (Hopp et al., 2010, Protein Eng. Des. Sel. 23, 827-834; Stork et al., 2007, Protein Eng. Des. Sel. 20, 569-576). The yield of ABD-TolA fusion protein expressed in *E. coli* BL21 host is 20 mg/L LB broth medium (Ahmad et al., 2012, Proteins 80, 774-789). In comparison, the yield of BHA is much higher. Our results have demonstrated that the production of BHA (SEQ ID NO: 75) is simple and cost-effective.

## Example 8: Expression and purification of BCA (S161C)-His (SEQ ID NO: 89)

An overnight seed culture of the *E. coli* strain BL21 (DE3)pLysS transformed with plasmid pET-3a-BCA(S161C)-His was prepared in 5 mL LB medium containing 100 $\mu$g/mL ampicilin and it was allowed to grow in the shake flask at 37 °C for 16 h with 280 rpm shaking. For 250 mL LB medium containing 100 $\mu$g/mL ampicilin, 2.5 mL overnight culture was added and cultured at 37 °C with shaking at 280 rpm until the optical density ($OD_{600}$) reached 0.8-0.9 at 600 nm. Isopropyl $\beta$-D-thio-galactopyranoside (IPTG) was added to a final concentration of 0.2 mM. After 4 h induction, the cells were harvested by centrifugation at 12,000 x g for 20 min at 4 °C. The cell pellet could either be disrupted by sonication and proceed to purification, or kept at -20 °C until required. The cell pellet was suspended in 20 mL solubilization buffer (50 mM Tris-HCl, 100 mM NaCl, pH 7.4) containing 10 mM $MnCl_2$. The cells were first disrupted by sonication on ice for 10 min with pulse for every 30 s at 40% amplitude. The cell debris was removed by centrifugation at 12,000 x g for 20 min at 4 °C. The supernatant was incubated at 70 °C for 15 min and precipitates formed during the heat treatment step were removed by centrifugation. Soluble fraction was filtered through 0.22 $\mu$m filter before loading to the 5 mL HisTrap Chelating HP column. The flow through fraction was collected during sample loading and the unbound proteins were washed out by 5 column volume (CV) of starting buffer or until the baseline level off. The target proteins bound on the column was eluted out by competitive gradient elution using imidazole (0.5 M imidazole in start buffer). The target fractions were identified and pooled together which would be buffer-exchanged in 20 mM sodium phosphate at pH 7.4 using Amicon Ultra-15 Centrifugal Filter Unit with 10 kDa MW cutoff following manufacture's instruction. The sample was filtered under sterile conditions through a by 0.22 $\mu$m filter and stored at 4 °C until required.

## Example 9: Purification of BCA (S161C)-His (SEQ ID NO: 89) from fed-batch fermentation

The fermentation of BCA (S161C)-His-expressing *E. coli* cells in a 5 L bioreactor produced 248.86 g wet cell weight. The cell pellet was re-suspended in solubilization buffer containing DNase and RNase which broke down the huge amount of DNA and RNA released by homogenization, thus reducing the viscosity of the cell lysate. The cell lysate, after passing through the homogenizer for three times, was incubated at 37 °C for 15 minutes to further reduce the viscosity, followed by centrifugation to obtain the soluble fraction. The first step of purification removes the non-target impurities by heat treatment at 70 °C for 15 min which precipitated the heat-labile proteins, and the heat-stable BCA (S161C)-His (SEQ ID NO: 89) was recovered by centrifugation.

The soluble fraction containing the target protein was further purified by Ni-affinity column with 196 mL bed volume packed in XK50 column. Briefly, a 4-segment elution program was used to elute the BCA (S161C)-His (SEQ ID NO: 89) from the Ni-affinity column. The first elution gradient from 0 to 30% elution buffer was intended to remove the protein that bound non-specifically on the column. The second segment was maintained at 30% which allowed complete elution of non-specific bound proteins. The target protein was eluted out in the third segment by increasing concentration of elution buffer from 30 to 70%. The fourth segment of 70 to 100% elution buffer eluted any remaining protein that was still bound on the column. The fractions that gave absorbance at UV wavelength 280 nm was analyzed by SDS-PAGE while the 24 fractions from E1 to A'5 (50 mL each), containing the target protein, were pooled together (FIG 6A and FIG 6B). The pooled fractions containing high concentration of imidazole were desalted by tangential flow filtration (TFF) system, based on our understanding of the hexameric structure of BCA (wild-type) (SEQ ID NO: 70), membranes with 30 kDa NMWC were used for TFF. The purified BCA (S161C)-His (SEQ ID NO: 89) was maintained in PBS buffer, filtered with 0.2 $\mu$m filter under sterile condition and stored at 4 °C until use. Through the 2-step pilot scale purification, about 1096 mg of BCA (S161C)-His (SEQ ID NO: 89) protein was obtained and the specific activity was determined to be 264 U/mg.

**Example 10: Determination of arginase enzyme activity**

The activity of BCA (S161C)-His (SEQ ID NO: 89) was assayed by direct colorimetric method using diacetyl monoxime (DAMO) which determines the amount of product, urea, in the enzymatic reaction. Briefly, a serial dilution of 0.3 mL BCA solutions together with 0.15 mL water and 720 mM arginine substrate at pH 7.4 was pre-incubated separately at 37 °C for 10 min. The enzymatic reaction was carried out by adding 0.3 mL arginine substrate to the pre-incubated enzyme solutions. The reaction mixture was incubated at 37°C for exactly 5 minutes and quenching reagent [50% trichloroacetic acid (TCA)] was added to stop the reaction. The sample blanks were prepared in a similar way but the same volume of enzyme solutions was added after TCA solution.

The urea formed from the above reaction was quantified using the DAMO direct colorimetric method described by World Health Organization (WHO). This protocol has been adopted by WHO as the Standard Operating Procedures (SOP) for measuring the blood urea concentration. Several reagents were prepared regarding the SOP, mixed acid reagent was prepared by slowly adding 100 mL concentrated sulfuric acid to 400 mL distilled water followed by 0.3 mL stock acid reagent (0.5 g ferric chloride hexahydrate in 15 mL of distilled water and make up to 25 mL by concentrated phosphoric acid). Mixed color reagent was prepared by mixing 35 mL stock color reagent A (1 g DAMO in 50 mL distilled water) and 35 mL stock color reagent B (0.25 g thiosemicarbazide in 50 mL distilled water) and make up to 500 mL with distilled water. Working urea standard was prepared by adding 5 mL of stock urea standard (0.5 g urea in 50 mL benzoic acid) to 45 mL benzoic acid (1 g/dL). The DAMO assay was carried out by adding 100 $\mu$L of 20-fold diluted reaction mixture to a glass boiling tube and followed by 3 mL of color development reagent which is freshly prepared by mixing distilled water, mixed color reagent and mixed acid reagent in 1:1:1 ratio. The tubes were incubated at 100 °C for 15 min and allowed to cool to room temperature for 5 min. The absorbance at 540 nm was measured. One unit of arginase is defined as the amount of enzyme that converts a micromole of arginine to ornithine and urea per minute at 37 °C, pH 7.4. The enzyme activity was calculated using the following equations, the absorbance value of the sample was subtracted from its blank:

$$\Delta A_{540nm}\ Sample = A_{540nm}\ Sample - A_{540nm}\ Sample\ Blank$$

The amount of urea produced is calculated from the standard curve:

$$Units/ml\ enzyme = \frac{(\mu mole\ of\ urea\ liberated)\ (df)}{(5)\ (0.3)}$$

df = Dilution factor of enzyme
0.3 = volume (in milliliters) of enzyme used
5 = time (in minutes) of the assay per the unit definition

**Example 11: Determination of protein concentration**

The protein concentration was determined by Bradford's assay. The Protein Assay Dye Reagent Concentrate (Bio-rad) was diluted 5 times before use. 20 $\mu$L of sample was mixed with 1 mL diluted dye reagent and incubated at room temperature for 10 min. To determine the protein concentration, the mixture was measure at 595 nm with Quick Start Bovine Serum Albumin Standard Set (Bio-rad) as standard in the range of 0-1 mg/mL.

**Example 12: Enzymatic activity of the BHA (SEQ ID NO: 75) fusion protein**

The arginine catabolic activity of BHA (SEQ ID NO: 75) was assayed in order to find out whether the enzymatic activity would be affected in the presence of HSA. The results showed that the activity of BHA (SEQ ID NO: 75) was not affected by the various amounts of HSA added to the activity assay. The mole ratio of BHA : HSA tested in the binding studies were 1:10, 1:5, 1:1, 5:1 and 10:1. Under all these conditions, the measured specific activities of BHA (SEQ ID NO: 75) alone and the BHA-HSA complexes were very similar, and were approximately 200 U/mg.

### Example 13: Binding of N-ABD-rhArg (SEQ ID NO: 49) or BHA (SEQ ID NO: 75) to HSA revealed by non-denaturing PAGE analysis

When ABD is fused to rhArg or BCA, it is important for the fusion protein to retain both the albumin binding capacity and the enzymatic activity. To demonstrate the binding capacity of ABD in the fusion proteins, a fixed amount of human serum albumin (HSA) was incubated with N-ABD-rhArg (SEQ ID NO: 49) or BHA (SEQ ID NO: 75) in different molar ratios. The reaction mixtures were analyzed in their native condition on non-denaturing PAGE where the proteins were separated by size and conformation.

In FIG. 7, the mobility of each of HSA and N-ABD-rhArg (SEQ ID NO: 49) alone are shown in Lanes 2 and 3 of the non-denaturing PAGE, respectively (FIG. 7). A fixed amount of HSA (30 pmoles) was titrated against N-ABD-rhArg (SEQ ID NO: 49) at various molar ratios and the results are illustrated in Lanes 5-9. By increasing the amount of N-ABD-rhArg (SEQ ID NO: 49) in the binding reaction, the amount of free HSA was reduced in a proportional manner and thus indicating the specific binding of N-ABD-rhArg (SEQ ID NO: 49) to HSA. In FIG. 8, the mobility of each of HSA and BHA (SEQ ID NO: 75) alone are shown in Lanes 6 and 7 of the non-denaturing PAGE, respectively (FIG. 8). A fixed amount of HSA (60 pmoles) was titrated against BHA (SEQ ID NO: 75) at various molar ratios and the results are illustrated in Lanes 1-5. By increasing the amount of BHA (SEQ ID NO: 75) in the binding reaction, the amount of free HSA was reduced in a proportional manner and thus indicating the specific binding of BHA (SEQ ID NO: 75) to HSA.

### Example 14: *In vivo* study of BHA (SEQ ID NO: 75)

Six BALB/c mice of 8 weeks old were used for the in vivo study of BHA (SEQ ID NO: 75). Prior to the injection of BHA (SEQ ID NO: 75), blood sample was taken from each mouse and used as control (0 h). The mice were randomly divided into two groups for intraperitoneal (i.p.) and intravenous (i.v.) injection, respectively. BHA (SEQ ID NO: 75) (250 U) was injected to each mouse, and blood samples were taken at 6, 24, 72, and 120 h after injection. The plasma of each blood sample at 16 $\mu$L was analyzed by an amino acid analyzer (Biochrom) for arginine and ornithine content.

The *in vivo* arginine-depleting effect of BHA (BHA SEQ ID NO: 75) was studied in BALB/c mice and is compared with that of BCA (S161C)-His (SEQ ID NO: 89), as shown in FIG. 9. According to the results, plasma arginine level quickly returns to normal within 24 h after the injection of BCA (S161C)-His (SEQ ID NO: 89) (FIG. 9). In contrast, an undetectable level of plasma arginine has been retained for at least 24 h after injection of BHA (SEQ ID NO: 75) (FIG. 9). This result indicates that the fusion of ABD has prolonged the circulating half-life of BCA (S161C)-His (SEQ ID NO: 89) *in vivo*. The results demonstrate that ABD fusion is a feasible and promising strategy for improving the pharmacodynamic properties of arginine-depleting enzymes. The route of administration seems to have no influence on the arginine-depleting effect of BHA (SEQ ID NO: 75) *in vivo* (FIG. 9). Besides, all mice have shown good tolerance with the administration of BHA (SEQ ID NO: 75) as no obvious side effects have been observed. Taken together, the fusion proteins described herein, such as BHA (SEQ ID NO: 75), have proven the feasibility of ABD fusion strategy by decreasing plasma arginine to an undetectable level for a much longer period (at least 24 hours) than that when using native BCA (S161C)-His (SEQ ID NO: 89) (8 hours) without the ABD fusion.

### Example 15: *In vivo* pharmacodynamics study of N-ABD-rhArg (SEQ ID NO: 49)

The anti-cancer effects of arginase had been investigated long time ago (Greenberg and Sassenrath, 1953, Cancer Res. 13, 709-715). Arginase was found to have very good anti-cancer effect *in vitro*. However, nearly no effect was observed *in vivo,* due to the short plasma half-life. Plasma arginine dropped to the lowest level 3 hours after injection of native arginase to C3H mice. Arginine increased to normal basal level 5 hours after injection in the same model (Greenberg and Sassenrath, 1953, Cancer Res. 13, 709-715). Therefore, arginase was considered as an ineffective anti-cancer agent, because of the short plasma half-life. This suggested that plasma half-life can affect the potency of the enzyme drug *in vivo.* N-ABD-rhArg (SEQ ID NO: 49) and N-ABD094-rhArg (SEQ ID NO: 50) showed similar specific activity. We found that extension of plasma half-life of rhArg (SEQ ID NO: 103) via fusion with ABD is an effective method. To reduce the production cost of rhArg (SEQ ID NO: 103) with extended plasma half-life, N-ABD-rhArg (SEQ ID NO: 49) and N-ABD094-rhArg (SEQ ID NO: 50) were developed.

Fifteen female BALB/c mice (10 weeks of age) were used in this study. They were kept in groups of five per cage. A single dose of N-ABD-rhArg (SEQ ID NO: 49) (500 U, 250 U, and 125 U, respectively) was administered to the mice via i.p. injection. Blood samples were collected from the saphenous vein using heparinized capillary tubes, and were centrifuged at 800 x g to obtain plasma. Sixteen microliters of plasma sample was subjected to measurement of L-arginine concentration using the Biochrom 30 Amino Acid Analyzer. The results are shown in FIG. 10. Measuring the plasma arginine concentrations showed that 500U of N-ABD-rhArg (SEQ ID NO: 49) can deplete plasma arginine to levels below the detection limit of the Biochrom 30 Amino Acid Analyzer (3 $\mu$M) for at least 9 days, 250U of N-ABD-rhArg (SEQ ID NO: 49) can deplete serum arginine to levels below the detection limit of the Biochrom 30 Amino Acid Analyzer for at least 7 days, and 125U of N-ABD-rhArg (SEQ ID NO: 49) can deplete serum arginine to levels below the detection limit of the Biochrom 30 Amino Acid Analyzer for at least 3 days in BALB/c mice. These results clearly suggest that the plasma half-life of rhArg can be extended by fusion with ABD. In comparing with BHA (SEQ ID NO: 75) (FIG. 9), N-ABD-rhArg (SEQ ID NO: 49) can deplete arginine to a very low level in the mice for exceptionally longer period, which highlights the importance of the requirement for rational design of the fusion protein.

**Example 16: *In vivo* pharmacodynamics and pharmacokinetics study of N-ABD094-rhArg (SEQ ID NO: 50)**

Fifty C57BL/6J male mice (10-12 weeks of age) were used in this study. A single dose of 500U N-ABD094-rhArg (SEQ ID NO: 50) was administered to the mouse via i.p. injection. Blood samples were collected from the saphenous vein using heparinized capillary tubes, and were centrifuged at 800 x g to obtain plasma. Five microliters of plasma sample was subjected to measurement of L-arginine concentration by liquid chromatography-tandem mass spectrometry using the Agilent 6460 Liquid Chromatography/Electrospray Ionization Triple Quadrupole Mass Spectrometer, with a detection limit of 0.3 $\mu$M L-arginine. Five microliters of plasma sample was subjected to measurement of N-ABD094-rhArg (SEQ ID NO: 50) concentrations using Human Arginase I ELISA (Enzyme-Linked Immunosorbent Assay) Kit (Abcam) that specifically detects human arginase, and does not react with mouse arginase.

Following injection, serum was collected at Hour 2, Hour 4, Hour 8, Hour 16, Hour 24 (Day 1) and from Day 2 to Day 12 at 24-hour intervals, and from Day 12 to Day 30 at 72-hour intervals. To strike a balance between the number of animals used and to minimize the effect on results of pharmacokinetics and pharmacodynamics study caused by removal of N-ABD094-rhArg (SEQ ID NO: 50) from the mouse as a consequence of blood collection, mice were divided into 10 groups, with n = 5 in each group, and subjected to blood collection at the indicated time-point(s) shown in brackets: Group 1 (Hour 2), Group 2 (Hour 4), Group 3 (Hour 8), Group 4 (Hour 16), Group 5 (Hour 24), Group 6 (Day 2, Day 7, Day 12), Group 7 (Day 3, Day 8, Day 18), Group 8 (Day 4, Day 9, Day 21), Group 9 (Day 5, Day 10, Day 15, Day 24), Group 10 (Day 0, Day 6, Day 11, Day 27, Day 30). The pharmacodynamics and pharmacokinetics results are respectively shown in FIG. 13 and FIG. 14.

Results of measurement of plasma arginine concentrations showed that 500U of N-ABD094-rhArg (SEQ ID NO: 50) administered via i.p. injection could deplete plasma arginine concentrations to 1 $\mu$M within 2 hour after injection (FIG. 13). Plasma arginine concentrations were maintained at $\leq$ 1 $\mu$M for 10 days before a gradual rise in subsequent days **(Table 4).** Concentrations of plasma arginine was returned to half of the basal level between Day 18 - Day 21. These results suggest that N-ABD094-rhArg (SEQ ID NO: 50) can maintain plasma arginine at a very low level ($\leq$ 1 $\mu$M) in the mice for exceptionally long period.

Table 4. Plasma arginine concentrations prior to and at different time points after i.p. injection of 500U N-ABD094-rhArg (SEQ ID NO: 50)

| Time point after injection | Plasma arginine concentrations ($\mu$M) (Mean of 5 mice $\pm$ SEM) |
|---|---|
| 0 | 134.543 $\pm$ 3.791 |
| Hour 2 | 1.007 $\pm$ 0.013 |
| Hour 4 | 0.934 $\pm$ 0.021 |
| Hour 8 | 0.912 $\pm$ 0.006 |
| Hour 16 | 0.943 $\pm$ 0.010 |
| Hour 24 | 0.934 $\pm$ 0.007 |
| Day 2 | 0.944 $\pm$ 0.007 |
| Day 3 | 0.965 $\pm$ 0.008 |
| Day 4 | 0.535 $\pm$ 0.121 |
| Day 5 | 0.681 $\pm$ 0.043 |
| Day 6 | 0.896 $\pm$ 0.089 |
| Day 7 | 0.792 $\pm$ 0.135 |

(continued)

| Time point after injection | Plasma arginine concentrations ($\mu$M) (Mean of 5 mice $\pm$ SEM) |
|---|---|
| Day 8 | 0.916 $\pm$ 0.079 |
| Day 9 | 1.224 $\pm$ 0.178 |
| Day 10 | 0.721 $\pm$ 0.054 |
| Day 11 | 3.828 $\pm$ 1.511 |
| Day 12 | 7.607 $\pm$ 2.325 |
| Day 15 | 31.283 $\pm$ 4.569 |
| Day 18 | 48.648 $\pm$ 3.989 |
| Day 21 | 73.55 $\pm$ 3.76 |
| Day 24 | 76.07 $\pm$ 4.92 |
| Day 27 | 111.09 $\pm$ 6.21 |
| Day 30 | 110.91 $\pm$ 3.81 |

The pharmacokinetic parameters are calculated with two compartment model.

The elimination rate constant $k_e$ is calculated by the following equation:

$$k_e = \frac{\ln C_1 - ln C_2}{t_2 - t_1}$$

The plasma half-life $t_{1/2}$ is calculated by the following equation:

$$t_{1/2} = \frac{\ln 2}{k_e}$$

Area under the curve (AUC) is calculated by the trapezoidal rule with the following equation:

$$AUC_{t_1 - t_2} = \frac{(C_1 + C_2)}{2} \times (t_2 - t_1)$$

For comparison, we use the reported half-life of the native rhArg in literature. The fusion protein N-ABD094-rhArg (SEQ ID No: 50) had elimination half-life of 3.9 $\pm$ 0.3 days (FIG. 14), implying that they are cleared much slower than a non-fusion protein rhArg, which has a much shorter half-life. For a specific comparison, human arginase has a half-life of only a few minutes in circulation (Georgiou et al., US 8440,184 B2), indicating that the conjugation prolongs the circulation time dramatically. These results clearly suggest that the plasma half-life of rhArg can be massively extended by fusion with ABD. The circulation half-life of native ADI (a few hours) was prolonged by PEGylation (Ensor et al., Cancer Res 2002, 62:5443-5450). The PEGylated ADI had a circulation half-life of 2-3 days and only the PEGylated ADI (not the native ADI) was effective in inhibiting growth of tumors *in vivo*. In comparing native rhArg and PEGylated rhArg administered to rats via i.p. injection, 1,500 U of PEGylated rhArg per rat (6 U/g of rat) could deplete circulating arginine to levels below the detection limit of the amino acid analyzer (3 $\mu$M) for 6 days, whereas a high dose of native rhArg (7,500 U/rat) only reduced circulating arginine to ~20 $\mu$M within 3 hour of injection, with arginine returning to basal level ~2 days post-injection (Tsui et al., Cancer Cell Int. 2009 Apr 17;9:9). Native arginase is cleared from circulation within minutes (Savoca et al., Cancer Biochem. Biophy's. 7:261-268, 1984). For clinical use, it is essential that the arginase is engineered so as to allow it to persist for many days or weeks in circulation. In the absence of any modification, human arginase has a half-life of only a few minutes in circulation because its size is not big enough to avoid filtration by the kidneys (Georgiou et al., US 8440,184 B2).

**Example 17: Treatment with N-ABD094-rhArg (SEQ ID NO: 50) reduces fat mass, suppresses lipogenesis and improves insulin sensitivity in normal lean mice**

Our data on a mouse model show that a single i.p. injection of 500U N-ABD094-rhArg (SEQ ID NO: 50) to 8-week old normal lean C57BL/6J male mice could maintain plasma arginine concentrations at $\leq 1$ $\mu$M for at least 10 days, demonstrating the long circulatory half-life of N-ABD094-rhArg (SEQ ID NO: 50) (FIG. 13). Thus, we treated normal lean C57BL/6J male mice fed an ordinary chow diet via i.p. injection of 500U N-ABD094-rhArg (SEQ ID NO: 50) at 10-day intervals for 4 weeks. After 4 weeks of treatment, there was over 25% reduction of visceral (perigondal, perirenal and mesenteric fad pad) and subcutaneous (inguinal fat pad) fat mass (FIG. 15A) in comparison with the control mice injected with the vehicle (saline). Histological examination of the visceral white adipose tissue (WAT) showed a prominent reduction in the size of adipocytes (FIG. 15B). We further analyzed lipogenesis and found that in mice treated with N-ABD094-rhArg (SEQ ID NO: 50), mRNA levels of several key lipogenic enzymes, including acetyl-CoA carboxylase 1 (Acc1), fatty acid synthase (Fasn) and stearoyl-CoA desaturase 1 (Scd1), were dramatically suppressed in the visceral WAT (FIG. 16A) and liver (FIG. 16B).

Obesity is highly associated with insulin resistance. We therefore conducted both *in vivo* and *in vitro* study to determine whether N-ABD094-rhArg (SEQ ID NO: 50) treatment will have effects on insulin sensitivity. Results of insulin tolerance test (ITT) showed that mice treated with N-ABD094-rhArg (SEQ ID NO: 50) exhibited a significant increase in insulin sensitivity after 2 weeks of drug treatment (FIG. 17A). We then specifically examined hepatic insulin sensitivity by treating primary hepatocytes prepared from C57BL/6J male mice with medium containing 5U/mL N-ABD094-rhArg (SEQ ID NO: 50), which at this concentration did not affect cell viability (FIG. 17B). At 24-hour after culture in N-ABD094-rhArg (SEQ ID NO: 50) or control (without addition of N-ABD094-rhArg) medium, primary hepatocytes were stimulated with 100 nM insulin for 20 min. Cells were then collected for Western blot analysis to detect phosphorylated protein kinase B (pAkt), which is commonly used as marker of insulin signaling. Results showed that there was a prominent increase in phosphorylation of Akt in the N-ABD094-rhArg (SEQ ID NO: 50) treated group when compared to the control group, demonstrating an enhancement in hepatic insulin signaling (FIG. 17C).

The findings on normal lean mice have demonstrated that N-ABD094-rhArg (SEQ ID NO: 50) has a long circulatory half-life, and can reduce fat mass, suppress lipogenesis, and improve insulin sensitivity both *in vivo* and *in vitro*. These results give support that ABD-rhArg fusion protein can be used for the prevention and treatment of obesity and associated metabolic disorders, such as insulin resistance and hepatic steatosis (fatty liver).

**Example 18: Treatment with N-ABD094-rhArg (SEQ ID NO: 50) effectively prevents HFD-induced obesity**

Treatment with recombinant human arginase [N-ABD094-rhArg (SEQ ID NO: 50)] can significantly reduce fat mass, suppress lipogenesis and improve insulin sensitivity in normal lean mice.

The effect of N-ABD094-rhArg (SEQ ID NO: 50) on adiposity and various metabolic parameters in obese animals was tested. A high-fat diet-induced obesity (DIO) mouse model [Wang and Liao, 2012, Methods Mol. Biol. 821:421-433], which shares many characteristics with human obesity and is widely used for testing prospective anti-obesity agents [Vickers et al., 2011, Br. J. Pharmacol. 164(4):1248-1262], was employed. Specifically, C57BL/6J, which is the most commonly used mouse strain for polygenic obesity model, was employed in the study. It is well-reported that C57BL/6J mice feeding on a high-fat diet will have prominent weight gain, become obese and develop hyperinsulinemia, hyperglycemia, impaired glucose tolerance and insulin resistance [Gallou-Kabani et al., 2007, Obesity 15(8):1996-2005].

To determine the efficacy of N-ABD094-rhArg (SEQ ID NO: 50) in preventing mice from diet-induced obesity and associated metabolic disorders, C57BL/6J male mice were fed a high-fat diet containing 60 kcal% fat (HFD; *Research Diets,* D12492) from 8 week old for 12 weeks, and concurrently received i.p. injection of 200-300U N-ABD094-rhArg (SEQ ID NO: 50) or saline as vehicle control at 7-day intervals. C57BL/6J mice fed a matched low fat diet containing 10 kcal% fat (LFD; *Research Diets,* D12450J) and injected with saline served as the lean control of various measurements.

Two cohorts, with n = 5 in each group, had been separately conducted. Mice were housed in group of 5. Similar results were obtained in the two independent cohorts. Data of one cohort of mice were summarized in Examples 18-22.

In comparing with the lean control fed a LFD and treated with vehicle [referred to as LFD(vehicle) group], vehicle-treated male mice fed a HFD [referred to as HFD(vehicle) group] showed a marked gain in bodyweight (FIG. 18A) and size (FIG. 18B), with a significant increase in the white adipose tissue (WAT) mass at main visceral (perigonadal, perirenal and mesenteric) and subcutaneous (inguinal) depots (FIG. 19A). However, mice fed a HFD but concurrently treated with N-ABD094-rhArg (SEQ ID NO: 50) [referred to as HFD(rhArg) group] could be effectively prevented from weight gain and expansion of WAT caused by HFD intake. Histological examination of WAT revealed that N-ABD094-rhArg (SEQ ID NO: 50) treatment prominently suppressed adipocyte hypertrophy (FIG. 19B). Analysis of mRNA expression levels using real-time qRT-PCR in gWAT further showed a dramatic downregulation of several critical adipogenic transcription factors including peroxisome proliferator-activated receptor gamma *(Pparg)* and sterol regulatory element-binding protein 1c *(Srebplc),* and lipogenic enzymes *(Acc1* and *Scd1)* (FIG.19C). These genes were also significantly downregulated in skeletal muscle of HFD-fed mice treated with N-ABD094-rhArg (SEQ ID NO: 50) (FIG.20), suggesting that the anti-lipogenesis effect of N-ABD094-rhArg (SEQ ID NO: 50) is not limited to WAT.

After confirming the anti-obesity effect of N-ABD094-rhArg (SEQ ID NO: 50) on male mice, the effect of N-ABD094-rhArg (SEQ ID NO: 50) on female mice (*n* = 5 in each group) was studied to determine if there was any sex difference. Results showed that concurrent treatment with N-ABD094-rhArg (SEQ ID NO: 50) was similarly effective in protecting female mice fed a HFD starting from 8 week old for 12 weeks from weight gain (FIG. 21A) and excessive accumulation of WAT at main visceral (perigonadal, perirenal and mesenteric) and subcutaneous (inguinal) depots (FIG. 21B) and could improve insulin sensitivity (FIG. 22A) and glucose tolerance (FIG. 22B).

To test the effect of N-ABD094-rhArg (SEQ ID NO: 50) on fat cells, mouse 3T3-L1 preadipocytes were used, which are commonly employed as cell-based model for studying adipogenesis [Green and Meuth, 1974, Cell 3:127-133]. It was found that N-ABD094-rhArg (SEQ ID NO: 50) could effectively inhibit adipogenesis and lipogenesis in 3T3-L1 cells (FIG. 23), and significantly suppressed mRNA levels of several adipogenic transcription factors (FIG. 24A) and lipogenic enzymes (FIG. 24B). Similar inhibitory effect was observed when 3T3-L1 preadipocytes were cultured in bovine arginase, or another arginine-depleting enzyme-arginine deiminase (ADI), or arginine-free medium (FIG. 23). Arginase converts L-arginine into L-ornithine and urea, whereas ADI converts L-arginine into L-citrulline. However, L-ornithine, urea or L-citrulline could not inhibit adipogenesis and lipogenesis in 3T3-L1 cells (FIG. 23).

Moreover, supplementing arginine-free medium with varying concentrations of L-arginine demonstrated the dose-dependent effect of arginine on adipogenesis and lipogenesis (FIG. 25A), with a threshold concentration between 10-40 $\mu$M arginine for activation of critical adipogenic transcription factors including Cebpa and Pparg and lipogenic enzyme Scd1 (FIG. 25B).

Together, these findings demonstrate that the anti-adipogenesis and lipogenesis effect of N-ABD094-rhArg (SEQ ID NO:50) is mediated via arginine deprivation.

Collectively, these findings support that arginine depletion by ABD-rhArg fusion protein or other forms of arginase (e.g., rhArg-PEG, BCA-PEG or BCA-ABD) or arginine-depleting enzymes (e.g., ADI-PEG, ADI-ABD, ADC-PEG or ADC-ABD) can be used as a preventive anti-obesity therapy.

**Example 19: Treatment with N-ABD094-rhArg (SEQ ID NO: 50) prevents HFD-induced insulin resistance, impaired glucose tolerance, and metabolic derangements**

Obesity is commonly associated with insulin resistance, impaired glucose tolerance and metabolic derangements. Indeed, in comparison with the vehicle-treated lean control mice fed a LFD, vehicle-treated mice fed a HFD showed a significant reduction in sensitivity to blood glucose-lowering effect of the injected insulin in the insulin tolerance test (ITT) conducted at 6 week after feeding on a HFD (FIG. 26A), which further worsened at 11 week (Fig. 26B). Concomitant with reduced insulin sensitivity, these mice were significantly less efficient in returning blood glucose to basal levels after being injected with D-glucose in the glucose tolerance test (GTT) by 5 weeks after feeding on a HFD (FIG. 27A), implying the development of impaired glucose tolerance, which further worsened after 10 weeks of HFD consumption (FIG. 27B). Indeed, the fasting (for 5 hours) blood glucose (Fig. 28A) and fasting plasma insulin levels (Fig. 28B) were significantly elevated, and the HOMA-IR score (Fig. 28C), a measurement of insulin resistance, were higher in the vehicle-treated HFD-fed mice in comparison to the lean control mice. Fasting plasma levels of leptin (Fig. 29A), total cholesterol (Fig. 29B), triglycerides (Fig. 29C) and free fatty acids (Fig. 29D) were significantly increased when measured at 12 weeks after feeding on the HFD. Notably, concurrent treatment with N-ABD094-rhArg (SEQ ID NO: 50) could completely prevent the development of insulin resistance, impaired glucose tolerance, hyperglycemia, hyperinsulinemia, hyperleptinemia, hypercholesterolemia, hypertriglyceridemia, and hyper-free fatty acidemia induced by the HFD.

Together, these results demonstrate that N-ABD094-rhArg (SEQ ID NO: 50) can effectively prevent insulin resistance and glucose intolerance, a precursor to developing type 2 diabetes. It may also prevent leptin resistance and other metabolic derangements including dyslipidemia associated with obesity.

Collectively, these results support that arginine depletion by ABD-rhArg fusion protein or other forms of arginase (e.g., rhArg-PEG, BCA-PEG or BCA-ABD) or arginine-depleting enzymes (e.g., ADI-PEG, ADI-ABD, ADC-PEG or ADC-ABD) can be used as a preventive anti-diabetes, anti-dyslipidemia, anti-atherosclerosis and antimetabolic derangement therapy.

**Example 20: Treatment with N-ABD094-rhArg (SEQ ID NO: 50) prevents HFD-induced steatosis**

Obesity is also associated with excessive accumulation of lipids in the kidney (renal steatosis), pancreas (pancreatic steatosis) and heart (cardiac steatosis), causing lipotoxicity and dysfunction of these organs. Indeed, it was found that in the vehicle-treated mice fed a HFD, there was a significant increase in the weight of the kidney (FIG. 33A), pancreas (FIG. 33B) and heart (FIG. 33C). Concurrent treatment with N-ABD094-rhArg (SEQ ID NO: 50) could effectively prevent weight gain in the these organs, which suggests that N-ABD094-rhArg (SEQ ID NO: 50) can prevent excessive accumulation of lipids and lipotoxicity in these organs.

Collectively, these results support that ABD-rhArg fusion protein can effectively prevent steatosis and arginine depletion by N-ABD094-rhArg (SEQ ID NO: 50) or other forms of arginase (e.g., rhArg-PEG, BCA-PEG or BCA-ABD) or arginine-depleting enzymes (e.g., ADI-PEG, ADI-ABD, ADC-PEG or ADC-ABD) can be used as a preventive anti-steatosis therapy.

**Example 21: Treatment with N-ABD094-rhArg (SEQ ID NO: 50) prevents HFD-induced chronic inflammation**

Excessive expansion of WAT will increase the production of a number of adipokines, which trigger chronic low-grade inflammation that contributes to the development of insulin resistance. Monocyte chemoattractant protein-1 (Mcp1) is one of the key proinflammatory adipokines secreted by WAT that regulates macrophages infiltration into the WAT, insulin resistance and hepatic steatosis in obesity. Indeed, we found that the mRNA level of *Mcp1* was significantly upregulated in perigonadal WAT of vehicle-treated mice fed a HFD (FIG. 34A). Concomitant with this, serum levels of circulatory Mcp1, measured by enzyme-linked immunosorbent assay (ELISA), were significantly elevated in comparison to the lean control (FIG. 34B). Other proinflammatory adipokines, which include interleukin 1 beta (*Il1b*) and tumor necrosis factor alpha (*Tnfa*), also showed increased mRNA expressions in perigondal WAT (FIG. 34A). Notably, concurrent treatment with N-ABD094-rhArg (SEQ ID NO: 50) could suppress the upregulation of these adipokines in perigonadal WAT and suppress an elevation in the serum levels of Mcp1.

These results demonstrate that N-ABD094-rhArg (SEQ ID NO: 50) can prevent chronic low-grade inflammation associated with obesity and support the potential of arginine depletion by ABD-rhArg fusion protein or other forms of arginase (e.g., rhArg-PEG, BCA-PEG or BCA-ABD) or arginine-depleting enzyme (e.g., ADI-PEG, ADI-ABD, ADC-PEG or ADC-ABD) as a preventive anti-inflammation therapy.

**Example 22: Treatment with N-ABD094-rhArg (SEQ ID NO: 50) prevents HFD-induced whitening and deregulation of fatty acid oxidation in brown adipose tissue and enhances non-shivering thermogenesis**

Brown adipose tissue (BAT) serves to dissipate oxidative fuels into heat (non-shivering thermogenesis). Recent studies show that it has great capacity in energy expenditure to counteract obesity. On the contrary, whitening of BAT is associated with obesity and impairs BAT function. Indeed, we found that in vehicle-treated mice fed a HFD, the interscapular BAT was prominently enlarged (FIG. 35A), with the weight doubled that of the lean control mice (FIG. 35B). Histological examination revealed extensive accumulation of large unilobular cells interspersed between multilobular brown adipocytes (FIG. 35C), suggesting a conversion of brown adipocytes to white-like cells. However, in HFD-fed mice concurrently treated with N-ABD094-rhArg (SEQ ID NO: 50), the weight, size, and histological appearance of the BAT were similar to that of the lean control mice. We further found that treatment with N-ABD094-rhArg (SEQ ID NO: 50) significantly upregulated the BAT-specific gene Uncoupling protein 1 (*Ucp1*) that is crucial for thermogenesis, and also upregulated peroxisome proliferator-activated receptor gamma coactivator 1-alpha (*Pgc1α*), the key transcriptional regulator of *Ucp1* in brown adipocytes, while suppressing HFD-induced downregulation of Acyl-CoA oxidase *1* (*Acox1*), a major gene involved in fatty acid oxidation (FIG. 36). It was found that N-ABD094-rhArg (SEQ ID NO: 50) treatment could block the downregulation of *Acox1,* indicating the potential of N-ABD094-rhArg (SEQ ID NO: 50) in normalizing fatty acid oxidation.

Together, these results demonstrate that N-ABD094-rhArg (SEQ ID NO: 50) treatment can effectively prevent brown fat whitening and deregulation of fatty acid oxidation in BAT associated with obesity, and enhances non-shivering thermogenesis to combat against the adiposity effect of HFD.

These findings support the potential of arginine depletion by ABD-rhArg fusion protein or other forms of arginase (e.g., ABD094-rhArg, BCA-PEG or BCA-ABD) or arginine-depleting enzyme (e.g., ADI-PEG, ADI-ABD, ADC-PEG or ADC-ABD) as a preventive anti-obesity therapy.

**Example 23: Treatment with N-ABD094-rhArg (SEQ ID NO: 50) effectively reduces HFD-induced obesity**

After confirming that concurrent treatment with N-ABD094-rhArg (SEQ ID NO: 50) can effectively prevent HFD-induced obesity and associated complications and comorbidities including insulin resistance, glucose intolerance, hyperglycemia, dyslipidemia, hepatic steatosis, chronic inflammation, and whitening of BAT, next, we studied the efficacy of N-ABD094-rhArg (SEQ ID NO: 50) in reducing obesity and reversing these disorders in mice with pre-existing diet-induced obesity (DIO). C57BL/6J male mice were fed a HFD from 5 week old for 12 weeks to induce obesity and associated complications and comorbidities. They were then stratified according to the bodyweight into 2 groups and treated with either 600-700U N-ABD094-rhArg (SEQ ID NO: 50) or saline as vehicle control at 7-day intervals for 12 weeks while continued to be fed the HFD. One group of mice were fed a matched low fat diet (LFD) and injected with saline throughout the study to serve as the lean control of various measurements. Two cohorts, with n=5 in each group, have been separately conducted. Mice were housed in groups of 5. Similar results were obtained in the two independent cohorts. Data of one cohort of mice were presented in Examples 23-28.

Twelve weeks of HFD feeding could increase the bodyweight of male mice to over 50g, which was markedly heavier than lean control male mice fed a LFD (FIG. 37A and 37B). Following treatment with N-ABD094-rhArg (SEQ ID NO: 50), the bodyweight of HFD-induced obese mice gradually decreased. By 5 weeks, their bodyweight had reduced by over 30% and was similar to the lean control mice. The bodyweight was maintained at this level till the completion of 12 weeks of treatment. The weight of the fat pad at the main visceral depots (perigonadal, perirenal and mesenteric WAT) and subcutaneous depot (inguinal WAT) in DIO mice treated with N-ABD094-rhArg (SEQ ID NO: 50) was significantly reduced and was highly similar to lean control mice (FIG. 38A). Histological examination of WAT revealed a prominent diminution of the adipocytes in DIO mice treated with N-ABD094-rhArg (SEQ ID NO: 50) (FIG. 38B).

Together with results in Example 18, these findings support that ABD-rhArg fusion protein or other forms of arginase (e.g., rhArg-PEG, BCA-PEG or BCA-ABD) or arginine-depleting enzyme (e.g., ADI-PEG, ADI-ABD, ADC-PEG or ADC-ABD) can be used for therapeutic treatment of obesity.

**Example 24: Treatment with N-ABD094-rhArg (SEQ ID NO: 50) reverses HFD-induced insulin resistance and impaired glucose tolerance, and corrects metabolic derangement**

Prior to treatment, mice feeding on a HFD for 12 weeks had developed peripheral insulin resistance (FIG. 39) and impaired glucose tolerance (FIG. 40), which are characteristics of prediabetes, in comparison to the lean control mice. However, by 4 weeks after treatment with N-ABD094-rhArg (SEQ ID NO: 50), DIO mice showed a marked increase in insulin sensitivity that became comparable to the lean control mice. Normalization of sensitivity to insulin in mice receiving N-ABD094-rhArg (SEQ ID NO: 50), while continuously being fed a HFD, could be sustained till the end of 12 weeks of drug treatment. Furthermore, mice treated with N-ABD094-rhArg (SEQ ID NO: 50) exhibited a significant improvement in glucose tolerance by 3 weeks of treatment, and was fully restored to a level similar to the lean control mice by 7 weeks of treatment. In line with these improvements, the fasting blood glucose (Fig. 41A) and plasma insulin (Fig. 41B) of DIO mice treated with N-ABD094-rhArg (SEQ ID NO: 50) had restored to levels similar to the lean control mice by 4 weeks of rhArg treatment and maintained within the normal range until the end of treatment. This resulted in a dramatic decrease in HOMA-IR score from 35.1 in mice prior to treatment to 1.2 in mice 4 weeks after treatment (Fig. 41C), which implicated a reversion of insulin resistance. Fasting plasma leptin (Fig. 42A) and total cholesterol levels (Fig. 42B) were also markedly reduced to normal levels.

Collectively, our findings demonstrate the great efficacy of N-ABD094-rhArg (SEQ ID NO: 50) in reversing insulin resistance, impaired glucose tolerance, hyperglycemia, hyperinsulinemia, hyperleptinemia and hypercholesterolemia within 4 weeks of treatment.

Together, these findings support that arginine depletion by ABD-rhArg fusion protein or other forms of arginase (e.g., rhArg-PEG, BCA-PEG or BCA-ABD) or arginine-depleting enzyme (e.g., ADI-PEG, ADI-ABD, ADC-PEG or ADC-ABD) can be used as an insulin-sensitizing agent, and for therapeutic treatment of prediabetes and type 2 diabetes characterized by insulin resistance, glucose intolerance and hyperglycemia, and other disorders associated with insulin resistance. It can also be used for therapeutic treatment of hyperleptinemia and hypercholesterolemia.

**Example 25: Treatment with N-ABD094-rhArg (SEQ ID NO: 50) reverses HFD-induced steatosis**

Twelve weeks of HFD feeding induced hepatic steatosis in male mice (FIG. 30 and 31). After extending the feeding of HFD for 12 weeks, the liver was further enlarged and appeared pale in colour (FIG. 43A). The liver weight was more than double of that of the lean control mice (FIG. 43B). Massive lipid accumulation had extended to the entire liver (FIG. 44A), with a corresponding increase in triglyceride content (FIG. 44B). The expression levels of critical adipogenic transcription factors *Pparg* and *Srebp1c* were downregulated to levels similar to the lean control, while expression levels of lipogenic enzymes *Acc1* was further reduced, and *Scd1* was drastically suppressed (FIG. 44C). In comparing with mice that were fed a HFD for 12 weeks (Example 20, FIG. 32), there was a further elevation of serum alanine aminotransferase (ALT) levels in mice fed a HFD for 24 weeks, and serum aspartate aminotransferase (AST) levels were also significantly increased (FIG. 45), demonstrating a further progression of liver damage. However, in DIO mice treated with N-ABD094-rhArg (SEQ ID NO: 50), the liver showed a marked diminution in size to such an extent that the weight was similar to the lean control (FIG. 43B). Moreover, the liver restored to a reddish colour (FIG. 43A), which was well correlated with a dramatic clearance of lipid droplets (FIG. 44A), with the triglyceride concentrations reduced to a level similar to the lean control (FIG. 44B). Notably, plasma ALT and AST concentrations were comparable to the lean control, which suggests that treatment with N-ABD094-rhArg (SEQ ID NO: 50) can prevent further progression and correct existing liver damage. Collectively, our findings reveal the marked effect of N-ABD094-rhArg (SEQ ID NO: 50) on reversal of hepatic steatosis.

Other than the liver, obesity is also associated with excessive accumulation of lipids in the kidney (renal steatosis), pancreas (pancreatic steatosis) and heart (cardiac steatosis), causing lipotoxicity and dysfunction of these vital organs. Indeed, there was a significant increase in the weight of kidney (FIG. 46A), pancreas (FIG. 47A) and heart (FIG. 48A) in DIO mice treated with vehicle. Histological examination revealed accumulation of vacuoles that resemble lipid droplets in the renal tubules (FIG. 46C). Oil red O staining of kidney sections showed that there was ectopic accumulation of lipids in the glomerulus (FIG. 46D). There was extensive intralobular and interlobular accumulation of patches of WAT in the pancreas (FIG. 47B), and islands of white adipocytes were found among cardiac muscle fibres (FIG. 48B). Triglyceride concentrations were significantly increased in the kidney (FIG. 46B), pancreas (FIG. 47C), heart (FIG. 48C). Moreover, a significant elevation in triglyceride concentrations was also found in skeletal muscle (FIG. 49). These findings indicate the development of steatosis in these organs of vehicle-treated DIO mice. However, treatment with N-ABD094-rhArg (SEQ ID NO: 50) could significantly reduce the weight of the kidney (FIG. 46A), pancreas (FIG. 47A) and heart (FIG. 48A) of DIO mice to a level similar to the lean control. Histological examination confirmed a marked reduction of vacuoles in the renal tubules (FIG. 46C) and lipids in glomeruli of kidneys identified by Oil red O staining (FIG. 46D). There was a prominent reduction of WAT within the pancreas (FIG. 47B) and heart (FIG. 48B). The triglyceride concentrations in the kidney (FIG. 46B), pancreas (FIG. 47C), heart (FIG. 48C) and skeletal muscle (FIG. 49) of DIO mice treated with N-ABD094-rhArg (SEQ ID NO: 50) were restored a level similar to the lean control. These findings demonstrate that N-ABD-rhArg (SEQ ID NO: 50) can effectively reverse steatosis in the kidney, pancreas and heart, and possibly other organs as well.

Collectively, these findings support that ABD-rhArg fusion proetin or other forms of arginase (e.g., rhArg-PEG, BCA-PEG or BCA-ABD) or arginine-depleting enzyme (e.g., ADI-PEG, ADI-ABD, ADC-PEG or ADC-ABD) can be used for therapeutic treatment of steatosis.

**Example 27: Treatment with N-ABD094-rhArg (SEQ ID NO: 50) reverses whitening of brown fat**

After prolonged feeding on a HFD, there was a further increase in the size (FIG. 54A) and weight of the interscapular BAT (FIG. 54B) in vehicle-treated DIO mice in comparison to the lean control mice, which correlated with the presence of massive amount of enlarged white-like unilocular cells (FIG. 54C). In contrast, treatment of DIO mice with N-ABD094-rhArg (SEQ ID NO: 50) could dramatically restore the interscapular BAT to a size and weight similar to the lean control mice, with almost complete remission of white-like unilocular cells. Collectively, these findings demonstrate the great capability of N-ABD094-rhArg (SEQ ID NO: 50) in reversing whitening of the brown fat.

Collectively, these results support that ABD-rhArg fusion protein or other forms of arginase (e.g., rhArg-PEG, BCA-PEG or BCA-ABD) or arginine-depleting enzyme (e.g., ADI-PEG, ADI-ABD, ADC-PEG or ADC-ABD) can be used for therapeutic treatment of obesity via reversing whitening of brown fat.

**Example 28: Treatment with N-ABD094-rhArg (SEQ ID NO: 50) lowers blood pressure**

Obesity is associated with cardiovascular disease and hypertension. DIO mice treated with 600U N-ABD094-rhArg (SEQ ID NO: 50) at 7-day intervals for 5 weeks were subjected to measurement of blood pressure by tail cuff method using the CODA Non-invasive Blood Pressure System (Kents Scientific Corporation). It was found that the systolic and diastolic blood pressure (FIG. 55A), and heart rate (FIG. 55B) of DIO mice treated with N-ABD094-rhArg (SEQ ID NO: 50) were significantly lower than DIO mice treated with vehicle, and were highly comparable to the lean control mice. Example 25 shows that N-ABD094-rhArg (SEQ ID NO: 50) can effectively reverse renal and cardiac steatosis, which may have beneficial effects on lowering blood pressure.

Collectively, these findings support that ABD-rhArg fusion protein or other forms of arginase (e.g., rhArg-PEG, BCA-PEG or BCA-ABD) or arginine-depleting enzyme (e.g., ADI-PEG, ADI-ABD, ADC-PEG or ADC-ABD) can be used for therapeutic treatment of hypertension.

**Example 29: N-ABD0094-rhArg (SEQ ID NO: 50) does not induce neutralizing antibody and remains effective for prolonged use**

Generation of neutralizing antibodies may affect the pharmacodynamics, pharmacokinetic, stability and efficacy of a drug. To determine the immunogenic response, 8 week old C57BL/6J male mice fed a LFD received i.p. injection of 500U of N-ABD094-rhArg (SEQ ID NO: 50), or saline as vehicle control, at 7-day intervals for 8 months. It was found that despite the presence of anti-drug antibodies against N-ABD094-rhArg (SEQ ID NO: 50) (FIG. 56A), these antibodies did not have neutralizing effects on arginase activity (FIG. 56B), which is in line with the findings that plasma arginine concentrations could be maintained at levels below the detection limit of the Biochrom 30 Amino Acid Analyzer (3 $\mu$M) throughout the study period.

To assess the safety of long-term use, serum and urine samples from LFD-fed mice treated with N-ABD094-rhArg (SEQ ID NO: 50) for 8 months were collected for analysis of ALT and AST, which are commonly used biomarkers of liver damage, and serum creatinine and urine albumin/creatinine ratio for assessing kidney functions. It was found that LFD-fed mice treated with the drug did not show any significant differences from LFD-fed mice treated with vehicle (FIG. 57A and 57B) in all these parameters. Furthermore, in examining functional responses and vascular reactivity of isolated arteries using wire myography, there were no significant differences between LFD-fed mice treated with N-ABD094-rhArg (SEQ ID NO: 50) and LFD-fed mice treated with vehicle in vascular smooth muscle contraction evoked by phenylephrine (FIG. 58A), acetylcholine-induced endothelium-dependent relaxation (FIG. 58B) and endothelium-independent relaxation in response to the nitric oxide donor sodium nitroprusside (FIG. 58C).

In a separate study in which diet-induced obese C57BL/6J male mice were treated with 600U N-ABD094-rhArg (SEQ ID NO: 50) for 20 weeks while continued to be fed a HFD, the bodyweight was reduced to a level similar to the lean control mice by 5 weeks and continued to be maintained at this level without rebound. Regular monitoring of plasma arginine concentrations showed that arginine remained at levels below the detection limit of the Biochrom 30 Amino Acid Analyzer (3 $\mu$M). These results support that N-ABD094-rhArg (SEQ ID NO: 50) remained effective throughout the treatment period. Together, these findings support that N-ABD094-rhArg (SEQ ID NO: 50) is safe and effective for long term use.

**Example 30: PEGylation protocol for recombinant human arginase (rhArg)**

Recombinant human arginase (His-rhArg SEQ ID NO: 101) was obtained by purification using affinity chromatography. Prior to PEGylation, the purified His-rhArg (SEQ ID NO: 101) was diafiltered against 20 mM sodium phosphate (pH 8.0), and the protein concentration was adjusted to 2 mg/mL. PEGylation reagents were made up in 20 mM sodium phosphate (pH 8.0). A molar ratio of PEG reagent to rhArg at 20:1 was used. The PEG reagent was added to the His-rhArg (SEQ ID NO: 101) and then incubated for 4 hours at room temperature with stirring. The reaction mixture was then diafiltered against 20 mM sodium phosphate (pH 7.4) using tangential flow filtration (TFF). Excess PEG was removed in the flow through using affinity chromatography and the PEGylated His-rhArg (SEQ ID NO: 101) was recovered by eluting the protein with 0.5 M imidazole. Any underivatized His-rhArg (SEQ ID NO: 101) was separated by another round of affinity chromatography. The reaction mixture was then analyzed by SDS-PAGE using 12% polyacrylamide gel. The gel was stained with Coomassie blue for molecular weight identification, or 0.1 M iodine solution for detection of free PEG.

For example, in a preparation, 150 mg of the PEGylated His-rhArg (SEQ ID NO: 101) was obtained, with specific activity of 245 U/mg and concentration of 7.5 mg/mL. FIG. 11 and **Table 5** shows an example of the production of PEG-rhArg and its analysis.

**Table 5.** Production of PEGylated His-rhArg (SEQ ID NO: 101) and its analysis.

| | Volume (mL) | Concentration (mg/mL) | Total (mg) | Specific Act. (U/mg) |
|---|---|---|---|---|
| PEGylated His-rhArg batch 3 | 11 | 3.5 | 38.5 | 189 |
| PEGylated His-rhArg batch 3 | 35 | 5.3 | 185.5 | 213 |

**Example 31: Treatment with PEGylated His-rhArg [SEQ ID NO: 101] reduces obesity and improves insulin sensitivity**

Production of PEGylated His-rhArg (SEQ ID NO: 101) is described in Example 30. C57BL/6J male mice with pre-existing diet-induced obesity (DIO) received i.p. injection of 300U PEGylated His-rhArg [SEQ ID NO: 101], or saline as vehicle control, at 7-day intervals for 5 weeks, while continuously being fed a HFD. The bodyweight of the mice treated with PEGylated His-rhArg (SEQ ID NO: 101) gradually reduced to 30g, which was similar to the vehicle-treated lean control mice fed a chow diet (FIG. 59). The weight of the fat pad at the main visceral depots (perigonadal, perirenal and mesenteric WAT) and subcutaneous depot (inguinal WAT) was significantly reduced and was similar to the lean control mice at the end of the study (FIG. 60). The weight of the liver, kidney, pancreas and heart was significantly lower than DIO mice treated with vehicle and was comparable to the lean control mice (FIG. 60). Regular monitoring of plasma arginine concentrations confirmed that arginine remained at levels below the detection limit of the Biochrom 30 Amino Acid Analyzer (3 $\mu$M) throughout the study.

Two weeks after treatment of PEGylated His-rhArg [SEQ ID NO: 101], DIO mice already showed a lower fasting blood glucose levels (FIG. 61A) and a significant increase in insulin sensitivity in comparison to DIO mice treated with vehicle (FIG. 61B).

Together, these findings support that PEGylated His-rhArg [SEQ ID NO: 101] has anti-adiposity and insulin-sensitizing effects similar to N-ABD094-rhArg [SEQ ID NO: 50]. Arginine deprivation by PEGylated His-rhArg (SEQ ID NO: 101) has therapeutic effects to treat obesity and diseases associated with insulin resistance.

## Example 32: Metal ion substitution

Wild type arginase typically includes a $Mn^{2+}$ metal ion (D'Antonio & Christianson, 2011, Biochemistry, 50:8018-27). It is found that metal ion substitution is a method to prepare a much more potent form of ABD094-rhArg (low $K_m$ value and high $k_{cat}/K_m$ value). Purified N-ABD094-rhArg (SEQ ID NO: 50) in 20 mM Tris-HCl buffer (pH 7.4) was mixed with 20 mM of different divalent metal ions ($Mn^{2+}$, $Ni^{2+}$ or $Co^{2+}$), and was incubated at 52 - 55 °C for 15 - 60 min and then buffer exchanged with PBS buffer (pH 7.4).

Samples were subjected to enzyme activity and enzyme kinetic studies to determine the specific activity and catalytic efficiency ($k_{cat}/K_m$). Samples were applied to inductively coupled plasma atomic emission spectroscopy (ICP-OES, Agilent 700 Series ICP optical emission spectrometer) for quantitative analysis of metal content.

Each protein sample (100 - 200 $\mu$L) was added to 10 ml of 1% w/v $HNO_3$ and then different elements and wavelengths were selected for ICP-OES analysis. The metal to protein ratio was determined by dividing metal concentration by protein concentration. Pierce™ BCA protein assay was used to evaluate the protein concentration following the manufacturer's instruction (Thermo Fisher Scientific).

Adding $Co^{2+}$ or $Ni^{2+}$ metal ion to N-ABD094-rhArg (SEQ ID NO: 50) resulted in good $Mn^{2+}$ removal and incorporation of $Co^{2+}$ or $Ni^{2+}$.

It was found that the cobalt-substituted enzyme (N-ABD094-rhArg-$Co^{2+}$) showed much smaller $K_m$ (0.27 - 0.35 mM), higher $k_{cat}$ (250 - 316 $s^{-1}$) and higher catalytic efficiency $k_{cat}/K_m$ (905 - 917 $s^{-1}$ $mM^{-1}$). For the original enzyme (N-ABD094-rhArg-$Mn^{2+}$), it has $K_m$ of 1.37 - 1.89 mM, $k_{cat}$ of 82 - 98 $s^{-1}$ and $k_{cat}/K_m$ of 44 - 72 $s^{-1}$ $mM^{-1}$. The metal substitution method successfully replaced $Mn^{2+}$ by $Co^{2+}$, leading to a saturated $Co^{2+}$ to protein ratio of approximately 2 - 4 to 1. The data suggest that a more potent form of N-ABD094-rhArg (SEQ ID NO: 50) or other fusion proteins described herein can be manufactured by substitution with other divalent metal ions (e.g., $Ni^{2+}$ or $Co^{2+}$).

## Example 33: Treatment with N-ABD094-rhArg-$Co^{2+}$ [SEQ ID NO: 50 (cobalt substituted)] reduces obesity

Production of N-ABD094-rhArg-$Co^{2+}$ [SEQ ID NO: 50 (cobalt substituted)] is described in Example 32. C57BL/6J male mice with pre-existing diet-induced obesity (DIO) received i.p. injection of 25-100U N-ABD094-rhArg-$Co^{2+}$ [SEQ ID NO: 50 (cobalt substituted)] for 5 weeks, while continuously being fed a HFD. The bodyweight of the mice gradually reduced from 55g to 30g within 5 weeks of treatment (FIG. 62), similar to the effect of 600-700U N-ABD094-rhArg (FIG. 37). This result supports that substitution of $Mn^{2+}$ with $Co^{2+}$ markedly increases the potency of the ABD-rhArg fusion protein in reducing the bodyweight.

## Example 34: Mammalian cell culture and proliferation assay

Cancer cell lines (e.g. MKN45, AGS, BGC823, HCT-15 and HCT-116) were maintained in RPMI Medium with 10% FBS and 100 units/mL penicillin/streptomycin by standard protocol.

For cell proliferation assay, cancer cells ($5 \times 10^3$) in a volume of 100 $\mu$L of growth medium were seeded to each well of a 96-well plate and incubated for 24 h. The culture medium was replenished by arginine free medium (AFM) supplemented with different concentrations of arginine, fresh medium containing various concentrations of different arginine depleting enzymes as well as using these enzymes in combination with citrulline. The plate was incubated for an additional 1-3 days at 37°C in an incubator containing a humidified atmosphere of 95% air and 5% $CO_2$.

Quantitative cell proliferation assays were performed using MTT (3-[4,5-dimethylthiazol-2-yl]-2,5-diphenyl tetrazolium bromide) assays as widely described elsewhere. The amount of arginine depleting enzyme required to kill 50% of the cells in a culture was defined as $IC_{50}$.

## Example 35: Inhibition of the proliferation of various human cancer cell lines by various arginine-depleting enzymes (ADI, BCA and ADC)

The cytotoxicities of arginine deiminase (ADI), bacterial arginase (BCA) and arginine decarboxylase (ADC) were determined in a comparative study in five tumor cell lines: A375, HeLa, BxPC-3, PANC-1, and HCT116.

As both ADI- and BCA-catalyzed reactions are steps of the urea cycle, the antitumor effects of ADI and BCA are both related to cellular ASS level. In contrast, the antitumor effect of ADC may be relatively less affected by the presence of ASS, as neither catalytic products of ADC are intermediates of the urea cycle. Our results have demonstrated that ADI, BCA and ADC all exert inhibitory effects in the cell lines studied. ADI is effective against A375, HCT116, BxPC-3, PANC-1, but not HeLa cells, while both BCA and ADC are effective against all these five cancer cell lines tested **(Table 6).** ADC is the least potent arginine-depleting enzyme but is advantageous over ADI and BCA in terms of efficacy, especially on ASS-positive cancer cells **(Table 6).**

Sensitivity of tumor cells to ADI displays a clear relationship to the cellular ASS level **(Table 6).** Taken together, ADC and BCA can inhibit the proliferation of human cancer cell lines in an ASS-independent manner compared to ADI.

**Table 6.** IC$_{50}$ and maximum cytotoxicity of ADI, BCA and ADC on five human cancer cell lines. Cells were treated for 72 h before MTT analysis. Data are the mean of three experiments performed in triplicate. IC$_{50}$ value is defined as the amount of enzyme needed to achieve 50% inhibition of cell viability. Maximum cytotoxicity is expressed as the maximum percentage of nonviable cells achieved by each enzyme. ADI-resistant cells are defined as cells of which more than 50% survive under the maximum dose of ADI tested.

**Table 6.**

| Cancer Type | Cell Line | ASS protein level | IC$_{50}$ (μg/mL) | | | Maximum cytotoxicity (%) | | |
|---|---|---|---|---|---|---|---|---|
| | | | ADI | BCA | ADC | ADI | BCA | ADC |
| Melanom a | A375 | Undetectable , uninducible | 0.03 | 0.12 | 5.61 | 93.9 2 | 86.9 2 | 84.7 6 |
| Colorectal | HCT11 6 | Low | 0.10 | 0.22 | 12.2 3 | 72.1 8 | 81.0 5 | 84.3 5 |
| Pancreatic | BxPC-3 | Medium | 0.09 | 0.44 | 16.85 | 57.53 | 84.82 | 93.16 |
| | PANC-1 | Low/mediu m, inducible | 0.04 | 1.81 | 4.65 | 55.6 8 | 63.0 1 | 82.9 8 |
| Cervical | HeLa | Low/high, inducible | Resistan t | 1.25 | 3.82 | <50 | 61.1 4 | 86.4 9 |

**Example 36: Cytotoxicity of BHA (SEQ ID NO: 75) on gastric cancer cells**

The *in vitro* efficacy of the albumin-binding arginase BHA (SEQ ID NO: 75) was tested by incubating the MKN45 gastric cancer cell line with various concentrations of BHA (SEQ ID NO: 75) for 72 h and determining the viability of MKN45 by MTT assay. As shown in FIG.63, the growth of MKN45 cancer cells was inhibited by BHA (SEQ ID NO: 75) in a dose-dependent manner. This further confirmed the cytotoxicity of the arginine-depleting fusion protein in culture condition where the medium was supplemented with fetal bovine serum (FBS) and, including albumin as well as other different proteins.

**Example 37: Evaluation of apoptosis by annexin V-FITC and PI double staining**

To evaluate the induction of apoptosis by the BCA arginase (SEQ ID NO: 71), 3 x 10$^5$ cells were seeded in 6-well plate one day prior to different treatment conditions. The medium was withdrawn and replenished with complete medium supplemented with different concentrations of BCA (SEQ ID NO: 71). After exposed to BCA for 24 h, 48 h and 72 h, both floating cells and adherent cells were collected. Briefly, the floating cells in culture medium were collected by centrifugation at 100 x g for 3 min at room temperature while the adherent cells were washed with PBS and lifted by incubating with 0.25% trypsin solution for 3 min at 37 °C. After addition of complete medium, the cells were collected by centrifugation. The cells were washed with PBS and resuspended in 0.1 mL binding buffer (0.01 M HEPES, pH 7.4; 0.14 M NaCl; 2.5 mM CaCl$_2$) containing propidium iodide (PI) and annexin V-FITC. The cells were stained in dark at room temperature for 15 min and 0.4 mL of binding buffer was added to each tube prior to flow cytometry analysis using FACSAria (BD biosciences). The cytometer was set to acquire the data from 10,000 cells for each sample and the data were analyzed by the FACSAria software.

According to the flow cytometry results, apoptosis was induced in HCT116 human colon cancer cells in a time-dependent manner upon BCA (SEQ ID NO: 71) treatment (50 μg/mL of BCA). At 24, 48, and 72 h post-BCA-treatment, ~16%, 21%, and 25% of HCT116 cancer cells have undergone apoptosis, respectively.

**Example 38: Cell viability assay and combination drug effects**

Breast cancer cell lines (e.g. human MCF-7 and MDA-MB-231 cells) were used in these studies. Cancer cells (5 x 10$^3$ cells) were seeded in 96-well plates (TPP) with 100 μL complete medium. The plates were incubated in incubator overnight and the cells were allowed to adhere to the plates. The His-rhArg arginase (SEQ ID NO: 101) (from 2 U/mL to 0.0032 U/mL) and autophagy inhibitor chloroquine (CQ, from 100 μM to 0.16 μM) were then added to the 96-well plate. For the drug combination group, His-rhArg (SEQ ID NO: 101) (from 2 U/mL to 0.0032 U/mL) were added with 20 μM CQ (non-constant ratio method) to the wells. Each dosage was triplicated in 3 wells to obtain the average change of cell viability. After 68 h incubation, 10 μL of 5 mg/mL MTT [3-(4,5-dimethylthiazol-2-yl)-2,5-diphenyltetrazolium bromide] solution was added. The plates were put back into the incubator and further incubated for 4 h. To dissolve the insoluble compound, formazan, formed by MTT, 100 μl of 10% SDS in 0.01 M HCl was added. The plates were incubated at 37°C overnight. The color change was measured by spectrophotometer at 570 nm. Control set up (without any drug) was used as base to calculate percentage of cell viability which was used to plot the dose response curve by the software Prism 6.0 (GraphPad) to find the $IC_{50}$ values. The interaction or combination effects between His-rhArg (SEQ ID NO: 101) and CQ can be calculated by combination index (CI). CalcuSyn software (Biosoft) was used for calculating the CI values.

The MCF-7 cancer cells were sensitive to arginine starvation by arginase for 72 h **(Table 7).** CQ also showed its cytotoxic effects on MCF-7 **(Table 8).** For the drug combination group, there were synergistic effects (CI < 1) between His-rhArg (SEQ ID NO: 101) and CQ **(Table 9). Table 10** shows the $IC_{50}$ of His-rhArg (SEQ ID NO: 101), CQ, and rhArg plus CQ against the MCF-7 cancer cells.

**Table 7.** Percentage of cell viability of the MCF-7 cancer cells after treating with His-rhArg (SEQ ID NO: 101)

| His-rhArg Concentration (U/mL) | Mean of % Cell Viability (n = 6) | SEM |
|---|---|---|
| 2 | 45.29% | 0.80% |
| 0.4 | 48.75% | 0.99% |
| 0.08 | 70.68% | 1.69% |
| 0.016 | 88.99% | 1.64% |
| 0.0032 | 80.88% | 2.40% |
| 0 | 100.00% | 1.44% |

**Table 8.** Percentage of cell viability of the MCF-7 cancer cells after treating with CQ

| CQ Concentration ($\mu$M) | Mean of % Cell Viability (n = 6) | SEM |
|---|---|---|
| 100 | 40.73% | 2.33% |
| 20 | 72.41% | 1.76% |
| 4 | 83.34% | 0.69% |
| 0.8 | 85.88% | 3.03% |
| 0.16 | 96.33% | 0.91% |
| 0 | 100.00% | 0.87% |

**Table 9.** Combination index (CI) and the percentage of cell viability of combination of His-rhArg (SEQ ID NO: 101) and CQ against the MCF-7 cells

| His-rhArg Concentration (U/mL) | CQ Concentration ($\mu$M) | Mean of % Cell Viability (n = 6) | SEM | CI |
|---|---|---|---|---|
| 2 | 20 | 35.98% | 0.37% | 0.463 |
| 0.4 | 20 | 37.34% | 0.51% | 0.190 |
| 0.08 | 20 | 45.90% | 0.70% | 0.253 |
| 0.016 | 20 | 59.78% | 1.32% | 0.669 |
| 0.0032 | 20 | 69.27% | 0.98% | 1.440 |
| 0 | 0 | 100.00% | 1.44% | |

**Table 10.** $IC_{50}$ of His-rhArg (SEQ ID NO: 101), CQ and combining His-rhArg (SEQ ID NO: 101) and CQ against the MCF-7 cancer cells

| MCF-7 | $IC_{50}$ | SEM |
|---|---|---|
| His-rhArg | 0.7869 U/mL | 0.0760 U/mL |
| CQ | 67.16 $\mu$M | 10.72 $\mu$M |
| His-rhArg + CQ | 0.07617 U/mL | 0.00576 U/mL |

The experimental data showed that the MDA-MB-231 breast cancer cells were sensitive to arginine starvation by His-rhArg (SEQ ID NO: 101) and CQ **(Table 11** and **Table 12).** Combination of His-rhArg (SEQ ID NO: 101) and CQ also showed synergistic cytotoxicity on the MDA-MB-231 cells. The CI value was less than 1 **(Table 13). Table 14** shows the $IC_{50}$ of His-rhArg (SEQ ID NO: 101), CQ, and His-rhArg (SEQ ID NO: 101) plus CQ against the MDA-MB-231 breast cancer cells.

**Table 11.** Percentage of cell viability of His-rhArg (SEQ ID NO: 101) against the MDA-MB-231 cells

| His-rhArg Concentration (U/mL) | Mean of % Cell Viability (n = 3) | SEM |
|---|---|---|
| 2 | 49.10% | 1.67% |
| 0.4 | 50.24% | 0.33% |
| 0.08 | 70.87% | 1.39% |
| 0.016 | 79.26% | 7.62% |
| 0.0032 | 93.70% | 2.62% |
| 0 | 100.00% | 1.47% |

**Table 12.** Percentage of cell viability of the MDA-MB-231 cells after incubated with CQ for 72 h

| CQ Concentration ($\mu$M) | Mean of % Cell Viability (n = 3) | SEM |
|---|---|---|
| 100 | 43.17% | 0.97% |
| 20 | 75.18% | 1.50% |
| 4 | 82.16% | 0.49% |
| 0.8 | 98.14% | 1.38% |
| 0.16 | 99.03% | 1.67% |
| 0 | 100.00% | 3.53% |

**Table 13.** Combination index (CI) and the percentage of cell viability of combination of His-rhArg (SEQ ID NO: 101) and CQ against the MDA-MB-231 breast cancer cells

| His-rhArg Concentration (U/mL) | CQ Concentration ($\mu$M) | Mean of % Cell Viability (n = 3) | SEM | CI |
|---|---|---|---|---|
| 2 | 20 | 44.55% | 1.88% | 1.697 |
| 0.4 | 20 | 45.92% | 0.65% | 0.584 |
| 0.08 | 20 | 55.33% | 1.42% | 0.568 |
| 0.016 | 20 | 61.59% | 2.68% | 0.623 |
| 0.0032 | 20 | 46.62% | 0.60% | 0.263 |
| 0 | 0 | 100.00% | 3.92% | |

**Table 14.** $IC_{50}$ of His-rhArg (SEQ ID NO: 101), CQ and combining His-rhArg (SEQ ID NO: 101) and CQ against the MDA-MB-231 cells

| MDA-MB-231 | $IC_{50}$ | SEM |
|---|---|---|
| His-rhArg | 0.9522 U/mL | 0.1299 U/mL |
| CQ | 73.39 $\mu$M | 5.36 $\mu$M |
| His-rhArg + CQ | 0.1408 U/mL | 0.0274 U/mL |

We also tested the drug combination of His-rhArg (SEQ ID NO: 101) (0.016, 0.08, or 0.4 U/mL) and CQ (20 $\mu$M) on a human liver cancer cell line "Red Flu HepG2". Synergistic effects (CI values less than 1) were also observed.

**Example 39: Trans-well migration assay and invasion assay**

Breast cancer cell lines (human MDA-MB-231 cells and mouse 4T1 cells) were used in these studies. Both migration assay and invasion assay were performed in 24 wells Costar (Corning) Trans-well system (polycarbonate membrane inserts, pore size 8.0 μm). Before the assay, the cells were cultivated in appropriate serum free medium for at least 24 h. One day before seeding cells to the upper chambers, 100 μL diluted matrigel was added to the upper chambers of the inserts for invasion assay. Serum free medium (100 μL) was added to the upper chamber used for migration assay. The 24 well plates were incubated in the incubator for overnight. The next day, cells were trypsinized and counted. In the studies, 3 x 10$^4$ cells of 4T1 or 5 x 10$^4$ cells of MDA-MB-231, with 200 μL appropriate serum free medium, were seeded into the upper chambers. Appropriate complete medium (750 μL) was added to the lower chambers. For the drug treatment group, 1 U/mL His-rhArg (SEQ ID NO: 101) was added to both chambers. The 24 well plates were incubated at 37 °C humidified atmosphere with 5% CO$_2$ for 20 h (4T1 cells) or 24 h (MDA-MB-231 cells).

After incubation, the medium in the upper chambers was removed. The trans-well inserts were washed with 1x PBS twice. Cold 100% methanol was added to both upper chambers and lower chambers for 10 min to fix the cells. The inserts were washed with 1x PBS for two times. After that, 1% crystal violet in 1x PBS was added to both chambers for 15 min to stain the cells. The inserts were washed with 1x PBS twice. The cells on the surface of the upper chamber were removed by cotton swab. The inserts were air dried for overnight. Photos were taken by confocal microscope. The results showed that the number of cancer cells that can migrate and invade via the transwell inserts decreased when the cells were treated with 1 U/mL His-rhArg (SEQ ID NO: 101), indicating that arginine depletion by arginase can inhibit cancer cell migration and invasion.

**Example 40: N-ABD-rhArg (SEQ ID NO: 49) inhibits growth and metastasis of aggressive breast cancer cells in vivo**

For the aggressive 4T1 breast cancer cell xenograft model studies, 10 female nude mice (11 - 12 weeks of age) were used. They were kept in groups of five per cage and provided food and water ad libitum. The 4T1 cancer cells were harvested by trypsinization and washed twice with 1X PBS and resuspended in 1X PBS for inoculation. The cancer cells (1 x 10$^6$ cells of 4T1) were injected subcutaneously into the left flank of the mice. Mice were separated into two groups by random when the tumor had a mean length of 6 mm after 3 days of inoculation. They were injected with either 500U (3.2 mg, 230 μL) N-ABD-rhArg (SEQ ID NO: 49) or 1X PBS once per week. Blood samples were collected from the saphenous vein. Tumor size and the body weight of mice were monitored regularly. The tumor volume was estimated by the following equation:

$$\text{Tumor volume (mm}^3) = \frac{length \; x \; width^2}{2}$$

The mice were sacrificed after 16 days of cancer cell inoculation (i.e. 13 days after starting of the experiment) because of necrosis of tumor in the PBS-injected control group. After being sacrificed, tumors were collected from the mice and the weight of the tumor was measured. Lungs were also collected and washed with 1X PBS. The lungs were fixed in Bouin's solution overnight and washed with 1X PBS after fixation. Lung metastatic nodules were counted.

Importantly, 500U of the albumin-binding arginase N-ABD-rhArg (SEQ ID NO: 49) depleted arginine to a level below the detection limit of the Biochrom 30 Amino Acid Analyzer (3 μM) for at least 10 days in BALB/c mice (FIG. 10). Therefore, 500 U of N-ABD-rhArg (SEQ ID NO: 49) was administered via i.p. injection once per week to the 4T1 breast cancer xenograft nude mice for efficacy study. **Table 15** shows that 500 U of N-ABD-rhArg (SEQ ID NO: 49) depleted arginine to an undetected level during the experiment.

**Table 15.** Serum arginine levels of the N-ABD-rhArg (SEQ ID NO: 49) treated group of mice (mouse 1-4). The levels were measured by using the Biochrom 30 Amino Acid Analyzer. BDL: Below Detection Level; Day 0: before treatment

|  | Day 0 | Day 6 | Day 13 |
|---|---|---|---|
|  | Arginine Concentration (μM) | Arginine Concentration (μM) | Arginine Concentration (μM) |
| 1 | 132.5 | 0 (BDL) | 0 (BDL) |
| 2 | 117.0 | 0 (BDL) | 0 (BDL) |
| 3 | 130.5 | 0 (BDL) | 0 (BDL) |
| 4 | 122.5 | 0 (BDL) | 0 (BDL) |

FIG. 64-67 show that 500U of N-ABD-rhArg (SEQ ID NO: 49) can inhibit the growth of 4T1 breast cancer xenograft in nude mice. There was significant difference in estimated tumor volume between the control group and experimental group 12 days and 13 days after starting the experiment (FIG. 64 and 65). There were also prominent differences in tumor size between the two groups (FIG. 66 and 67).

These results suggested that arginine depletion by using 500U of N-ABD-rhArg (SEQ ID NO: 49) injected once per week can inhibit the growth of 4T1 aggressive breast cancer xenograft in nude mice significantly. The *in vivo* study showed that N-ABD-rhArg (SEQ ID NO: 49) can inhibit growth of cancer in nude mice in a safe and efficient way.

Furthermore, we found that arginine starvation can inhibit 4T1 breast cancer cell metastasis which was consistent with our *in vitro* results. Lungs of the mice from the treatment group were collected and carefully examined for signs of metastasis after the mice were sacrificed. Three lung metastatic nodules were found in 3 different lungs of the control group; whereas there were no metastatic nodules found in the N-ABD-rhArg (SEQ ID NO: 49) treated group **(Table 16).** The results showed convincingly that arginine starvation by N-ABD-rhArg (SEQ ID NO: 49) can inhibit tumor metastasis. This suggested that arginine might play an important role in cell migration and tumor metastasis. Depletion of arginine by N-ABD-rhArg (SEQ ID NO: 49) may inhibit some pathways that are important for the metastasis of cancer cells (Knott et al., 2018, Nature, 554:378-381).

**Table 16.** Number of metastatic nodules in animal studies.

| Group | Number of metastatic nodulesfound in lungs | % of metastasis free animals |
|---|---|---|
| PBS Control (n = 6) | 3 | 50% |
| 500 U of N-ABD-rhArg (n = 4) | 0 | 100% |

**Example 41: Cytotoxicity on human colon cancer cell lines with the N-ABD094-rhArg (SEQ ID NO: 50) fusion enzyme**

The *in vitro* efficacy of ABD and rhArg fusion protein was tested on human colon cancer cell lines. Human colon cancer cell lines (HCT116, LOVO and HT29) were seeded in 96-well plate at densities of $5\times10^3$ cells/well. After 1 day of incubation, the culture medium was replaced with medium containing different concentrations (2, 0.4, 0.08, 0.016 and 0.0032 U/ml) of N-ABD094-rhArg (SEQ ID NO: 50). MTT assay was conducted at Day 3 [3 days after N-ABD094-rhArg (SEQ ID NO: 50) treatment] to determine the $IC_{50}$ values. The culture medium was replaced with MTT solution (1 mg/ml) (Invitrogen) and incubated at 37°C for 4 h. After incubation, MTT solution was replaced with dimethyl sulfoxide (DMSO), and the absorbance at 570 nm was measured with a reference of 650 nm. The cell viability was determined by dividing the absorbance of N-ABD094-rhArg-treated cells by the average absorbance of untreated cells. The $IC_{50}$ values of N-ABD094-rhArg (SEQ ID NO: 50) on the cancer cell lines (at 95% confidence interval) were analyzed by software Prism 6.0. Three independent sets of experiments (n=3) were performed for each cell line. Results are shown in **Table 17.**

**Table 17.** Single drug experiment using N-ABD094-rhArg (SEQ ID NO: 50); Drug = N-ABD094-rhArg (SEQ ID NO: 50); Cell line = HCT116

| Drug conc. (U/mL) | 0 | 0.0032 | 0.016 | 0.08 | 0.4 | 2 |
|---|---|---|---|---|---|---|
| Mean cell viability (%) | 1 | 0.938 | 0.950 | 0.608 | 0.452 | 0.419 |
| SEM | 0 | 0.042 | 0.116 | 0.086 | 0.051 | 0.046 |

Cell line = LOVO

| Drug conc. (U/mL) | 0 | 0.0032 | 0.016 | 0.08 | 0.4 | 2 |
|---|---|---|---|---|---|---|
| Mean cell viability (%) | 1 | 0.922 | 0.848 | 0.566 | 0.432 | 0.392 |
| SEM | 0 | 0.048 | 0.037 | 0.075 | 0.040 | 0.038 |

Cell line = HT29

| Drug conc. (U/mL) | 0 | 0.0032 | 0.016 | 0.08 | 0.4 | 2 |
|---|---|---|---|---|---|---|
| Mean cell viability (%) | 1 | 0.962 | 0.853 | 0.521 | 0.423 | 0.383 |
| SEM | 0 | 0.070 | 0.084 | 0.016 | 0.009 | 0.029 |

All the colon cancer cell lines tested were very sensitive to the N-ABD094-rhArg (SEQ ID NO: 50) fusion enzyme in vitro with $IC_{50}$ values between 0.22 and 0.58 U/ml. HCT116 was the most sensitive tumor with an $IC_{50}$ of 0.22 U/ml. The $IC_{50}$ values for LOVO and HT29 were 0.38 U/ml and 0.58 U/ml, respectively.

**Example 42: Combination of N-ABD094-rhArg (SEQ ID NO: 50) and chloroquine exert synergistic cytotoxicity effects on human colon cancer cell lines**

The $IC_{50}$ values of the autophagy inhibitor chloroquine (CQ) were determined as follows: colon cancer cell lines HCT116, LOVO and HT29 were seeded separately and after 1 day of incubation, the culture medium was replaced with medium containing different concentrations of CQ (100, 20, 4, 0.8, 0.16 $\mu$M). MTT assay was conducted at Day 3. Three independent sets of experiments (n=3) were performed for each cell line. Results are shown in **Table 18.**

**Table 18.** Single drug experiment using CQ; Drug = Chloroquine (CQ); Cell line = HCT116

| Drug conc. (U/mL) | 0 | 0.16 | 0.8 | 4 | 20 | 100 |
|---|---|---|---|---|---|---|
| Mean cell viability (%) | 1 | 0.981 | 0.967 | 0.921 | 0.897 | 0.874 |
| SEM | 0 | 0.032 | 0.037 | 0.059 | 0.033 | 0.027 |

Cell line = LOVO

| Drug conc. (U/mL) | 0 | 0.16 | 0.8 | 4 | 20 | 100 |
|---|---|---|---|---|---|---|
| Mean cell viability (%) | 1 | 0.973 | 0.921 | 0.759 | 0.495 | 0.060 |
| SEM | 0 | 0.029 | 0.017 | 0.027 | 0.018 | 0.002 |

Cell line = HT29

| Drug conc. (U/mL) | 0 | 0.16 | 0.8 | 4 | 20 | 100 |
|---|---|---|---|---|---|---|
| Mean cell viability (%) | 1 | 1.057 | 1.009 | 0.983 | 1.018 | 0.037 |
| SEM | 0 | 0.079 | 0.071 | 0.083 | 0.073 | 0.006 |

The $IC_{50}$ values of CQ for HCT116, LOVO and HT29 were >100 $\mu$M, 15 $\mu$M and 71 $\mu$M, respectively. For drug-combination assay, a non-constant ratio analysis was performed (Hastak et al., 2010, Cancer Res. 70(20):7970-7980). Colon cancer cells were seeded and after 1 day of incubation, the culture medium was replaced with medium containing different concentrations of N-ABD094-rhArg (SEQ ID NO: 50) (2, 0.4, 0.08, 0.016, 0.0032 U/ml) combined with fixed amount of CQ according to its $IC_{50}$. MTT assay was conducted at Day 3. The combination index (CI) of combined N-ABD094-rhArg (SEQ ID NO: 50) and CQ treatment was analyzed by software CalcuSyn version 2.1. Three independent sets of experiments (n=3) were performed for each cell line. Results are shown in **Table 19.**

**Table 19.** Combination drug experiment (Drug 1 plus Drug 2); Drug 1 = N-ABD094-rhArg (SEQ ID NO: 50); Drug 2 = Chloroquine (CQ) (fixed concentration at 20 $\mu$M); Cell line = HCT116

| Drug 1 conc. (U/mL) | 0 | 0.0032 | 0.016 | 0.08 | 0.4 | 2 |
|---|---|---|---|---|---|---|
| Mean cell viability (%) | 1 | 0.951 | 0.885 | 0.476 | 0.273 | 0.231 |
| SEM | 0 | 0.024 | 0.027 | 0.008 | 0.008 | 0.006 |

Cell line = LOVO

| Drug 1 conc. (U/mL) | 0 | 0.0032 | 0.016 | 0.08 | 0.4 | 2 |
|---|---|---|---|---|---|---|
| Mean cell viability (%) | 1 | 0.555 | 0.542 | 0.211 | 0.186 | 0.177 |
| SEM | 0 | 0.008 | 0.008 | 0.002 | 0.010 | 0.005 |

Cell line = HT29

| Drug 1 conc. (U/mL) | 0 | 0.0032 | 0.016 | 0.08 | 0.4 | 2 |
|---|---|---|---|---|---|---|
| Mean cell viability (%) | 1 | 0.979 | 0.983 | 0.480 | 0.369 | 0.367 |
| SEM | 0 | o.o35 | 0.054 | 0.036 | 0.027 | 0.023 |

Results indicate that the CI values were < 1.0 when 0.08 or 0.4 U/ml of N-ABD094-rhArg (SEQ ID NO: 50) was combined with 20 $\mu$M of CQ in all the 3 colon cancer cell lines. This suggests that N-ABD094-rhArg (SEQ ID NO: 50) is active alone and is synergistic in combination with CQ. Taken together, autophagy inhibitor chloroquine (CQ) enhances the cell death inducing effect of the N-ABD094-rhArg (SEQ ID NO: 50) fusion protein in human colon cancer cells.

**Example 43: N-ABD-rhArg (SEQ ID NO: 49) and PEGylated His-rhArg (SEQ ID NO: 101) both inhibit growth and metastasis of aggressive breast cancer cells in vivo**

In another 4T1 breast cancer allograft study, female nude mice (5 - 6 weeks of age) were used. They were kept in groups of five per cage and provided food and water ad libitum. Cells of 4T1 breast cancer (1 x 105) were injected subcultuaneously into the right flank of the mice. Mice were separated into four groups by random when the tumor had a mean length of 8 mm after 11 days of inoculation. There were one control group (n = 8) and three experimental groups, including 500U PEGylated His-rhArg (SEQ ID NO: 101) (n = 8), 500U N-ABD-rhArg (SEQ ID NO: 49) (n = 6) and 250U N-ABD-rhArg (SEQ ID NO: 49) (n = 8). Mice in the control group were injected with 1X PBS weekly. For the 500U ABD-rhArg group and the 250U ABD-rhArg group, mice were injected with 500U and 250U ABD-rhArg once a week, respectively; one group was injected with 500U PEG-rhArg once per week. The mice were sacrificed after 24 days of inoculation (i.e. 14 days after starting of experiment) because of necrosis of tumor in PBS control group. After sacrificing, the mice were dissected. Tumors were collected from the mice and measured their weight. Lungs were also collected and washed with 1X PBS. The lungs were fixed in Bouin's solution overnight and washed with 1X PBS after fixation. Lung metastatic nodules were counted.

For the results, Fig. 68 shows that both 500U of ABD-rhArg and 500U of PEG-rhArg inhibited the growth of the aggressive 4T1 solid tumor significantly. However, a lower dose of 250U ABD-rhArg did not inhibit the growth of the tumor. The result also matched with the change of tumor weight from different groups (Fig. 69). Therefore, ABD-rhArg inhibited tumor growth in a dose-dependent manner. Importantly, the data showed clearly that arginine starvation (either by ABD-rhArg or by PEG-rhArg) inhibited or reduced tumor metastasis to the lungs (Fig. 70A and Fig. 70B). The metastasis nodules from the lungs in both the 500U PEG-rhArg group and 500U ABD-rhArg group were very significantly reduced compared with the control group. Lung histological sections with hematoxylin and eosin (H&E) staining showed that the nodules were formed by a series of dense cells of breast cancer. The results suggested that the nodules were metastatic foci. Taken together, depletion of arginine by long-acting arginase inhibits metastasis of aggressive cancer cells in the mouse model. As cancer metastasis is the major cause of death in patients (Knott et al., 2018, Nature, 554:378-381), this finding may open up new possibilities for a more effective cancer therapy.

**Claims**

1. An arginine depleting agent for use in treating at least one condition selected from the group consisting of obesity, a metabolic disorder, and a related complication in a subject in need thereof, wherein the arginine depleting agent is an arginine catabolic enzyme selected from the group consisting of an arginase protein, an arginine deiminase protein, and an arginine decarboxylase protein, the metabolic disorder is selected from the group consisting of glucose intolerance, hyperglycemia, diabetes mellitus, hepatic steatosis, renal steatosis, pancreatic steatosis, cardiac steatosis, and insulin resistance; and the related complication is one or more conditions selected from the group consisting of diabetic nephropathy, diabetic vasculopathy, diabetic neuropathy, hypercholesterolemia, dyslipidemia, hypertriglyceridemia, hyperleptinemia, hyperinsulinemia, steatosis, steatohepatitis, hypertension, cardiovascular disease, and whitening of brown fat.

2. The arginine depleting agent for the use of claim 1, wherein the metabolic disorder is insulin resistance.

3. The arginine depleting agent for the use of claim 1, wherein the treatment of obesity comprises at least one of preventing fat mass gain and reducing fat mass.

4. The arginine depleting agent for the use of claim 1, wherein the metabolic disorder is selected from the group consisting of hepatic steatosis, renal steatosis, pancreatic steatosis and cardiac steatosis.

5. The arginine depleting agent for the use of claim 1, wherein the arginine concentration in the subject's serum is maintained below 50 $\mu$M.

6. The arginine depleting agent for the use of claim 1, wherein the arginase protein, arginine deiminase protein, or arginine decarboxylase protein further comprises one or more polyethylene glycol (PEG) groups.

7. The arginine depleting agent for the use of claim 6, wherein the arginase protein comprises a polypeptide having SEQ ID NO: 101, SEQ ID NO: 102, SEQ ID NO: 103, or SEQ ID NO: 104.

8. The arginine depleting agent for the use of claim 1, wherein the arginase protein comprises a polypeptide with at least 95% sequence homology with SEQ ID NO: 69, SEQ ID NO: 70, SEQ ID NO: 71, or SEQ ID NO: 72.

9. The arginine depleting agent for the use of claim 1, wherein the arginase protein, arginine deiminase protein, or arginine decarboxylase protein further comprises an albumin binding domain (ABD) or a human serum albumin, or a human IgG Fc domain.

10. The arginine depleting agent for the use of claim 9, wherein the ABD comprises a polypeptide with at least 93% sequence homology with SEQ ID NO: 66, SEQ ID NO: 67, or SEQ ID NO: 68.

11. The arginine depleting agent for the use of claim 9, wherein the arginine catabolic enzyme is a fusion protein comprising an ABD polypeptide and an arginase polypeptide; an ABD polypeptide and an arginine deiminase polypeptide; or an ABD polypeptide and an arginine decarboxylase polypeptide.

12. The arginine depleting agent for the use of claim 11, wherein the arginine catabolic enzyme is a fusion protein comprising an ABD polypeptide and an arginase polypeptide.

13. The arginine depleting agent for the use of claim 12, wherein the arginine catabolic enzyme comprises a polypeptide having at least 98% sequence homology with SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 75, or SEQ ID NO: 76.

14. The arginine depleting agent for the use of claim 12, wherein the arginine catabolic enzyme comprises two $Mn^{2+}$ metal ions.

15. The arginine depleting agent for the use of claim 12, wherein the arginine catabolic enzyme comprises one or more divalent metal ions selected from the group consisting of $Co^{2+}$ and $Ni^{2+}$.

**Patentansprüche**

1. Arginin abbauendes Mittel zur Verwendung bei der Behandlung mindestens eines Zustands, ausgewählt aus der Gruppe bestehend aus Fettleibigkeit, einer Stoffwechselstörung und einer damit verbundenen Komplikation bei einem Subjekt, das dessen bedarf, wobei das Arginin abbauende Mittel ein Arginin-abbauendes Enzym ist, ausgewählt aus der Gruppe bestehend aus einem Arginase-Protein, einem Arginin-Deimase-Protein und einem Arginin-Decarboxylase-Protein, einem Arginin-Desimi-nase-Protein und einem Arginin-Decarboxylase-Protein besteht, die Stoffwechselstörung aus der Gruppe ausgewählt ist, die aus Glukoseintoleranz, Hyperglykämie, Diabetes mellitus, Lebersteatose, Nierensteatose, Bauchspeicheldrüsensteatose, Herzsteatose und Insulinresistenz besteht; und die damit verbundene Komplikation eine oder mehrere Erkrankungen ist, die aus der Gruppe ausgewählt sind, die aus diabetischer Nephropathie, diabetischer Vaskulopathie, diabetischer Neuropathie, Hypercholesterinämie, Dyslipidämie, Hypertriglyceridämie, Hyperleptinämie, Hyperinsulinämie, Steatose, Steatohepatitis, Bluthochdruck, kardiovaskulären Erkrankungen und Aufhellung des braunen Fetts besteht.

2. Das Arginin abbauende Mittel für die Verwendung nach Anspruch 1, wobei die Stoffwechselstörung Insulinresistenz ist.

3. Das Arginin abbauende Mittel zur Verwendung nach Anspruch 1, wobei die Behandlung von Fettleibigkeit mindestens eine der folgenden Maßnahmen umfasst: Verhinderung der Zunahme von Fettmasse und Verringerung der Fettmasse.

4. Das Arginin abbauende Mittel zur Verwendung nach Anspruch 1, wobei die Stoffwechselstörung ausgewählt ist aus der Gruppe bestehend aus Lebersteatose, Nierensteatose, Pankreassteatose und Herzsteatose.

5. Das Arginin abbauende Mittel zur Verwendung nach Anspruch 1, wobei die Argininkonzentration im Serum des Patienten unter 50 $\mu$M gehalten wird.

6. Das Arginin abbauende Mittel zur Verwendung nach Anspruch 1, wobei das Arginaseprotein, Arginindeiminaseprotein oder Arginindecarboxylaseprotein weiterhin eine oder mehrere Polyethylenglykol (PEG)-Gruppen umfasst.

7. Arginin abbauendes Mittel für die Verwendung nach Anspruch 6, wobei das Arginaseprotein ein Polypeptid mit SEQ ID NO: 101, SEQ ID NO: 102, SEQ ID NO: 103 oder SEQ ID NO: 104 umfasst.

8. Arginin abbauendes Mittel zur Verwendung nach Anspruch 1, wobei das Arginaseprotein ein Polypeptid mit mindestens 95% Sequenzhomologie mit SEQ ID NO: 69, SEQ ID NO: 70, SEQ ID NO: 71 oder SEQ ID NO: 72 umfasst.

9. Arginin abbauendes Mittel zur Verwendung nach Anspruch 1, wobei das Arginase-Protein, Arginin-Deiminase-Protein oder Arginin-Decarboxylase-Protein ferner eine Albumin-Bindungsdomäne (ABD) oder ein menschliches Serumalbumin oder eine menschliche IgG-Fc-Domain umfasst.

10. Arginin abbauendes Mittel zur Verwendung nach Anspruch 9, wobei die ABD ein Polypeptid mit mindestens 93 % Sequenzhomologie mit SEQ ID NO: 66, SEQ ID NO: 67 oder SEQ ID NO: 68 umfasst.

11. Arginin abbauendes Mittel zur Verwendung nach Anspruch 9, wobei das Argininabbauende Enzym ein Fusionsprotein ist, das ein ABD-Polypeptid und ein Arginase-Polypeptid; ein ABD-Polypeptid und ein Arginin-Deiminase-Polypeptid; oder ein ABD-Polypeptid und ein Arginin-Decarboxylase-Polypeptid umfasst.

12. Arginin abbauendes Mittel für die Verwendung nach Anspruch 11, wobei das Argininabbauende Enzym ein Fusionsprotein ist, das ein ABD-Polypeptid und ein Arginase-Polypeptid umfasst.

13. Arginin abbauendes Mittel zur Verwendung nach Anspruch 12, wobei das Argininabbauende Enzym ein Polypeptid mit mindestens 98% Sequenzhomologie mit SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 75 oder SEQ ID NO: 76 umfasst.

14. Arginin abbauendes Mittel für die Verwendung nach Anspruch 12, wobei das Argininabbauende Enzym zwei $Mn^{2+}$ Metallionen umfasst.

15. Arginin abbauendes Mittel zur Verwendung nach Anspruch 12, wobei das Argininabbauende Enzym ein oder mehrere zweiwertige Metallionen umfasst, ausgewählt aus der Gruppe bestehend aus $Co^{2+}$ und $Ni^{2+}$.

## Revendications

1. Agent d'appauvrissement de l'arginine utilisé pour traiter au moins une condition sélectionnée dans le groupe constitué par l'obésité, un trouble métabolique et une complication connexe chez un sujet qui en a besoin, dans lequel l'agent d'appauvrissement de l'arginine est une enzyme catabolique de l'arginine sélectionnée dans le groupe constitué par une protéine arginase, une protéine arginine déiminase et une protéine arginine décarboxylase, une protéine arginine déiminase et une protéine arginine décarboxylase, le trouble métabolique est choisi dans le groupe constitué par l'intolérance au glucose, l'hyperglycémie, le diabète sucré, la stéatose hépatique, la stéatose rénale, la stéatose pancréatique, la stéatose cardiaque et la résistance à l'insuline ; et la complication associée est une ou plusieurs affections choisies dans le groupe constitué par la néphropathie diabétique, la vasculopathie diabétique, la neuropathie diabétique, l'hypercholestérolémie, la dyslipidémie, l'hypertriglycéridémie, l'hyperleptinémie, l'hyperinsulinémie, la stéatose, la stéatohépatite, l'hypertension, les maladies cardiovasculaires et le blanchiment de la graisse brune.

2. L'agent de déplétion de l'arginine pour l'utilisation de la revendication 1, dans lequel le trouble métabolique est la résistance à l'insuline.

3. L'agent de déplétion de l'arginine pour l'utilisation de la revendication 1, dans lequel le traitement de l'obésité comprend au moins l'un des éléments suivants : prévention de l'augmentation de la masse grasse et réduction de la masse grasse.

4. L'agent de déplétion de l'arginine pour l'utilisation de la revendication 1, dans lequel le trouble métabolique est choisi dans le groupe constitué de la stéatose hépatique, de la stéatose rénale, de la stéatose pancréatique et de la stéatose cardiaque.

5. L'agent de déplétion de l'arginine pour l'utilisation de la revendication 1, dans lequel la concentration d'arginine dans le sérum du sujet est maintenue en dessous de 50 $\mu$M.

6. L'agent de déplétion de l'arginine pour l'utilisation de la revendication 1, dans lequel la protéine arginase, la protéine arginine déiminase, ou la protéine arginine décarboxylase comprend en outre un ou plusieurs groupes de polyéthylène glycol (PEG).

7. L'agent de déplétion de l'arginine pour l'utilisation de la revendication 6, dans lequel la protéine arginase comprend un polypeptide ayant SEQ ID NO : 101, SEQ ID NO : 102, SEQ ID NO : 103, ou SEQ ID NO : 104.

8. L'agent de déplétion de l'arginine pour l'utilisation de la revendication 1, dans lequel la protéine arginase comprend un polypeptide avec au moins 95% d'homologie de séquence avec SEQ ID NO : 69, SEQ ID NO : 70, SEQ ID NO : 71, ou SEQ ID NO : 72.

9. L'agent de déplétion de l'arginine pour l'utilisation de la revendication 1, dans lequel la protéine arginase, la protéine arginine déiminase, ou la protéine arginine décarboxylase comprend en outre un domaine de liaison à l'albumine (ABD) ou une sérum-albumine humaine, ou un domaine IgG Fc humaine.

10. L'agent de déplétion de l'arginine pour l'utilisation de la revendication 9, dans lequel l'ABD comprend un polypeptide avec au moins 93% d'homologie de séquence avec SEQ ID NO : 66, SEQ ID NO : 67, ou SEQ ID NO : 68.

11. L'agent de déplétion de l'arginine pour l'utilisation de la revendication 9, dans lequel l'enzyme catabolique de l'arginine est une protéine de fusion comprenant un polypeptide ABD et un polypeptide arginase ; un polypeptide ABD et un polypeptide arginine déiminase ; ou un polypeptide ABD et un polypeptide arginine décarboxylase.

12. L'agent de déplétion de l'arginine pour l'utilisation de la revendication 11, dans lequel l'enzyme catabolique de l'arginine est une protéine de fusion comprenant un polypeptide ABD et un polypeptide arginase.

13. L'agent de déplétion de l'arginine pour l'utilisation de la revendication 12, dans lequel l'enzyme catabolique de l'arginine comprend un polypeptide ayant au moins 98% d'homologie de séquence avec SEQ ID NO : 49, SEQ ID NO : 50, SEQ ID NO : 51, SEQ ID NO : 52, SEQ ID NO : 53, SEQ ID NO : 54, SEQ ID NO : 55, SEQ ID NO : 56, SEQ ID NO : 75, ou SEQ ID NO : 76.

14. L'gent d'épuisement de l'arginine pour l'utilisation de la revendication 12, dans lequel l'enzyme catabolique de l'arginine comprend deux ions métalliques Mn2+.

15. L'agent de déplétion de l'arginine pour l'utilisation de la revendication 12, dans lequel l'enzyme catabolique de l'arginine comprend un ou plusieurs ions métalliques divalents choisis dans le groupe constitué de Co 2+ et Ni 2+.

**FIG. 1**

ABD094-rhArg
1220 bp

**FIG. 2A**

```
          10          20          30          40          50          60
MHHHHHHDEA VDANSLAEAK EAANAELDSY GVSDFYKRLI DKAKTVEGVE ALKDAILAAL

          70          80          90         100         110         120
PSGSNNNNNN GSGGMSAKSR TIGIIGAPFS KGQPRGGVEE GPTVLRKAGL LEKLKEQECD

         130         140         150         160         170         180
VKDYGDLPFA DIPNDSPFQI VKNPRSVGKA SEQLAGKVAE VKKNGRISLV LGGDHSLAIG

         190         200         210         220         230         240
SISGHARVHP DLGVIWVDAH TDINTPLTTT SGNLHGQPVS FLLKELKGKI PDVPGFSWVT

         250         260         270         280         290         300
PCISAKDIVY IGLRDVDPGE HYILKTLGIK YFSMTEVDRL GIGKVMEETL SYLLGRKKRP

         310         320         330         340         350         360
IHLSFDVDGL DPSFTPATGT PVVGGLTYRE GLYITEEIYK TGLLSGLDIM EVNPSLGKTP

         370         380         390
EEVTRTVNTA VAITLACFGL AREGNHKPID YLNPPK
```

# FIG. 2B

MKPISIIGVPMDLGQTRRGVDMGPSAMRYAGVIERLERLHYDIEDLGDIPIGKAERLHEQ
GDSRLRNLKAVAEANEKLAAAVDQVVQRGRFPLVLGGDHSIAIGTLAGVAKHYERLGVIW
YDAHGDVNTAETSPSGNIHGMPLAASLGFGHPALTQIGGYCPKIKPEHVVLIGVRSLDEG
EKKFIREKGIKIYTMHEVDRLGMTRVMEETIAYLKERTDGVHLSLDLDGLDPSDAPGVGT
PVIGGLTYRESHLAMEMLAEAQIITSAEFVEVNPILDERNKTASVAVALMGSLFGEKLMH
HHHHH

# FIG. 3

```
BCA    MKPISIIGVPMDLGQTRRGVDMGPSAMRYAGVIERLERLHYDIEDLGDIPIGKAERLHEQ
BHA    MKPISIIGVPMDLGQTRRGFDMGPSAMRYAGVIERLERLHYDIEDLGDIPIGKAERLHEQ
BAH    MKPISIIGVPMDLGQTRRGFDMGPSAMRYAGVIERLERLHYDIEDLGDIPIGKAERLHEQ
       ****************** *****************************************

BCA    GDSRLRNLKAVAEANEKLAAAVDQVVQRGRFPLVLGGDHSIAIGTLAGVAKHYERLGVIW
BHA    GDSRLRNLKAVAEANEKLAAAVDQVVQRGRFPLVLGGDHSIAIGTLAGVAKHYERLGVIW
BAH    GDSRLRNLKAVAEANEKLAAAVDQVVQRGRFPLVLGGDHSIAIGTLAGVAKHYERLGVIW
       ************************************************************

BCA    YDAHGDVNTAETSPSGNIHGMPLAASLGFGHPALTQIGGYCPKIKPEHVVLIGVRSLDEG
BHA    YDAHGDVNTAETSPSGNIHGMPLAASLGFGHPALTQIGGYCPKIKPEHVVLIGVRSLDEG
BAH    YDAHGDVNTAETSPSGNIHGMPLAASLGFGHPALTQIGGYCPKIKPEHVVLIGVRSLDEG
       ************************************************************

BCA    EKKFIREKGIKIYTMHEVDRLGMTRVMEETIAYLKERTDGVHLSLDLDGLDPSDAPGVGT
BHA    EKKFIREKGIKIYTMHEVDRLGMTRVMEETIAYLKERTDGVHLSLDLDGLDPSDAPGVGT
BAH    EKKFIREKGIKIYTMHEVDRLGMTRVMEETIAYLKERTDGVHLSLDLDGLDPSDAPGVGT
       ************************************************************

BCA    PVIGGLTYRESHLAMEMLAEAQIITSAEFVEVNPILDERNKTASVAVALMGSLFGEKLMH
BHA    PVIGGLTYRESHLAMEMLAEAQIITSAEFVEVNPILDERNKTASVAVALMGSLFGEKLMH
BAH    PVIGGLTYRESHLAMEMLAEAQIITSAEFVEVNPILDERNKTASVAVALMGSLFGEKLMA
       ************************************************************

BCA    HHHHH-------------------------------------------------------
BHA    HHHHHAQHDEAVDANSLAEAKVLANRELDKYGVSDYYKNLINNAKTVEGVKALIDEILAA
BAH    QHDEAVDAN------SLAEAKVLANRELDKYGVSDYYKNLINNAKTVEGVKALIDEILAA
       :*..

BCA    ----------
BHA    LF--------
BAH    LFHHHHHH
```

**FIG. 4**

**FIG. 5A**

**FIG. 5B**

**FIG. 5C**

**FIG. 5D**

**FIG. 6A**

**FIG. 6B**

**FIG. 7**

FIG. 8

FIG. 9

**A**

## 125U N-ABD-rhArg

**B**

## 250U N-ABD-rhArg

**C**

## 500U N-ABD-rhArg

**FIG. 10**

**FIG. 11**

1    2    3    4    5    6    7

**FIG. 12**

**Plasma arginine concentration**

FIG. 13

**Plasma drug concentration**

FIG. 14

# A

Legend: ■ Chow(vehicle) □ Chow(rhArg)

# B

### H&E staining of WAT

Chow(vehicle)          Chow(rhArg)

# FIG. 15

**A**

Lipogenesis related genes in visceral WAT

■ Chow(vehicle)
□ Chow(rhArg)

**B**

Lipogenesis related genes in liver

FIG. 16

FIG. 17

**FIG. 18**

**A** <u>WAT weight</u>

**B** <u>H&E staining of WAT</u>

LFD(vehicle)    HFD(vehicle)    HFD(rhArg)

**C** <u>Lipogenesis related genes in WAT</u>

**FIG. 19**

**Lipogenesis related genes in skeletal muscle**

FIG. 20

**A**   **Bodyweight (female)**

FIG. 21

**A**    <u>Insulin tolerance test</u>

**B**    <u>Glucose tolerance test</u>

# FIG. 22

FIG. 23

**FIG. 24**

FIG. 25

**Insulin tolerance test**

FIG. 26

## Glucose tolerance test

**A**

LFD(vehicle)
HFD(vehicle)
HFD(rhArg)

5wk

FIG. 27

**B**

10wk

**A** <u>Fasting blood glucose</u>

**B** <u>Fasting plasma insulin</u>

**C** <u>Fasting HOMA-IR</u>

FIG. 28

**FIG. 29**

**A**  **Whole fresh liver**

LFD(vehicle)    HFD(vehicle)    HFD(rhArg)

**B**  **Liver weight**

**FIG. 30**

**A**    <u>Oil red O staining of lipids in liver</u>

LFD(vehicle)    HFD(vehicle)    HFD(rhArg)

**B**    <u>Liver triglyceride</u>

**C**    <u>Lipogenesis related genes in liver</u>

**FIG. 31**

**Liver damage marker**

FIG. 32

**A** **Kidney weight**

**B** **Pancreas weight**

**C** **Heart weight**

FIG. 33

**A**  **Proinflammatory adipokines in visceral WAT**

**B**  **Proinflammatory cytokine in serum**

**FIG. 34**

**A** <u>Whole fresh interscapular BAT</u>

LFD(vehicle)    HFD(vehicle)    HFD(rhArg)

**B** <u>Interscapular BAT weight</u>

**C** <u>H&E staining of BAT</u>

LFD(vehicle)    HFD(vehicle)    HFD(rhArg)

**FIG. 35**

**Regulator of thermogenesis and fatty acid oxidation in BAT**

FIG. 36

**A**

<u>Bodyweight</u>

**B**

LFD(vehicle)    HFD(vehicle)    HFD(rhArg)

**FIG. 37**

**A**

<u>WAT weight</u>

**B**

<u>H&E staining of WAT</u>

FIG. 38

## Insulin tolerance test

FIG. 39

## Glucose tolerance test

**FIG. 40**

**FIG. 41**

FIG. 42

**A** <u>Whole fresh liver</u>

LFD(vehicle)  HFD(vehicle)  HFD(rhArg)

**B** <u>Liver weight</u>

**FIG. 43**

**A**

Oil red O staining of lipids in liver

LFD(vehicle)    HFD(vehicle)    HFD(rhArg)

**B**

Liver triglyceride

**C**

Lipogenesis related genes in liver

FIG. 44

**Liver damage marker**

FIG. 45

**A** Kidney weight

LFD(vehicle)
HFD(vehicle)
HFD(rhArg)

**B** Kidney triglyceride

**C** H&E staining of kidney

Vacuolated structures within renal tubules

LFD(vehicle)        HFD(vehicle)        HFD(rhArg)

**D** Oil red O staining of lipids in kidney

Ectopic accumulation of lipid droplets within glomerulus (circled)

LFD(vehicle)        HFD(vehicle)        HFD(rhArg)

FIG. 46

**A**

**Pancreas weight**

- LFD(vehicle)
- HFD(vehicle)
- HFD(rhArg)

**B**

**H&E staining of pancreas**

Excessive intralobular and interlobular accumulation
of white adipose tissue

**C**

**Pancreas triglyceride**

**FIG. 47**

**A**

**Heart weight**

**B**

**H&E staining of heart**

Excessive accumulation of lipid droplets

LFD(vehicle)　　　HFD(vehicle)　　　HFD(rhArg)

**C**

**Heart triglyceride**

**FIG. 48**

**Skeletal muscle triglyceride**

FIG. 49

**A**     **Proinflammatory adipokine in visceral WAT**

**B**     **Proinflammatory cytokine in serum**

## FIG. 50

**A** Proinflammatory genes in liver

**B** Profibrotic gene in liver

**C** Anti-inflammatory genes in liver

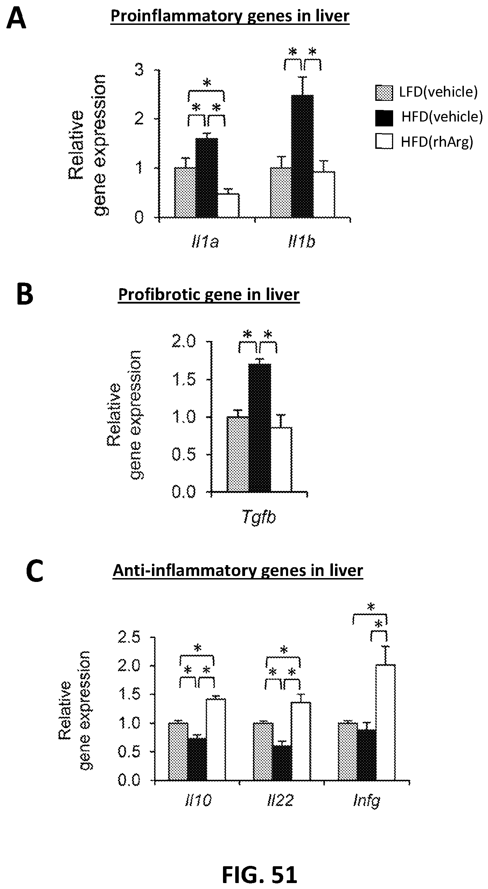

FIG. 51

**A**

**Proinflammatory genes in kidney**

Legend:
- LFD(vehicle)
- HFD(vehicle)
- HFD(rhArg)

**B**

**Profibrotic gene in kidney**

**C**

**Sirius red staining of collagen fibres**

Excessive deposit of collagen fibres around renal
tubules and hypertrophic glomeruli (g)

LFD(vehicle)          HFD(vehicle)          HFD(rhArg)

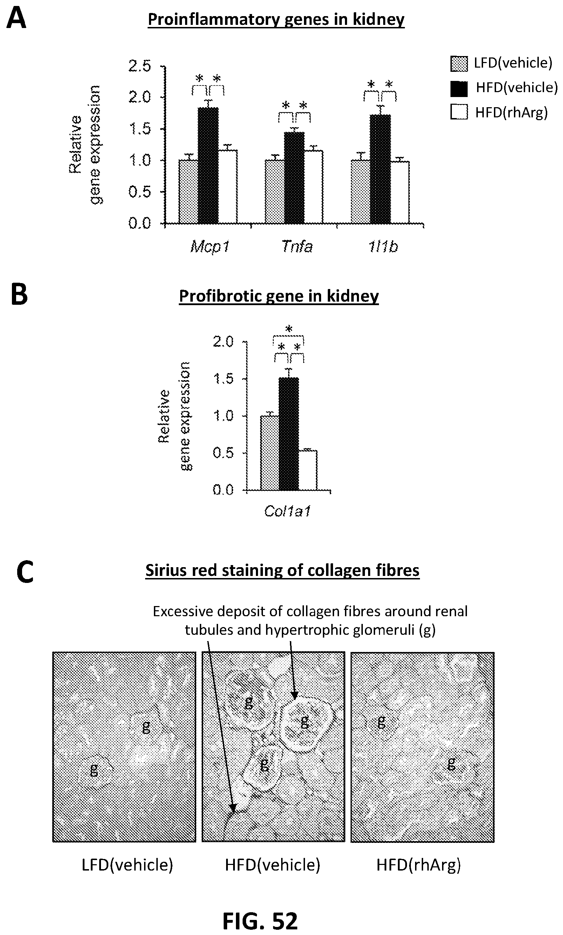

**FIG. 52**

**A**  <u>Proinflammatory gene in pancreas</u>

**B**  <u>Profibrotic gene in pancreas</u>

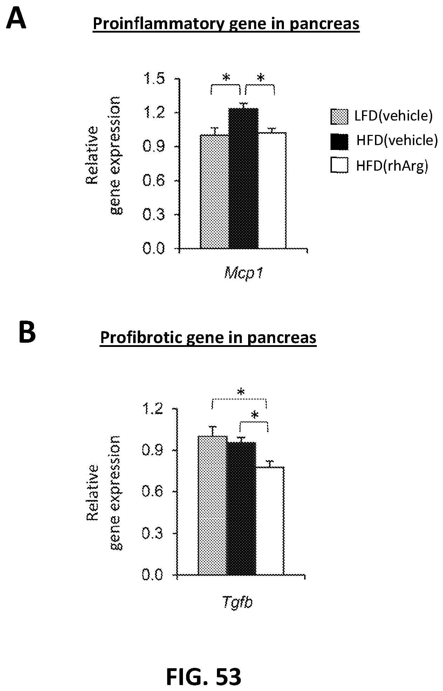

**FIG. 53**

**A**  **Interscapular BAT weight**

**B**  **Whole fresh interscapular BAT**

LFD(vehicle)    HFD(vehicle)    HFD(rhArg)

**C**  **H&E staining of interscapular BAT**

White adipocytes with single large lipid globule

LFD(vehicle)    HFD(vehicle)    HFD(rhArg)

**FIG. 54**

**A**

**Blood pressure**

**B**

**Heart rate**

**FIG. 55**

**A**    <u>Anti-rhArg antibody</u>

**B**    <u>Neutralizing activity</u>

**FIG. 56**

**A**        <u>Liver damage marker</u>

**B**        <u>Kidney function/disease marker</u>

**FIG. 57**

**A** **Vascular smooth muscle contraction**

- ◆ - LFD(vehicle)
——◇—— LFD(rhArg)

**B** **Endothelium-dependent relaxation**

**C** **Endothelium-independent relaxation**

**FIG. 58**

**Bodyweight**

FIG. 59

**Organ weight**

FIG. 60

**A**     **Fasting blood glucose**

**B**     **Insulin tolerance test**

## FIG. 61

**Bodyweight**

HFD (N-ABD094-rhArg-Co2+)

## FIG. 62

MKN45

FIG. 63

**Tumor volume**

FIG. 64

**Relative tumor volume**

FIG. 65

**Tumor weight**

FIG. 66

**Isolated tumors**

Control

N-ABD-rhArg

## FIG. 67

**Relative tumor volume**

FIG. 68

**Tumor weight**

FIG. 69

FIG. 70

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5196195 A
- EP 0956864 A1
- WO 2014138319 A2
- US 7670595 B2, Gillies
- US 8507245 B
- US 9789169 B
- US 8440184 B2, Georgiou

**Non-patent literature cited in the description**

- **PEARSON** ; **LIPMAN**. *Proc. Natl. Acad. Sci. USA*, 1988, vol. 85 (8), 2444-2448
- **ALTSCHUL et al.** *J. Mol. Biol.*, 1990, vol. 215 (3), 403-410
- **THOMPSON et al.** *Nucleic Acids Res.*, 1994, vol. 22 (2), 4673-4680
- **HIGGINS et al.** *Methods Enzymol.*, 1996, vol. 266, 383-402
- **ALTSCHUL et al.** *Nature Genetics*, 1993, vol. 3, 266-272
- **KARLIN** ; **ALTSCHUL**. *Proc. Natl. Acad. Sci. USA*, 1990, vol. 87, 2267-2268
- **ALTSCHUL et al.** *J. Mol. Biol.*, 1990, vol. 215, 403-410
- **ALTSCHUL et al.** *Nuc. Acids Res.*, 1997, vol. 25, 3389-3402
- *Prot Exp Purif.*, 2008, vol. 61, 73-7
- *Adv Drug Deliv Rev.*, 2013, vol. 65, 1357-69
- *Pharm Res.*, 2006, vol. 23, 2116-21
- *Curr Pharmaceut Biotechnol.*, 2014, vol. 15, 856-63
- **HALLET W. et al.** Exploiting arginase to prevent GVHD. *Blood*, 2010, vol. 116, 5440-5441
- **SAHIN E. et al.** Macrophage PTEN regulates expression and secretion of arginase I modulating innate and adaptive immune responses. *J Immunol.*, 15 August 2014, vol. 193 (4), 1717-27
- **FOUDA AY. et al.** Arginase 1 promotes retinal neurovascular protection from ischemia through suppression of macrophage inflammatory responses. *Cell Death Dis.*, 25 September 2018, vol. 9 (10), 1001
- **AIDA Y.** ; **PABST M.J.** *Journal of Immunological Methods.*, 1990, vol. 132, 191-195
- **BEWLEY et al.** *Structure*, 1999, vol. 7, 435-448
- **JONSSON et al.** *Protein Eng. Des. Sel.*, 2008, vol. 21, 515-527
- **MAKRIDES et al.** *J. Pharmacol. Exp. Ther.*, 1996, vol. 277, 534-542
- **HOPP et al.** *Protein Eng. Des. Sel.*, 2010, vol. 23, 827-834
- **STORK et al.** *Protein Eng. Des. Sel.*, 2007, vol. 20, 569-576
- **AHMAD et al.** *Proteins*, 2012, vol. 80, 774-789
- **GREENBERG** ; **SASSENRATH**. *Cancer Res.*, 1953, vol. 13, 709-715
- **ENSOR et al.** *Cancer Res*, 2002, vol. 62, 5443-5450
- **TSUI et al.** *Cancer Cell Int.*, 17 April 2009, vol. 9, 9
- **SAVOCA et al.** *Cancer Biochem. Biophy's.*, 1984, vol. 7, 261-268
- **WANG** ; **LIAO**. *Methods Mol. Biol.*, 2012, vol. 821, 421-433
- **VICKERS et al.** *Br. J. Pharmacol.*, 2011, vol. 164 (4), 1248-1262
- **GALLOU-KABANI et al.** *Obesity*, vol. 15 (8), 1996-2005
- **GREEN** ; **MEUTH**. *Cell*, 1974, vol. 3, 127-133
- **D'ANTONIO** ; **CHRISTIANSON**. *Biochemistry*, 2011, vol. 50, 8018-27
- **KNOTT et al.** *Nature*, 2018, vol. 554, 378-381
- **HASTAK et al.** *Cancer Res.*, 2010, vol. 70 (20), 7970-7980